# EUROPÄISCHE PATENTSCHRIFT

(11) **EP 2 993 216 B1**
(45) Veröffentlichungstag und Bekanntmachung des Hinweises auf die Patenterteilung: **26.07.2017**
(21) Anmeldenummer: 15002311.7
(22) Anmeldetag: 03.08.2015
(51) Int. Cl.: C09K 19/44, C09K 19/54, C09K 19/34, C09K 19/30, C09K 19/12, C09K 19/04, C07D 401/12

(54) **VERBINDUNGEN UND FLÜSSIGKRISTALLINES MEDIUM**
COMPOUNDS AND LIQUID CRYSTALLINE MEDIUM
COMPOSÉS ET MILIEU LIQUIDE CRISTALLIN

(30) Priorität: 02.09.2014 EP 14003014
(43) Veröffentlichungstag der Anmeldung: 09.03.2016
(73) Patentinhaber: Merck Patent GmbH, 64293 Darmstadt (DE)
(72) Erfinder: Goetz, Achim, 64665 Alsbach-Haehnlein (DE); Fortte, Rocco, 65933 Frankfurt am Main (DE); Engel, Martin, 64291 Darmstadt (DE); Maag, Sabrina, 64319 Pfungstadt (DE); Almeroth, Ingo, 64625 Bensheim (DE); Mergner, Thomas, 64747 Breuberg (DE); Kodex, Thorsten, 64546 Moerfelden-Walldorf (DE); Pauluth, Detlef, 64372 Ober-Ramstadt (DE)

(56) Entgegenhaltungen:
- EP-A2- 2 722 381
- WO-A1-2012/076104
- DE-A1-102011 013 007
- DE-A1-102011 117 937
- DE-A1-102011 119 144

## Beschreibung

Die vorliegende Erfindung betrifft Verbindungen der Formel **I** insbesondere zur Verwendung in Flüssigkristallmedien, aber auch die Verwendung dieser Flüssigkristallmedien in Flüssigkristallanzeigen sowie diese Flüssigkristallanzeigen.

Die erfindungsgemäßen Flüssigkristallmedien zeichnen sich durch eine besonders niedrige Schaltzeit in den erfindungsgemäßen Anzeigen bei gleichzeitig hohem Spannungshaltevermögen (Englisch "voltage holding ratio", kurz VHR oder auch nur HR) aus.

Flüssige Kristalle werden vor allem als Dielektrika in Anzeigevorrichtungen verwendet, da die optischen Eigenschaften solcher Substanzen durch eine angelegte Spannung beeinflusst werden können. Elektrooptische Vorrichtungen auf der Basis von Flüssigkristallen sind dem Fachmann bestens bekannt und können auf verschiedenen Effekten beruhen. Derartige Vorrichtungen sind beispielsweise Zellen mit dynamischer Streuung, DAP-Zellen (Deformation aufgerichteter Phasen), GastNVirt-Zellen, TN-Zellen mit verdrillt nematischer ("twisted nematic") Struktur, STN-Zellen ("super-twisted nematic"), SBE-Zellen ("superbirefringence effect") und OMI-Zellen ("optical mode interference"). Die gebräuchlichsten Anzeigevorrichtungen beruhen auf dem Schadt-Helfrich-Effekt und besitzen eine verdrillt nematische Struktur. Daneben gibt es auch Zellen, die mit einem elektrischen Feld parallel zur Substrat- und Flüssigkristallebene arbeiten, wie beispielsweise die IPS-Zellen ("in-plane switching"). Vor allem die TN-, STN-, FFS- (Fringe Field Switching)- und IPS- Zellen, sind derzeit kommerziell interessante Einsatzgebiete für die erfindungsgemäßen Medien.

Zudem sind Flüssigkristallanzeigen die den ECB- (electrically controlled birefringence) Effekt mit dielektrisch negativen Flüssigkristallen in einer homeotropen Ausgangsorientierung verwenden bekannt.

Das Prinzip der elektrisch kontrollierten Doppelbrechung, der ECB-Effekt oder auch DAP-Effekt (Deformation aufgerichteter Phasen) wurde erstmals 1971 beschrieben (M.F. Schieckel und K. Fahrenschon, "Deformation of nematic liquid crystals with vertical orientation in electrical fields", Appl. Phys. Lett. 19 (1971), 3912). Es folgten Arbeiten von J.F. Kahn (Appl. Phys. Lett. 20 (1972), 1193) und G. Labrunie und J. Robert (J. Appl. Phys. 44 (1973), 4869).

Die Arbeiten von J. Robert und F. Clerc (SID 80 Digest Techn. Papers (1980), 30), J. Duchene (Displays 7 (1986), 3) und H. Schad (SID 82 Digest Techn. Papers (1982), 244) haben gezeigt, dass flüssigkristalline Phasen hohe Werte für das Verhältnis der elastischen Konstanten K₃/K₁, hohe Werte für die optische Anisotropie Δn und Werte für die dielektrische Anisotropie Δε ≤ -0,5 aufweisen müssen, um für hochinformative Anzeigeelemente basierend auf dem ECB-Effekt eingesetzt werden zu können. Auf dem ECB-Effekt basierende elektrooptische Anzeigeelemente weisen eine homöotrope Randorientierung auf (VA-Technologie = Vertical Aligned). Auch bei Anzeigen, die den so genannten IPS- (In Plane Switching) Effekt verwenden, können dielektrisch negative Flüssigkristallmedien zum Einsatz kommen.

Für die technische Anwendung der oben genannten Effekte in elektrooptischen Anzeigeelementen werden FK-Phasen benötigt, die einer Vielzahl von Anforderungen genügen müssen. Besonders wichtig sind hier die chemische Beständigkeit gegenüber Feuchtigkeit, Luft und physikalischen Einflüssen wie Wärme, Strahlung im infraroten, sichtbaren und ultravioletten Bereich und elektrischen Gleich- und Wechselfeldern.

Ferner wird von technisch verwendbaren FK-Phasen eine flüssigkristalline Mesophase in einem geeigneten Temperaturbereich und eine niedrige Viskosität gefordert.

In keiner der bisher bekannten Reihen von Verbindungen mit flüssigkristalliner Mesophase gibt es eine Einzelverbindung, die allen diesen Erfordernissen entspricht. Es werden daher in der Regel Mischungen von zwei bis 25, vorzugsweise drei bis 18, Verbindungen hergestellt, um als FK-Phasen verwendbare Substanzen zu erhalten.

Matrix-Flüssigkristallanzeigen (MFK-Anzeigen) sind bekannt. Als nichtlineare Elemente zur individuellen Schaltung der einzelnen Bildpunkte können beispielsweise aktive Elemente (d.h. Transistoren) verwendet werden. Man spricht dann von einer "aktiven Matrix", wobei im Allgemeinen Dünnfilm-Transistoren (TFT) verwendet werden, die in der Regel auf einer Glasplatte als Substrat angeordnet sind.

Man unterscheidet zwei Technologien: TFT's aus Verbindungshalbleitern wie z.B. CdSe oder TFT's auf der Basis von polykristallinem und u. a. amorphem Silizium. Letztere Technologie hat derzeit weltweit die größte kommerzielle Bedeutung.

Die TFT-Matrix ist auf der Innenseite der einen Glasplatte der Anzeige aufgebracht, während die andere Glasplatte auf der Innenseite die transparente Gegenelektrode trägt. Im Vergleich zu der Größe der Bildpunkt-Elektrode ist der TFT sehr klein und stört das Bild praktisch nicht. Diese Technologie kann auch für voll farbtaugliche Bilddarstellungen erweitert werden, wobei ein Mosaik von roten, grünen und blauen Filtern derart angeordnet ist, dass je ein Filterelement einem schaltbaren Bildelement gegenüber liegt.

Die bisher am meisten verwendeten TFT-Anzeigen arbeiten üblicherweise mit gekreuzten Polarisatoren in Transmission und sind von hinten beleuchtet. Für TV Anwendungen werden IPS-Zellen oder ECB- (bzw. VAN-) Zellen verwendet, wohingegen für Monitore meist IPS-Zellen oder TN-(Twisted Nematic) Zellen und für "note Books", "Lap Tops" und für mobile Anwendungen meist TN-Zellen Verwendung finden.

Der Begriff MFK-Anzeigen umfasst hier jedes Matrix-Display mit integrierten nichtlinearen Elementen, d.h. neben der aktiven Matrix auch Anzeigen mit passiven Elementen wie Varistoren oder Dioden (MIM = Metall-Isolator-Metall).

Derartige MFK-Anzeigen eignen sich insbesondere für TV-Anwendungen, Monitore und "Note Books" oder für Displays mit hoher Informationsdichte z.B. in Automobil- oder Flugzeugbau. Neben Problemen hinsichtlich der Winkelabhängigkeit des Kontrastes und der Schaltzeiten resultieren bei MFK-Anzeigen Schwierigkeiten bedingt durch einen nicht ausreichend hohen spezifischen Widerstand der Flüssigkristallmischungen [TOGASHI, S., SEKIGUCHI, K., TANABE, H., YAMAMOTO, E., SORIMACHI, K., TAJIMA, E., WATANABE, H., SHIMIZU, H., Proc. Eurodisplay 84, Sept. 1984: A 210-288 Matrix LCD Controlled by Double Stage Diode Rings, p. 141 ff, Paris; STROMER, M., Proc. Eurodisplay 84, Sept. 1984: Design of Thin Film Transistors for Matrix Adressing of Television Liquid Crystal Displays, p. 145 ff, Paris]. Mit abnehmendem Widerstand verschlechtert sich der Kontrast einer MFK-Anzeige. Da der spezifische Widerstand der Flüssigkristallmischung durch Wechselwirkung mit den inneren Oberflächen der Anzeige im Allgemeinen über die Lebenszeit einer MFK-Anzeige abnimmt, ist ein hoher (Anfangs)-Widerstand sehr wichtig für Anzeigen die akzeptable Widerstandswerte über eine lange Betriebsdauer aufweisen müssen.

Anzeigen, die den ECB-Effekt verwenden haben sich als so genannte VAN- (Vertically Aligned Nematic) Anzeigen neben IPS-Anzeigen (z.B.: Yeo, S.D., Vortrag 15.3: "A LC Display for the TV Application", SID 2004 International Symposium, Digest of Technical Papers, XXXV, Buch II, S. 758 und 759) und den lange bekannten TN-Anzeigen, als eine der drei zur Zeit wichtigsten neueren Typen von Flüssigkristallanzeigen insbesondere für Fernsehanwendungen etabliert.

Als wichtigste Bauformen sind zu nennen: MVA (Multi-Domain Vertical Alignment, z. B.: Yoshide, H. et al., Vortrag 3.1: "MVA LCD for Notebook or Mobile PCs ...", SID 2004 International Symposium, Digest of Technical Papers, XXXV, Buch I, S. 6 bis 9 und Liu, C.T. et al., Vortrag 15.1: "A 46-inch TFT-LCD HDTV Technology ...", SID 2004 International Symposium, Digest of Technical Papers, XXXV, Buch II, S. 750 bis 753), PVA (Patterned Vertical Alignment, z. B.: Kim, Sang Soo, Vortrag 15.4: "Super PVA Sets New State-of-the-Art for LCD-TV", SID 2004 International Symposium, Digest of Technical Papers, XXXV, Buch II, S. 760 bis 763) und ASV (Avanced Super View, z. B.: Shigeta, Mitzuhiro und Fukuoka, Hirofumi, Vortrag 15.2: "Development of High Quality LCDTV", SID 2004 International Symposium, Digest of Technical Papers, XXXV, Buch II, S. 754 bis 757).

In allgemeiner Form werden die Technologien z.B. in Souk, Jun, SID Seminar 2004, Seminar M-6: "Recent Advances in LCD Technology", Seminar Lecture Notes, M-6/1 bis M-6/26 und Miller, Ian, SID Seminar 2004, Seminar M-7: "LCD-Television", Seminar Lecture Notes, M-7/1 bis M-7/32, verglichen. Obwohl die Schaltzeiten moderner ECB-Anzeigen durch Ansteuerungsmethoden mit Übersteuerung (overdrive) bereits deutlich verbessert wurden, z.B.: Kim, Hyeon Kyeong et al., Vortrag 9.1: "A 57-in. Wide UXGA TFT-LCD for HDTV Application", SID 2004 International Symposium, Digest of Technical Papers, XXXV, Buch I, S. 106 bis 109, ist die Erzielung von videotauglichen Schaltzeiten insbesondere beim Schalten von Graustufen immer noch ein noch nicht zufriedenstellend gelöstes Problem.

ECB-Anzeigen verwenden wie ASV-Anzeigen flüssigkristalline Medien mit negativer dielektrischer Anisotropie (Δε), wohingegen TN- und bislang alle gebräuchlichen IPS-Anzeigen flüssigkristalline Medien mit positiver dielektrischer Anisotropie verwenden.

In allen genannten Flüssigkristallanzeigen werden die Flüssigkristalle als Dielektrika verwendet, deren optische Eigenschaften sich bei Anlegen einer elektrischen Spannung reversibel ändern.

Da bei Anzeigen im Allgemeinen, also auch bei Anzeigen nach diesen erwähnten Effekten, die Betriebsspannung möglichst gering sein soll, werden Flüssigkristallmedien eingesetzt, die in der Regel überwiegend aus Flüssigkristallverbindungen zusammengesetzt sind, die alle das gleiche Vorzeichen der dielektrischen Anisotropie aufweisen und einen möglichst großen Betrag der dielektrischen Anisotropie haben. Es werden in der Regel allenfalls geringere Anteile an neutralen Verbindungen und möglichst keine Verbindungen mit einem Vorzeichen der dielektrischen Anisotropie, das dem des Mediums entgegengesetzt ist, eingesetzt.

Für viele praktische Anwendungen in Flüssigkristallanzeigen sind die bekannten Flüssigkristallmedien nicht stabil genug. Insbesondere ihre Stabilität gegen die Bestrahlung mit UV, aber auch bereits mit den üblichen Hintergrundbeleuchtungen führt zu einer Verschlechterung insbesondere der elektrischen Eigenschaften. So nimmt z. B. die Leitfähigkeit signifikant zu.

Zur Stabilisierung von Flüssigkristallmischungen wurde bereits die Verwendung von sogenannten "Hindered Amine Light Stabilisators", kurz HALS, vorgeschlagen, wie beispielsweise in EP 2 722 381 oder DE 10 2011 013007.

In DE 102011117937.6 werden Flüssigkristallmischungen mit positiver dielektrischer Anisotropie, die TINUVIN 770® zur Stabilisierung enthalten, beschrieben.

Die DE 102011119144.9 und WO 2012/076104 beschreiben Flüssigkristallmischungen mit negativer dielektrischer Anisotropie, die, unter anderem, HALS-N-Oxide zur Stabilisierung enthalten.

Nematische Flüssigkristallmischungen mit negativer dielektrische Anisotropie, die eine geringe Menge an TINUVIN^{®}770, einer Verbindung der Formel 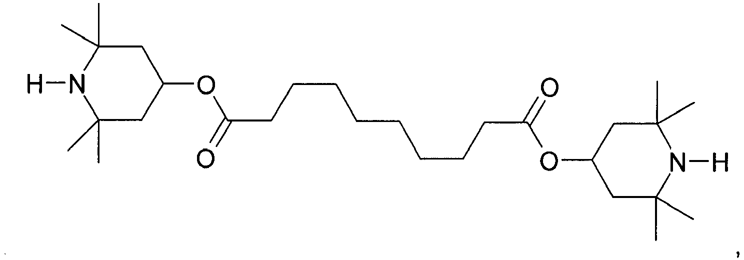 als Stabilisatoren enthalten, werden z.B. in WO 2009/129911 A1 vorgeschlagen. Die entsprechenden Flüssigkristallmischungen haben jedoch für einige praktische Anwendungen in einigen Fällen nicht ausreichende Eigenschaften. Unter anderem sind sie manchmal nicht genügend stabil gegen die Belastung durch die Bestrahlung mit typischen CCFL-((Cold

Die Verwendung verschiedener Stabilisatoren in flüssigkristallinen Medien wird z. B. in JP (S)55-023169 (A), JP (H)05-117324 (A), WO 02/18515 A1 und JP (H) 09-291282 (A) beschrieben.

Auch TINUVIN^{®}123, eine Verbindung der Formel 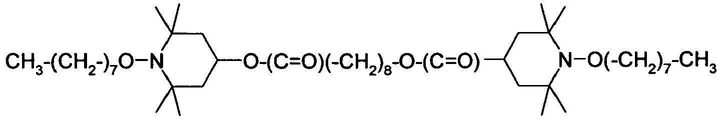 wurde zu Stabilisierungszwecken vorgeschlagen.

Mesogene Verbindungen mit einer oder zwei HALS-Einheiten werden in EP 1 1784 442 A1 offenbart.

HALS mit verschiedenen Substituenten am Stickstoffatom werden in Ohkatsu, Y., J. of Japan Petroleum Institute, 51, 2008, Seiten 191-204 bezüglich ihrer pK_{B}-Werte verglichen. Dabei werden die folgenden Typen von Strukturformeln offenbart.

| Typ | Aktive Gruppe des Stabilisators |
|---|---|
| "HALS" | 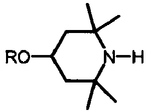 |
| "R-HALS" oder "NR-HALS" | 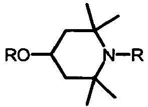 |
| "NOR-HALS" | 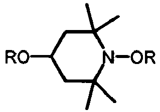 |

Die Verbindung TEMPOL der folgenden Formel 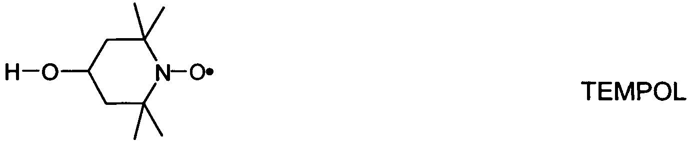 ist bekannt, sie wird z.B. in Miéville, P. et al, Angew. Chem. 2010, 122, Seiten 6318-6321 erwähnt. Sie ist von verschiedenen Herstellern kommerziell erhältlich und wird z. B. in Formulierungen für Vorläufer für Polyolefine, Polystyrole, Polyamide, Beschichtungen und PVC als Polymerisationsinhibitor und, insbesondere in Kombination mit UV-Absorbern, als Licht- bzw. UV-Schutz eingesetzt.

Die Flüssigkristallmedien des Standes der Technik mit entsprechend niedrigen Ansteuerspannungen haben relativ geringe elektrische Widerstände bzw. eine geringe VHR und führen in den Anzeigen oft zu unerwünschtem "Flicker" und/oder ungenügender Transmission. Außerdem sind sie nicht ausreichend stabil gegen Temperatur- und/oder UV-Belastung, zumindest dann, wenn sie eine entsprechend hohe Polarität aufweisen, wie sie für niedrige Ansteuerspannungen nötig ist.

Andererseits ist die Ansteuerspannung der Anzeigen des Standes der Technik, die eine hohe VHR aufweisen, oft zu groß, insbesondere für Anzeigen die nicht direkt oder nicht durchgehend an das Stromversorgungsnetz angeschlossen werden wie z. B. Anzeigen für mobile Anwendungen.

Außerdem muss der Phasenbereich der Flüssigkristallmischung ausreichend breit für die beabsichtigte Anwendung der Anzeige sein.

Die Schaltzeiten der Flüssigkristallmedien in den Anzeigen müssen verbessert, also verringert, werden. Dies ist besonders für Anzeigen für Fernseh- oder Multi-Media Anwendungen wichtig. Zur Verbesserung der Schaltzeiten ist in der Vergangenheit wiederholt vorgeschlagen worden, die Rotationsviskosität der Flüssigkristallmedien (γ₁) zu optimieren, also Medien mit einer möglichst geringen Rotationsviskosität zu realisieren. Die dabei erzielten Ergebnisse sind jedoch nicht ausreichend für viele Anwendungen und lassen es daher wünschenswert erscheinen, weitere Optimierungsansätze aufzufinden.

Ganz besonders wichtig ist eine ausreichende Stabilität der Medien gegen extreme Belastungen, insbesondere gegen UV- und Temperaturbelastung.

Besonders bei Anwendungen in Anzeigen in mobilen Geräten wie z. B. Mobiltelefonen kann dieses entscheidend sein.

Der Nachteil der bisher bekannten MFK-Anzeigen beruht auf ihrem vergleichsweise niedrigen Kontrast, der relativ hohen Blickwinkelabhängigkeit und der Schwierigkeit in diesen Anzeigen Graustufen zu erzeugen, sowie ihrer ungenügenden VHR und ihrer ungenügenden Lebensdauer.

Es besteht somit immer noch ein großer Bedarf nach MFK-Anzeigen mit sehr hohem spezifischen Widerstand bei gleichzeitig großem Arbeitstemperaturbereich, kurzen Schaltzeiten und niedriger Schwellenspannung, mit deren Hilfe verschiedene Graustufen erzeugt werden können und die insbesondere eine gute und stabile VHR aufweisen.

Der Erfindung liegt die Aufgabe zugrunde MFK-Anzeigen, nicht nur für Monitor- und TV-Anwendungen, sondern auch für Mobiltelefone und Navigationssysteme bereitzustellen, die die oben angegebenen Nachteile nicht oder nur in geringerem Maße und gleichzeitig sehr hohe spezifische Widerstände aufweisen. Insbesondere muss für Mobiltelefone und Navigationssysteme gewährleistet sein, dass diese auch bei extrem hohen und extrem niedrigen Temperaturen arbeiten.

Überraschenderweise wurde nun gefunden, dass flüssigkristalline Medien mit einem geeigneten Δε, einem geeigneten Phasenbereich und Δn verwirklicht werden können, welche die Nachteile der Materialien des Standes der Technik nicht oder zumindest nur in erheblich geringerem Maße aufweisen.

Hier wurde überraschend gefunden, dass die Verbindungen der Formel I, auch wenn sie alleine ohne zusätzliche Temperaturstabilisatoren verwendet werden, zu einer erheblichen, in vielen Fällen ausreichenden, Stabilisierung von Flüssigkristallmischungen sowohl gegen UV-Belastung, als auch gegen Temperaturbelastung führen.

Die Erfindung betrifft somit Verbindungen der Formel I,

Stabilisierung von Flüssigkristallmischungen sowohl gegen UV-Belastung, als auch gegen Temperaturbelastung führen.

Die Erfindung betrifft somit Verbindungen der Formel I, 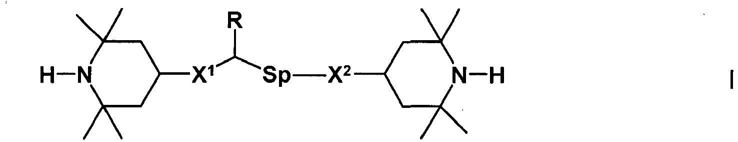 worin
- X¹ und X²: jeweils unabhängig voneinander -O-, -(CO)-O-, -O-(CO)-, -O-(CO)-O-, -NH- oder -NH-(CO)-, bevorzugt -O-,-(CO)-O-, -O-(CO)-,
- Sp: geradkettiges Alkyl mit 1 bis 20 C-Atomen, bevorzugt mit 1 bis 15 C-Atomen und besonders bevorzugt mit 1 bis 10 C-Atomen, auch Cycloalkyl, Cycloalkylalkyl, oder Alkylcycloalkyl, wobei in allen diesen Gruppen eine oder mehrere -CH₂- Gruppen durch -O- so ersetzt sein können, dass im Molekül keine zwei O-Atome direkt miteinander verbunden sind, und
- R: geradkettiges oder verzweigtes Alkyl mit 1 bis 10 C-Atomen auch Cycloalkyl, Cycloalkylalkyl, oder Alkylcycloalkyl, wobei in allen diesen Gruppen eine oder mehrere -CH₂- Gruppen durch -O- so ersetzt sein können, dass im Molekül keine zwei O-Atome direkt miteinander verbunden sind, und , bevorzugt geradkettiges oder verzweigtes Alkyl mit 1 bis 7 C-Atomen und besonders bevorzugt Alkyl mit 1 bis 4 C-Atomen,
bedeuten.

Bevorzugte Verbindungen der Formel I werden nachfolgend genannt: 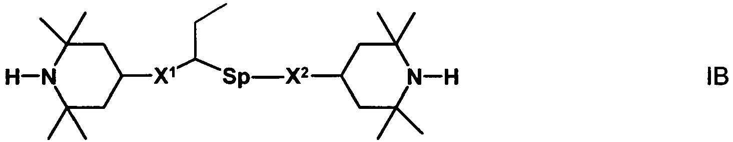 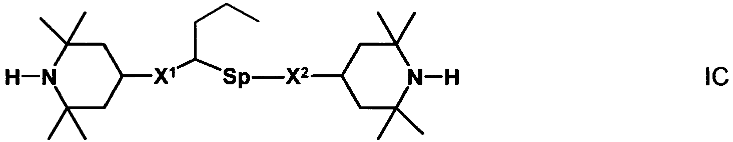 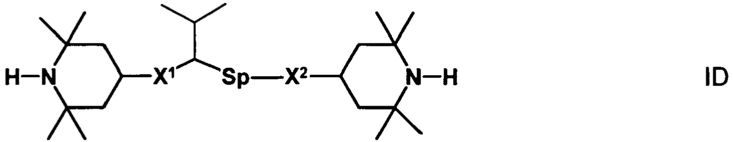 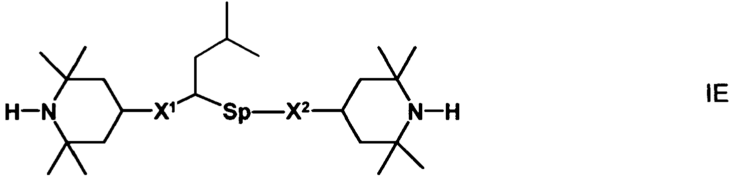 worin X¹, X² und Sp eine der unter Formel I angegebenen Bedeutungen haben.

Ferner bevorzugte Verbindungen der Formel I sind ausgewählt aus den folgenden Verbindungen: 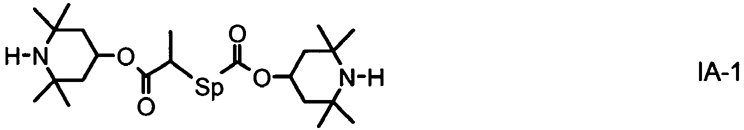 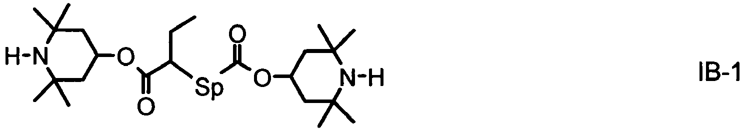 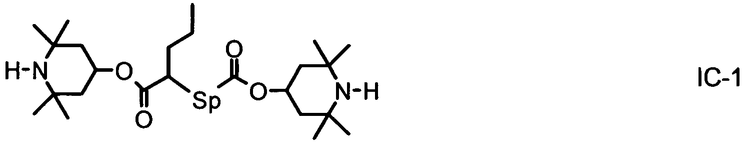 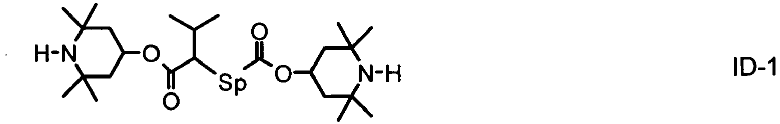 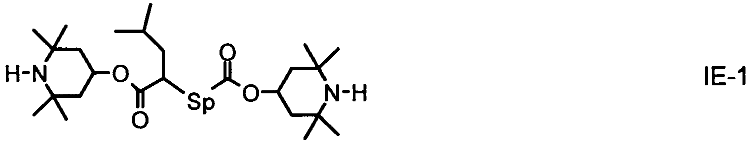 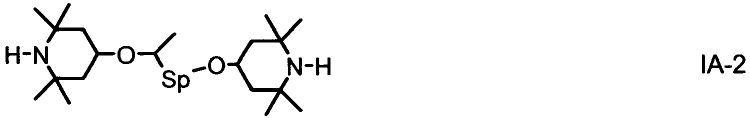 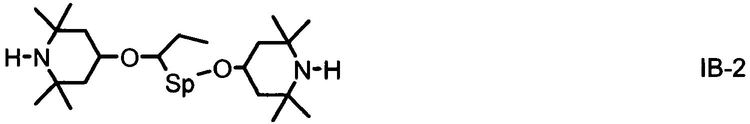 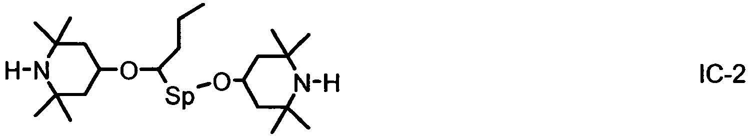 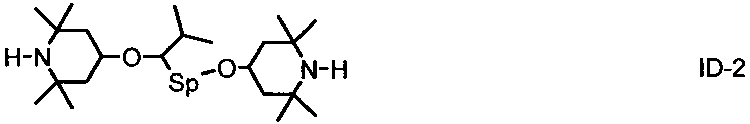 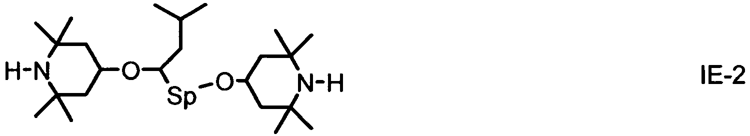 worin Sp eine der unter Formel I angegebenen Bedeutung hat. Falls in den obenstehenden Formeln Sp einen Alkylrest bedeutet, so kann dieser geradkettig oder verzweigt sein. Vorzugsweise ist er geradkettig, und hat 1, 2, 3, 4, 5, 6, 7, 8, 9, oder 10 C-Atom(e) und bedeutet demnach bevorzugt Methyl, Ethyl, Propyl, Butyl, Pentyl, Hexyl, Heptyl, Octyl, Nonyl oder Decyl.

Insbesondere sind Verbindungen der Formel I bevorzugt ausgewählt aus den folgenden Verbindungen:

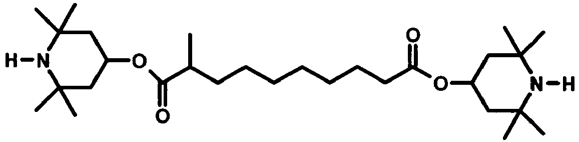

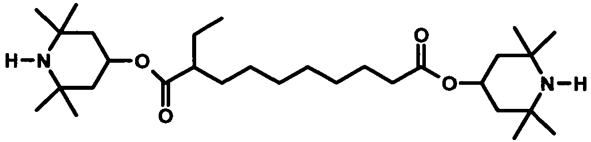

Die Verbindungen der Formel I sind in reinem Zustand farblos und werden nach an sich bekannten Methoden dargestellt, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Gegenstand der vorliegenden Erfindung ist auch ein flüssigkristallines Medium mit einer nematischen Phase, welches eine oder mehrere Verbindungen der Formel I enthält, bevorzugt in einer Konzentration im Bereich von 1 ppm bis zu 5.000 ppm, bevorzugt im Bereich von 1 ppm bis zu 2.000 ppm, besonders bevorzugt im Bereich von 1 ppm bis zu 1.000 ppm, insbesondere 1 ppm bis zu 500 ppm.

Die Verbindungen der Formel I führen zu LC-Mischungen mit den oben angegebenen gewünschten Eigenschaften, insbesondere zu LC-Mischungen mit besonders niedrige Schaltzeiten bei gleichzeitig hohem Spannungshaltevermögen (Englisch "voltage holding ratio", kurz VHR oder auch nur HR) aus. Weiterhin sind die Verbindungen der Formel I sehr gut in flüssigkristallinen Medien löslich.

Gegenstand der vorliegenden Erfindung sind auch elektrooptische Anzeigen oder elektrooptische Komponenten, die erfindungsgemäße flüssigkristalline Medien enthalten.

Dementsprechend ist die Verwendung eines erfindungsgemäßen flüssigkristallinen Mediums in einer elektrooptischen Anzeige oder in einer elektrooptischen Komponente ebenfalls Gegenstand der vorliegenden Erfindung, ebenso wie ein Verfahren zur Herstellung der erfindungsgemäßen flüssigkristallinen Medien, dadurch gekennzeichnet, dass eine oder mehrere Verbindungen der Formel I mit einer oder mehreren Verbindungen der nachfolgenden Verbindungen gemischt wird.

Außerdem betrifft die vorliegenden Erfindung ein Verfahren zur Stabilisierung eines flüssigkristallinen Mediums mit nematischer Phase, das eine oder mehrere Verbindungen der der nachfolgenden Verbindungen enthält, dadurch gekennzeichnet, dass dem Medium eine oder mehrere Verbindungen der Formel I zugesetzt werden.

Hierbei bedeutet der Begriff "eine nematische Phase aufweisen" einerseits, dass bei tiefen Temperaturen bei der entsprechenden Temperatur keine smektische Phase und keine Kristallisation beobachtet wird und andererseits, dass beim Aufheizen aus der nematischen Phase noch keine Klärung auftritt. Die Untersuchung bei tiefen Temperaturen wird in einem Fließviskosimeter bei der entsprechenden Temperatur durchgeführt sowie durch Lagerung in Testzellen einer der elektrooptischen Anwendung entsprechenden Schichtdicke für mindestens 100 Stunden überprüft. Wenn die Lagerstabilität bei einer Temperatur von -20°C in einer entsprechenden Testzelle 1.000 h oder mehr beträgt, wird das Medium als bei dieser Temperatur stabil bezeichnet. Bei Temperaturen von -30°C bzw. -40°C betragen die entsprechenden Zeiten 500 h bzw. 250 h. Bei hohen Temperaturen wird der Klärpunkt nach üblichen Methoden in Kapillaren gemessen.

Bevorzugte Verbindungen die in einem erfindungsgemäßen flüssigkristallinen Medium eingesetzt werden können sind im Folgenden angegeben:
- Das Medium enthält eine oder mehrere neutrale Verbindungen der Formeln II und/oder III, 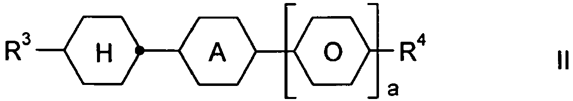 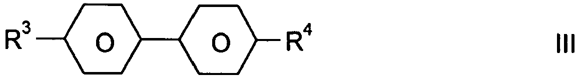 worin
   - A: 1,4-Phenylen oder trans-1,4-Cyclohexylen bedeutet,
   - a: 0 oder 1 ist,
   - R³: Alkenyl mit 2 bis 9 C-Atomen bedeutet, und
   - R⁴: einen Alkyl- oder Alkoxyrest mit 1 bis 15 C-Atomen, wobei in diesen Resten auch eine oder mehrere CH₂-Gruppen jeweils unabhängig voneinander durch -C≡C-, -CF₂O-, -CH=CH-, 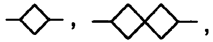
   -O-, -CO-O-, -O-CO- so ersetzt sein können, dass O-Atome nicht direkt miteinander verknüpft sind, und worin auch ein oder mehrere H-Atome durch Halogen ersetzt sein können, vorzugsweise Alkyl mit 1 bis 12 C-Atomen oder Alkenyl mit 2 bis 9 C-Atomen bedeutet.
- Die Verbindungen der Formel II sind vorzugsweise ausgewählt aus folgenden Formeln, 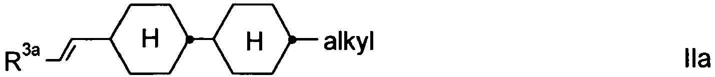 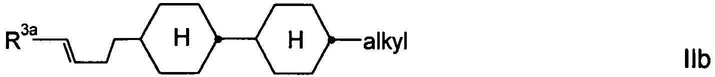 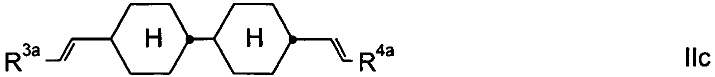 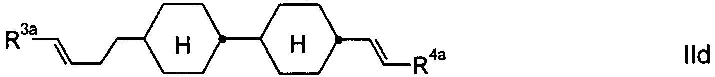 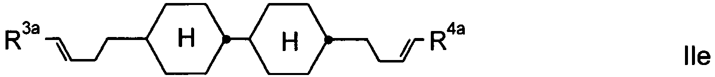 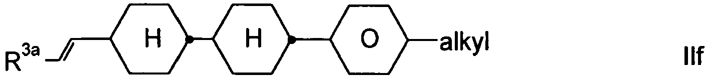 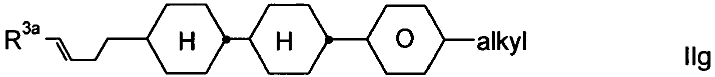 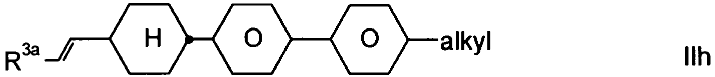 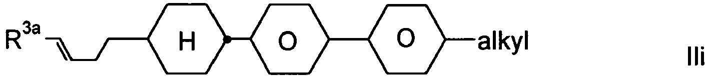 worin R^{3a} und R^{4a} jeweils unabhängig voneinander H, CH₃, C₂H₅ oder C₃H₇ bedeuten, und "alkyl" eine geradkettige Alkylgruppe mit 1 bis 8 C-Atomen bedeutet. Besonders bevorzugt sind Verbindungen der Formeln IIa und IIf, insbesondere worin R^{3a} H oder CH₃ bedeutet, und Verbindungen der Formel IIc, insbesondere worin R^{3a} und R^{4a} H, CH₃ oder C₂H₅ bedeuten.
   Weiterhin sind Verbindungen der Formel II bevorzugt, die eine nichtendständige Doppelbindung in der Alkenylseitenkette aufweisen: 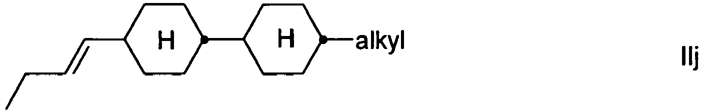 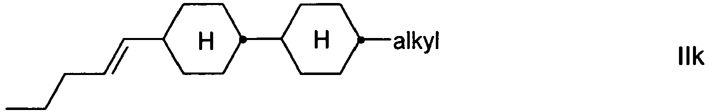 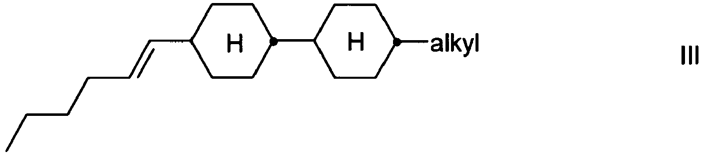 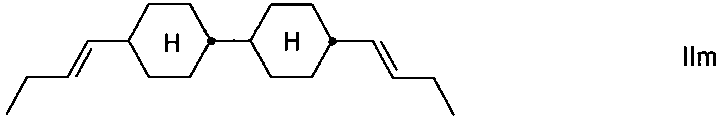 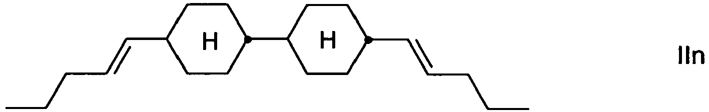 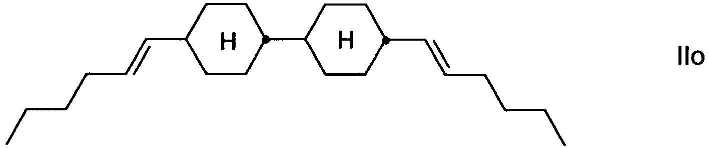 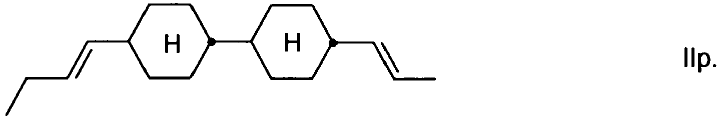
   Ganz besonders bevorzugte Verbindungen der Formel II sind die Verbindungen der Formeln 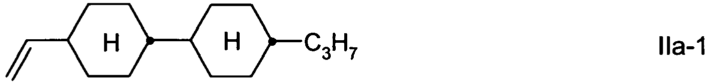 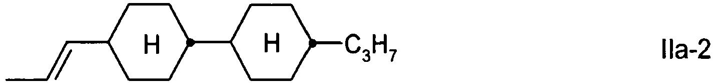 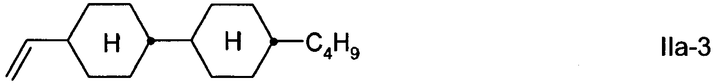 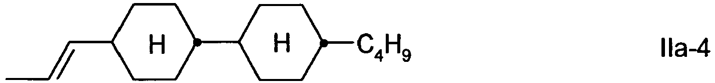 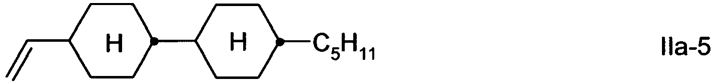 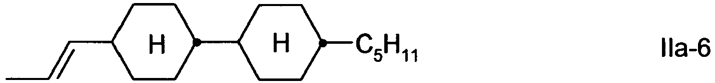 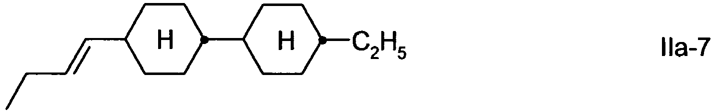 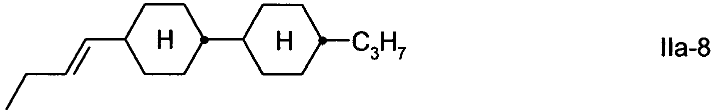 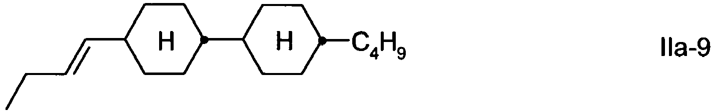 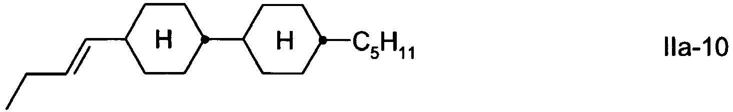 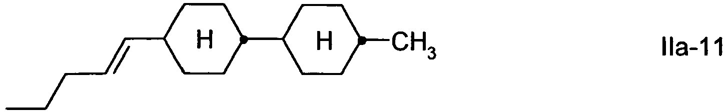 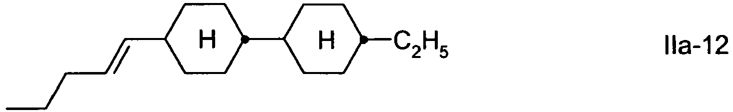 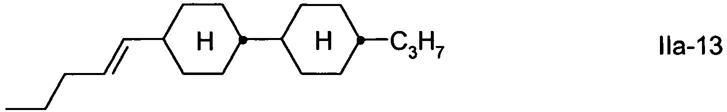 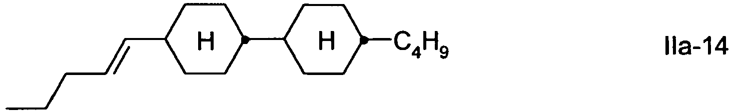 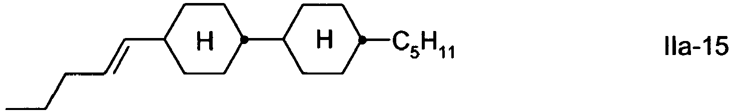 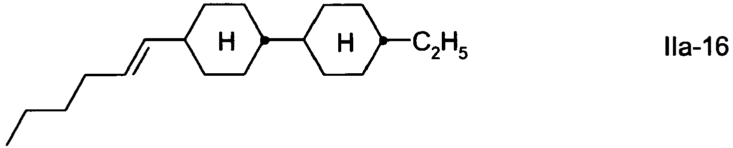 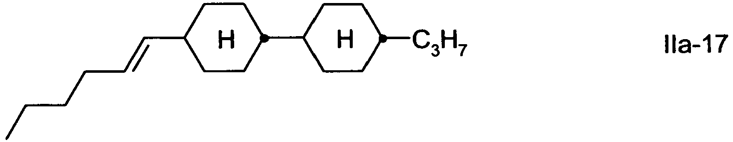 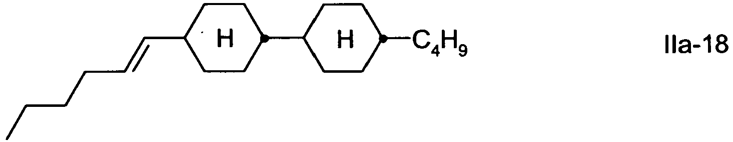 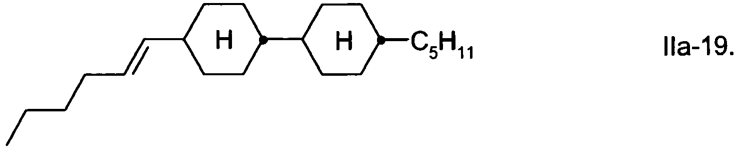
   Von den Verbindungen der Formeln IIa-1 bis IIa-19 sind inbesondere die Verbindungen der Formeln IIa-1, IIa-2, IIa-3 und IIa-5 besonders bevorzugt.
   Besonders bevorzugt enthalten die erfindungsgemäßen flüssigkristallinen Medien neben einer oder mehrerer Verbindungen der Formel I 5 - 70 Gew.%, insbesondere 10 - 50 Gew.% und ganz besonders bevorzugt 15 - 40 Gew.%, an Verbindungen der Formel 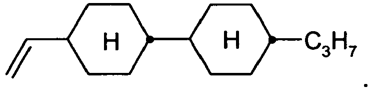
- Die Verbindungen der Formel III sind vorzugsweise ausgewählt aus den folgenden Formeln, 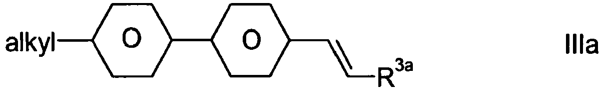 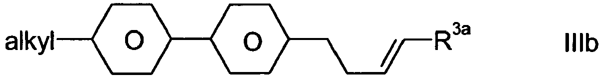 worin "Alkyl" und R^{3a} die oben angegebenen Bedeutungen haben und R^{3a} vorzugsweise H oder CH₃ bedeutet. Besonders bevorzugt sind Verbindungen der Formel IIIb;
   Ganz besonders bevorzugt ist die Verbindung der Formel IIIb-1, 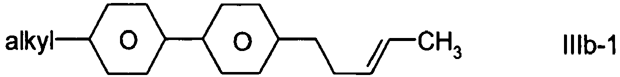 worin "alkyl" die oben angegebene Bedeutung hat und vorzugsweise CH₃, ferner C₂H₅ oder n-C₃H₇, bedeutet.
- Das Medium enthält vorzugsweise eine oder mehrere Verbindungen ausgewählt aus folgenden Formeln IV bis VIII, 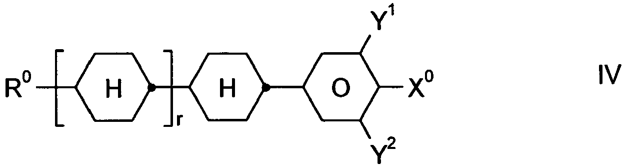 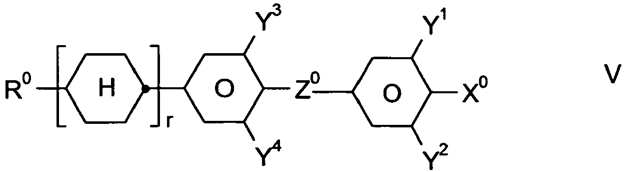 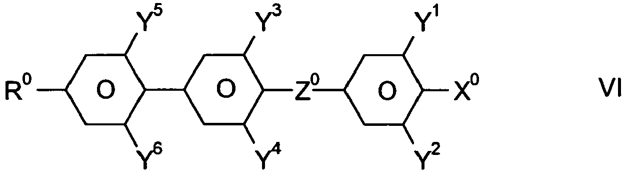 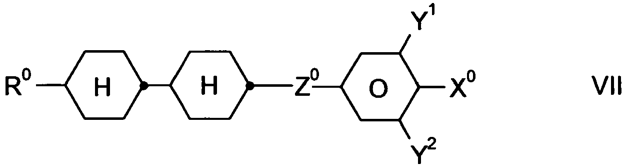 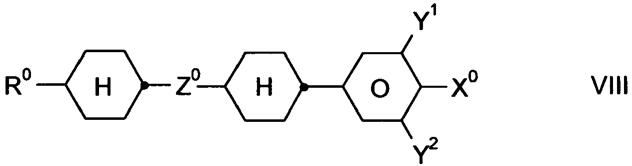 worin
   - R⁰: einen Alkyl- oder Alkoxyrest mit 1 bis 15 C-Atomen, wobei in diesen Resten auch eine oder mehrere CH₂-Gruppen jeweils unabhängig voneinander durch -C≡C-, -CF₂O-, -CH=CH-, 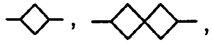 -O-, -CO-O- oder -O-CO- so ersetzt sein können, dass O-Atome nicht direkt miteinander verknüpft sind, und worin auch ein oder mehrere H-Atome durch Halogen ersetzt sein können,
   - X⁰: F, Cl, ein- oder mehrfach fluorierter Alkyl- oder Alkoxyrest mit jeweils 1 bis 6 C-Atomen, ein- oder mehrfach fluorierter Alkenyl- oder Alkenyloxyrest mit jeweils 2 bis 6 C-Atomen.
   - Y¹⁻⁶: jeweils unabhängig voneinander H oder F,
   - Z⁰: -C₂H₄-, -(CH₂)₄-, -CH=CH-, -CF=CF-, -C₂F₄-, -CH₂CF₂-, -CF₂CH₂-, -CH₂O-, -OCH₂-, -COO-,-CF₂O- oder -OCF₂-, in den Formeln V und VI auch eine Einfachbindung, und
   - r: 0 oder 1
   bedeuten.
   In den obenstehenden Formeln ist X⁰ vorzugsweise F, Cl oder ein ein- oder mehrfach fluorierter Alkyl- oder Alkoxyrest mit 1, 2 oder 3 C-Atomen oder ein ein- oder mehrfach fluorierter Alkenylrest bzw. Alkenyloxyrest mit 2 oder 3 C-Atomen. X⁰ ist besonders bevorzugt F, Cl, CF₃, CHF₂, OCF₃, OCHF₂, OCHFCF₃, OCHFCHF₂, OCHFCH₂F, OCF₂CH₃, OCF₂CHF₂, OCF₂CH₂F, OCF₂CF₂CHF₂, OCF₂CF₂CH₂F, OCFHCF₂CF₃, OCFHCF₂CHF₂, OCH=CF₂, OCF=CF₂, OCF₂CHFCF₃, OCF₂CF₂CF₃, OCF₂CF₂CClF₂, OCClFCF₂CF₃, CF=CF₂, CF=CHF, OCH=CF₂, OCF=CF₂, oder CH=CF₂.
   In den Verbindungen der Formel IV bis VIII bedeutet X⁰ vorzugsweise F oder OCF₃, ferner OCHF₂, CF₃, CF₂H, Cl, OCH=CF₂. R⁰ ist vorzugsweise geradkettiges Alkyl oder Alkenyl mit bis zu 6 C-Atomen.
- Die Verbindungen der Formel IV sind vorzugsweise ausgewählt aus
   den folgenden Formeln, 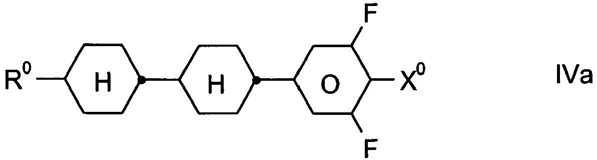 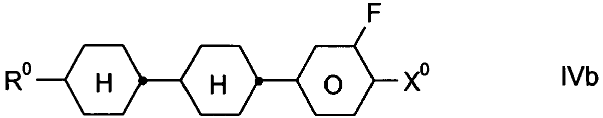 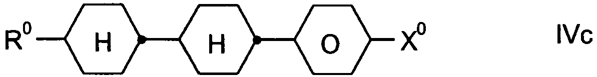 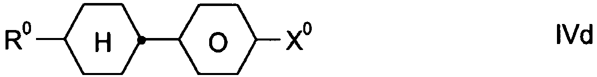 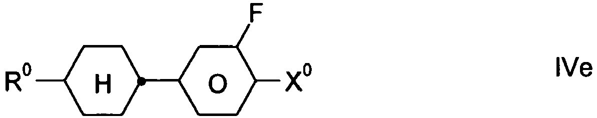 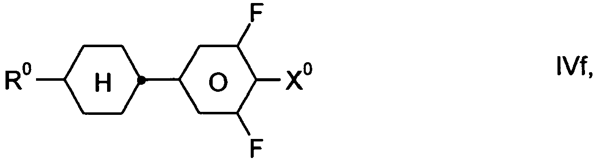 worin R⁰ und X⁰ die unter Formel IV angegebenen Bedeutungen haben.
   Vorzugsweise bedeutet in Formel IV R⁰ Alkyl mit 1 bis 8 C-Atomen und X⁰ F, Cl, OCHF₂ oder OCF₃, ferner OCH=CF₂. In der Verbindung der Formel IVb bedeutet R⁰ vorzugsweise Alkyl oder Alkenyl. In der Verbindung der Formel IVd bedeutet X⁰ vorzugsweise Cl, ferner F.
- Die Verbindungen der Formel V sind vorzugsweise ausgewählt aus den Formeln Va bis Vj, 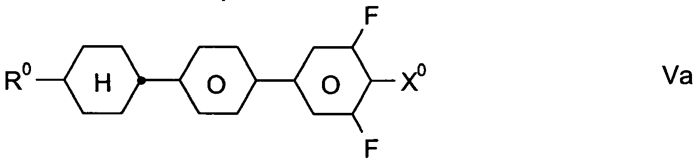 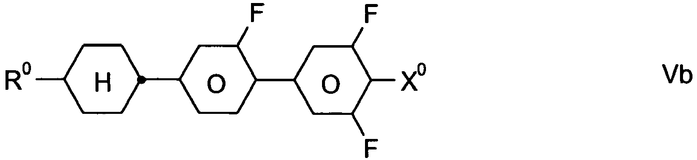 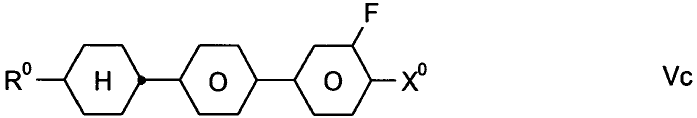 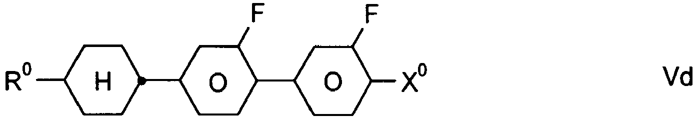 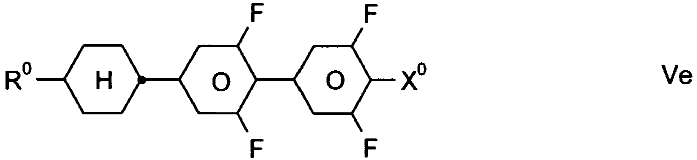 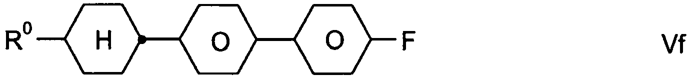 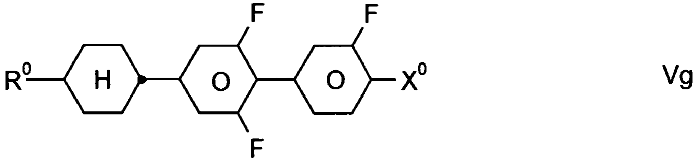 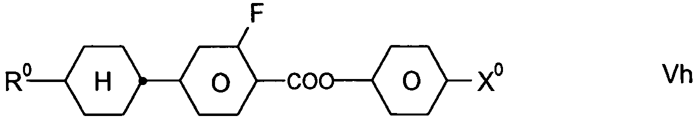 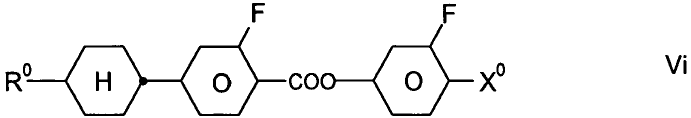 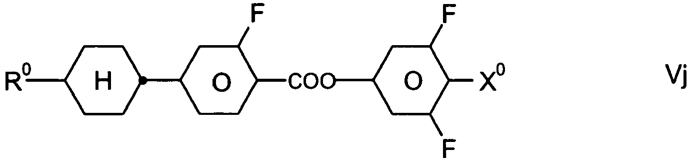 worin R⁰ und X⁰ die in Anspruch 7 angegebenen Bedeutungen haben. Vorzugsweise bedeutet in Formel V R⁰ Alkyl mit 1 bis 8 C-Atomen und X⁰ F, OCF₃ oder OCH=CF₂.
- Das Medium enthält eine oder mehrere Verbindungen der Formel VI-1, 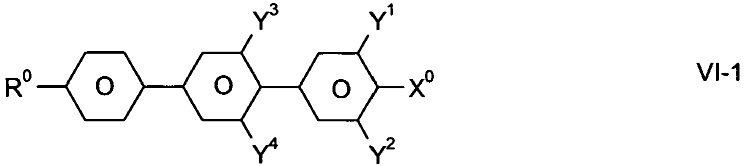 besonders bevorzugt solche ausgewählt aus den folgenden Formeln, 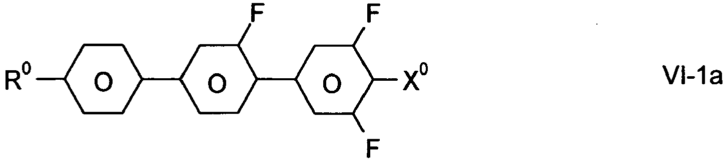 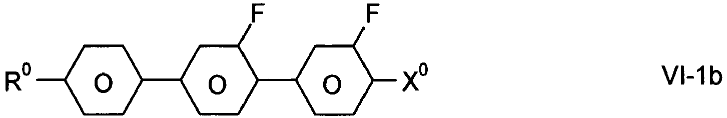 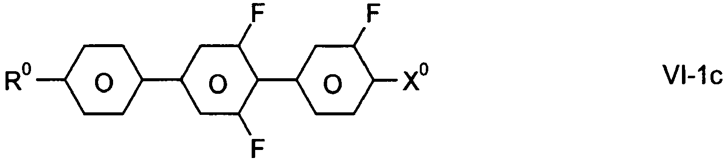 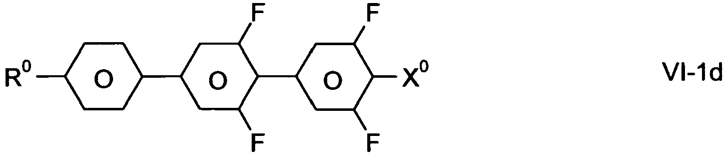 worin R⁰ und X⁰ die in Anspruch 7 angegebenen Bedeutungen haben. Vorzugsweise bedeutet in Formel VI R⁰ Alkyl mit 1 bis 8 C-Atomen und X⁰ F, ferner CF₃ und OCF₃.
- Das Medium enthält eine oder mehrere Verbindungen der Formel VI-2, 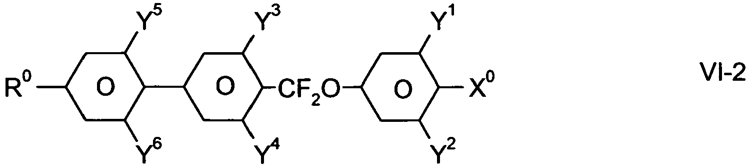 besonders bevorzugt solche ausgewählt aus den folgenden Formeln, 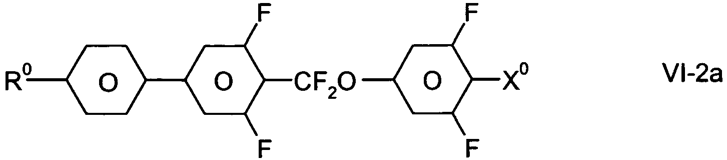 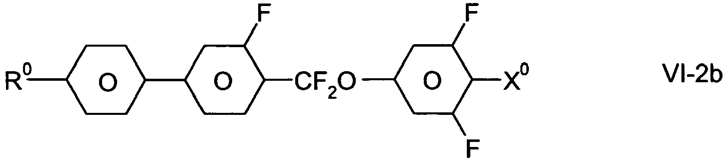 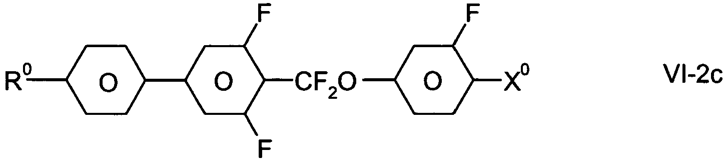 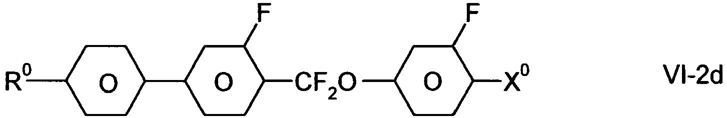 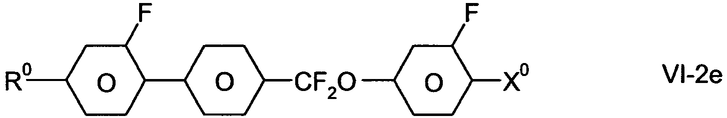 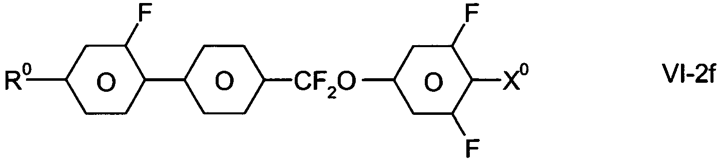 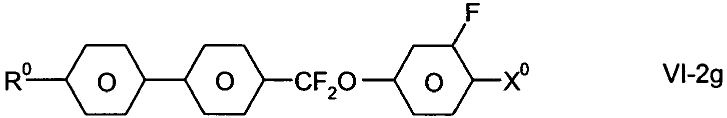 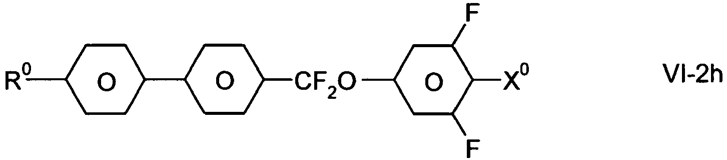 worin R⁰ und X⁰ die in Anspruch 7 angegebenen Bedeutungen haben. Vorzugsweise bedeutet in Formel VI R⁰ Alkyl mit 1 bis 8 C-Atomen und X⁰ F;
- Das Medium enthält vorzugsweise eine oder mehrere Verbindungen der Formel VII, worin Z⁰ -CF₂O-, -CH₂CH₂- oder -COO- bedeutet, besonders bevorzugt solche ausgewählt aus folgenden Formeln, 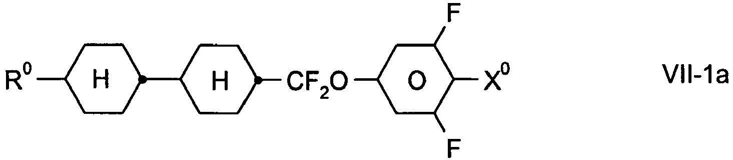 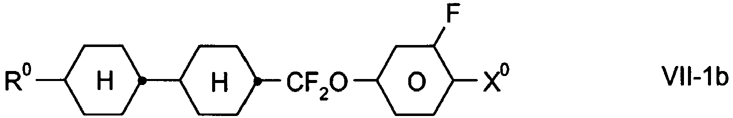 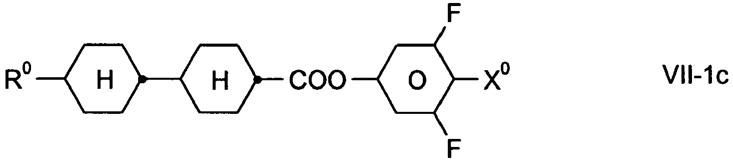 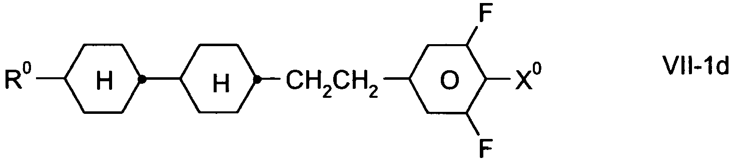 worin R⁰ und X⁰ die in Anspruch 7 angegebenen Bedeutungen haben. Vorzugsweise bedeutet in Formel VII R⁰ Alkyl mit 1 bis 8 C-Atomen und X⁰ F, ferner OCF₃ und CF₃
   Die Verbindungen der Formel VIII sind vorzugsweise ausgewählt aus den folgenden Formeln, 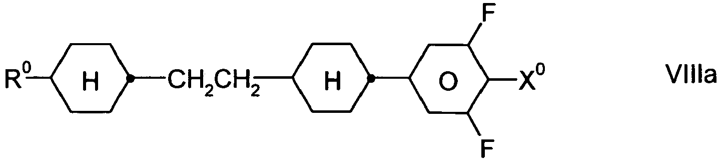 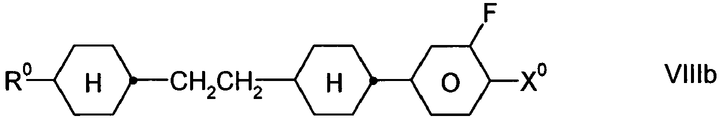 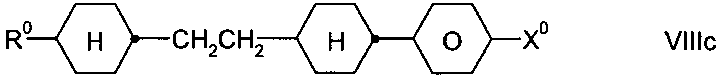 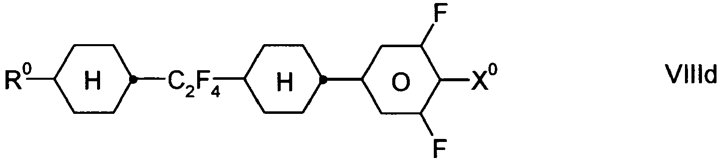 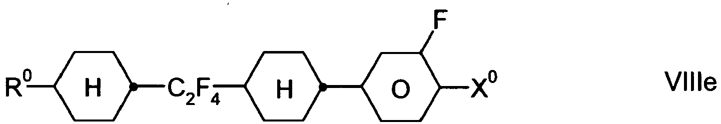 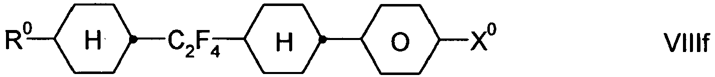 worin R⁰ und X⁰ die oben angegebenen Bedeutungen haben. Vorzugsweise bedeutet in Formel VIII R⁰ einen geradkettigen Alkylrest mit 1 bis 8 C-Atomen. X⁰ bedeutet vorzugsweise F.
- Das Medium enthält eine oder mehrere Verbindungen der folgenden Formel, 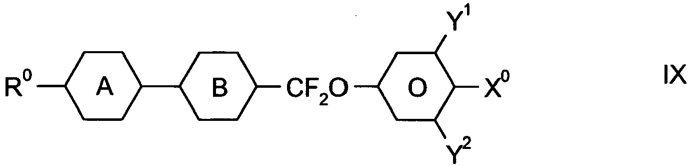 worin R⁰, X⁰, Y¹ und Y² die oben angegebene Bedeutung besitzen, und 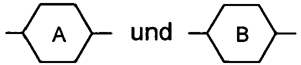 jeweils unabhängig voneinander 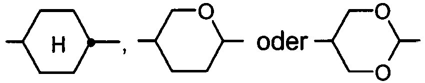 bedeuten,
   wobei die Ringe A und B nicht beide gleichzeitig 1,4-Cyclohexylen bedeuten;
- Die Verbindungen der Formel IX sind vorzugsweise ausgewählt aus folgenden Formeln, 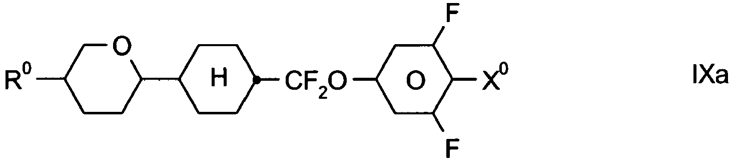 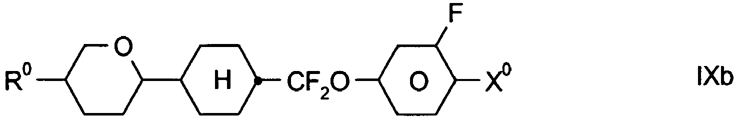 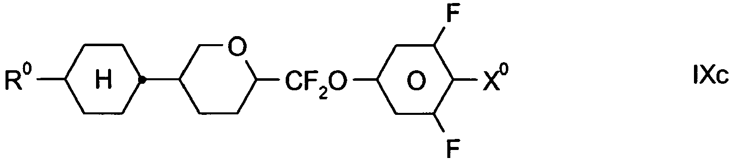 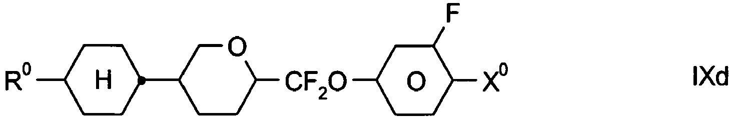 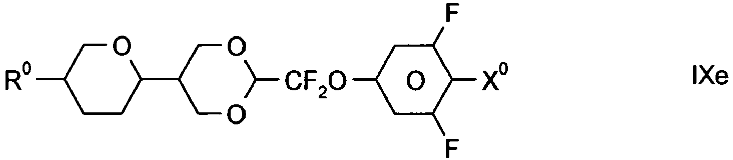 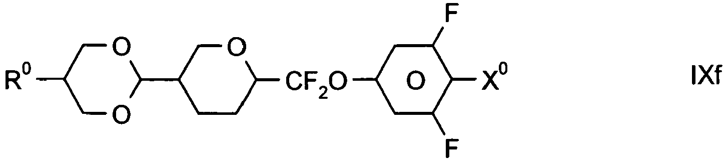 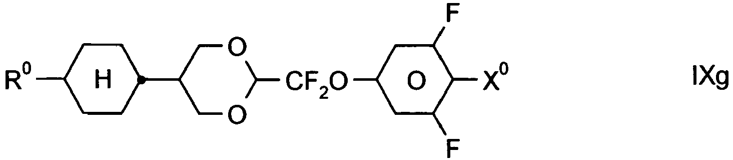 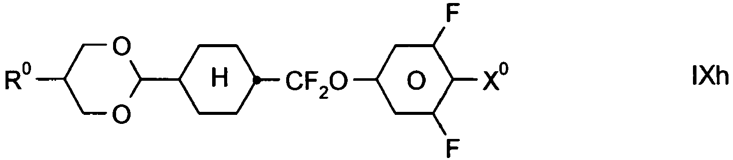 worin R⁰ und X⁰ die in Anspruch 7 angegebenen Bedeutungen haben. Vorzugsweise bedeutet in Formel IX R⁰ Alkyl mit 1 bis 8 C-Atomen und X⁰ F. Besonders bevorzugt sind Verbindungen der Formel IXa;
- Das Medium enthält eine oder mehrere Verbindungen ausgewählt aus den folgenden Formeln, 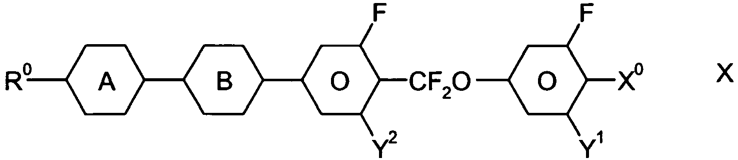 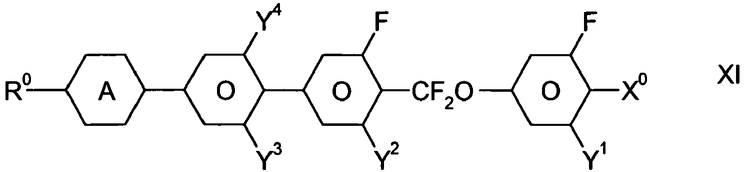 worin R⁰, X⁰ und Y¹⁻² die in Anspruch 7 angegebenen Bedeutungen besitzen, Y³ und Y⁴ jeweils unabhängig voneinander H oder F bedeuten, und 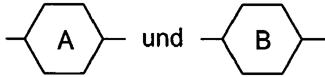 jeweils unabhängig voneinander 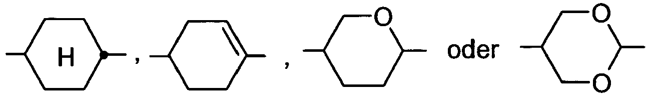 bedeuten;
- Die Verbindungen der Formeln X und XI sind vorzugsweise ausgewählt aus den folgenden Formeln, 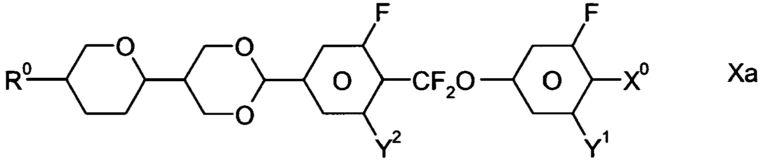 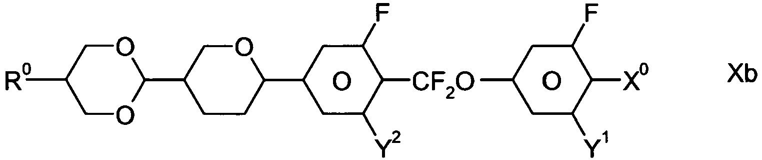 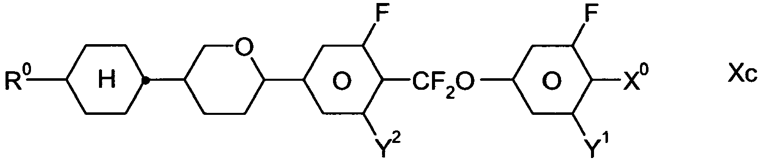 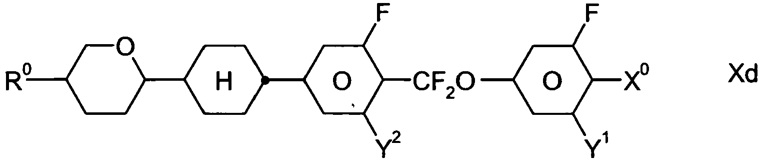 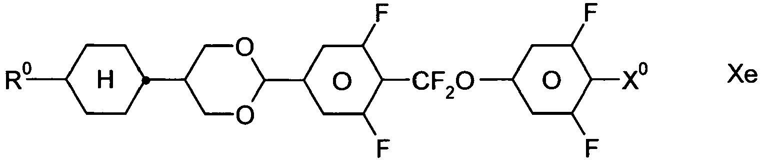 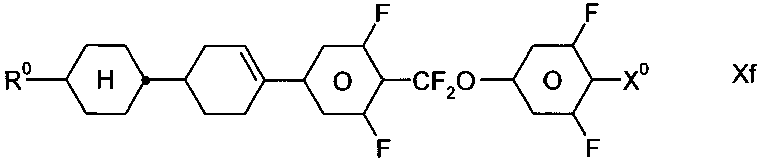 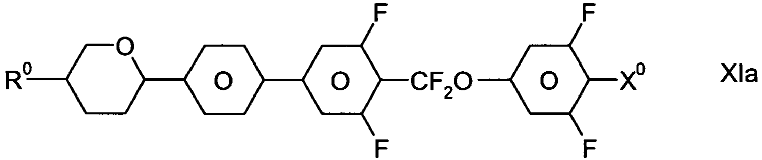 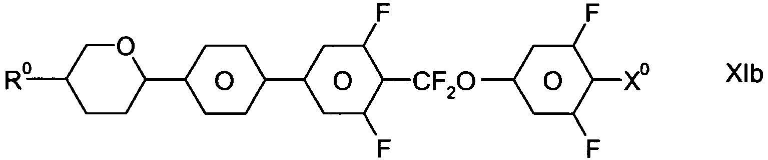 worin R⁰ und X⁰ die in Anspruch 7 angegebenen Bedeutungen haben. Vorzugsweise bedeutet R⁰ Alkyl mit 1 bis 8 C-Atomen und X⁰ F. Besonders bevorzugte Verbindungen sind solche, worin Y¹ F und Y² H oder F, vorzugsweise F, bedeuten;
- Das Medium enthält eine oder mehrere Verbindungen der folgenden Formel XII, 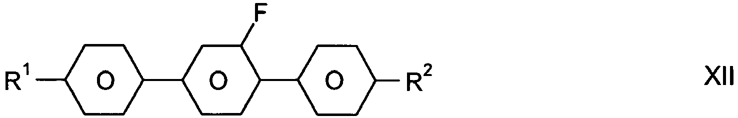 worin R¹ und R² jeweils unabhängig voneinander Alkyl, Alkenyl, Alkoxy, Oxaalkyl, Fluoralkyl oder Alkenyloxy mit jeweils bis zu 9 C-Atomen bedeuten, und vorzugsweise jeweils unabhängig voneinander Alkyl oder Alkenyl mit 1 bis 8 C-Atomen bzw. 2 bis 8 C-Atomen bedeuten.
   Bevorzugte Verbindungen der Formel XII sind die Verbindungen der Formel, 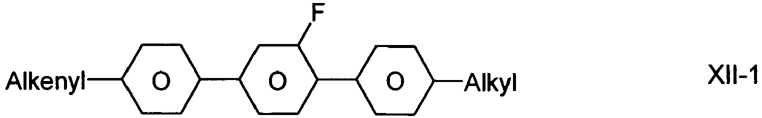 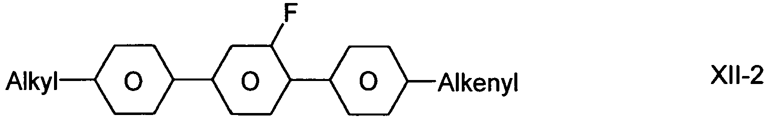 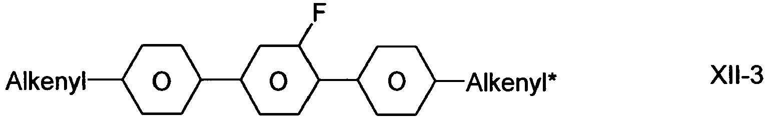 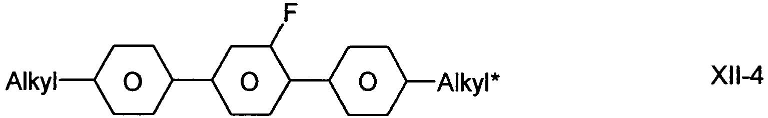 worin
   - Alkyl und Alkyl*: jeweils unabhängig voneinander einen geradkettigen Alkylrest mit 1 bis 8 C-Atomen, und
   - Alkenyl und Alkenyl*: jeweils unabhängig voneinander einen geradkettigen Alkenylrest mit 2 bis 8 C-Atomen
   bedeuten.
   Besonders bevorzugt sind die Verbindungen der Formeln XII-2 und XII-4.
   Besonders bevorzugte Verbindungen der Formel XII-2 sind die Verbindungen der Formel XII-2a, XII-2b und XII-2c: 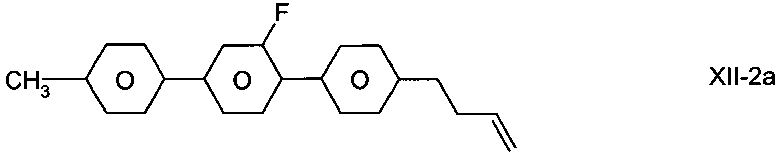 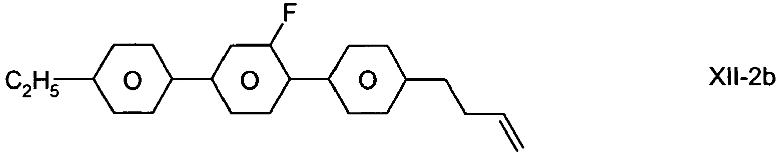 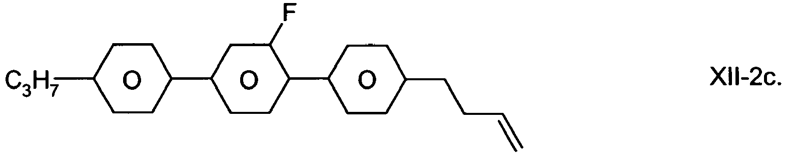
   Besonders bevorzugte Verbindungen der Formel XII-4 sind die Verbindungen der Formel XII-4a, XII-4b und XII-4c: 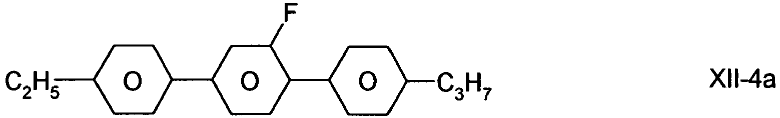 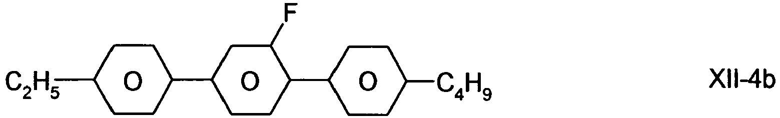 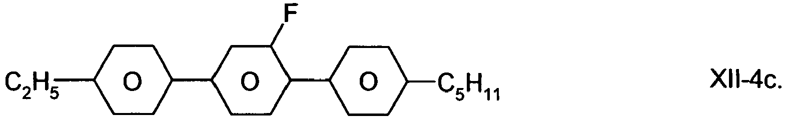
- Das Medium enthält eine oder mehrere Verbindungen ausgewählt aus folgenden Formeln, 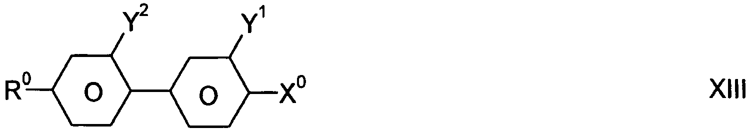 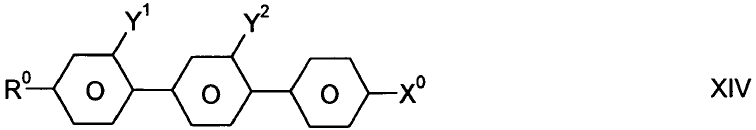 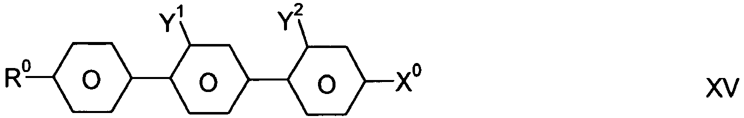 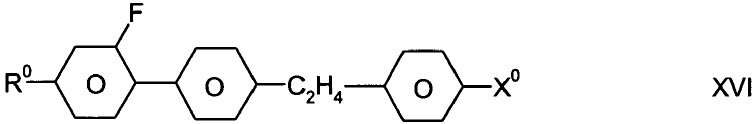 worin R⁰, X⁰, Y¹ und Y² die in Anspruch 7 angegebenen Bedeutungen haben. Vorzugsweise bedeutet R⁰ Alkyl mit 1 bis 8 C-Atomen und X⁰ F oder Cl;
- Die Verbindungen der Formeln XIII und XIV sind vorzugsweise ausgewählt aus den Verbindungen der Formeln, 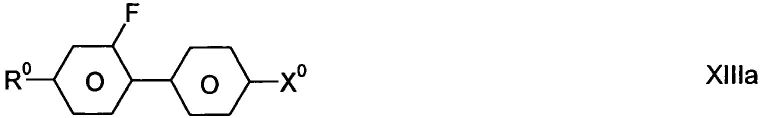 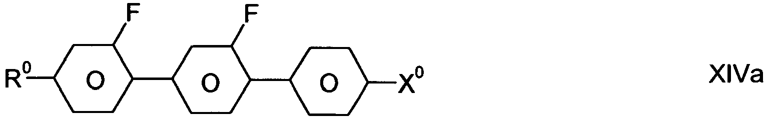 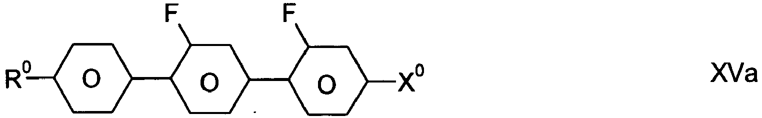 worin R⁰ und X⁰ die in Anspruch 7 angegebenen Bedeutungen haben. Vorzugsweise bedeutet R⁰ Alkyl mit 1 bis 8 C-Atomen. In den Verbindungen der Formel XIII bedeutet X⁰ vorzugsweise F oder Cl.
- Das Medium enthält eine oder mehrere Verbindungen der Formeln D1, D2, D3, D4 und/oder D5, 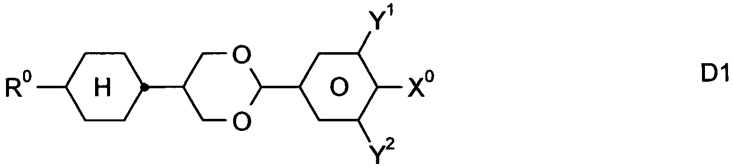 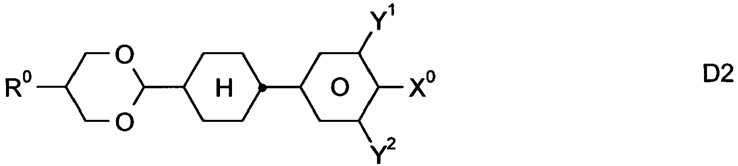 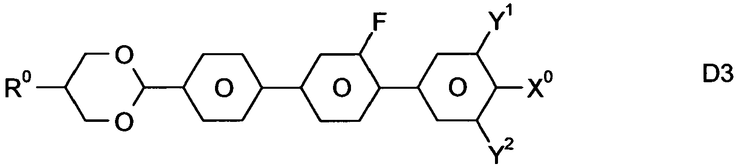 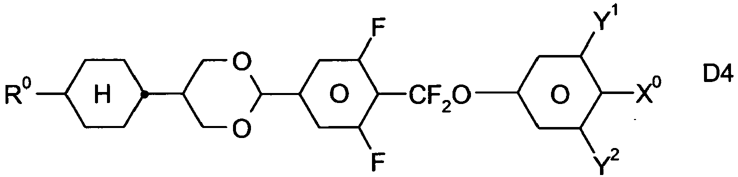 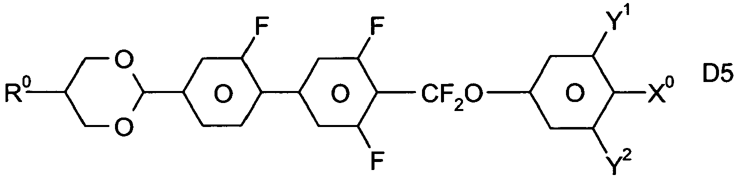 worin Y¹, Y², R⁰ und X⁰ die in Anspruch 7 angegebenen Bedeutungen besitzen. Vorzugsweise bedeutet R⁰ Alkyl mit 1 bis 8 C-Atomen und X⁰ F. Y¹ und Y² bedeuten vorzugsweise beide F.
   Besonders bevorzugt sind Verbindungen der Formeln, 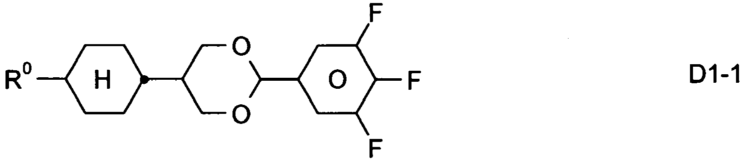 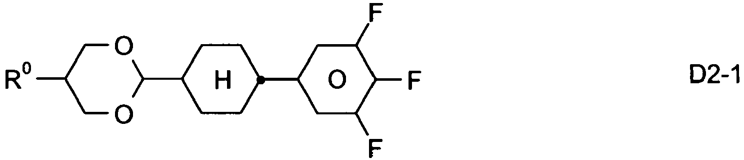 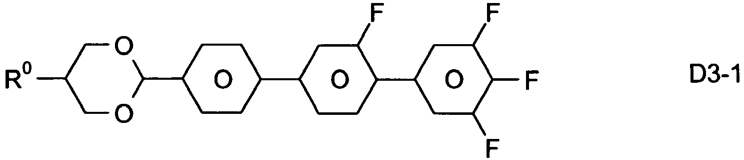 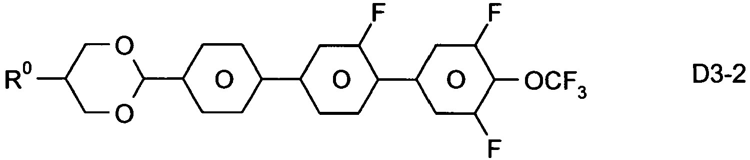 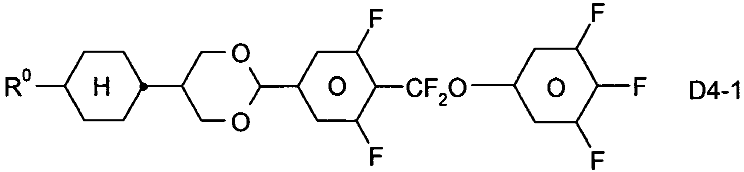 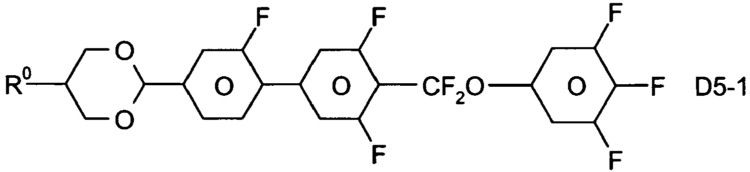 worin R⁰ die oben angegebenen Bedeutungen hat und vorzugsweise geradkettiges Alkyl mit 1 bis 6 C-Atomen, insbesondere C₂H₅, n-C₃H₇ oder n-C₅H₁₁ bedeutet.
- Das Medium enthält eine oder mehrere Verbindungen der folgenden Formel XVII, 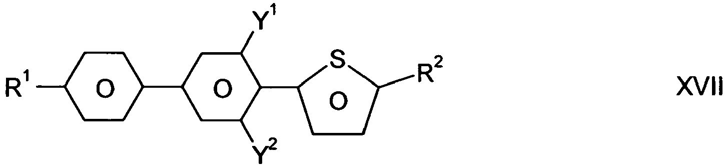 worin Y¹, R¹ und R² die oben angegebene Bedeutung besitzen. R¹ und R² bedeuten vorzugsweise jeweils unabhängig voneinander Alkyl oder Alkenyl mit 1 bzw. 2 bis 8 C-Atomen; Y¹ und Y² bedeuten vorzugsweise beide F.
- Das Medium enthält eine oder mehrere Verbindungen der folgenden Formel, 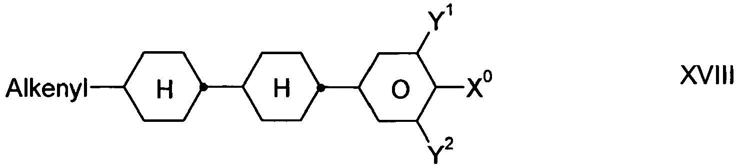 worin X⁰, Y¹ und Y² die in Anspruch 7 angegebenen Bedeutungen besitzen und "Alkenyl" C₂₋₇-Alkenyl bedeutet. Besonders bevorzugt sind Verbindungen der folgenden Formel, 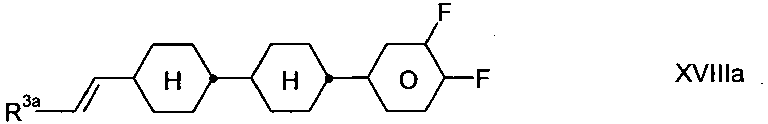 worin R^{3a} die oben angegebene Bedeutung hat und vorzugsweise H bedeutet;
- Das Medium enthält zusätzlich eine oder mehrere Vierkern-Verbindungen ausgewählt aus den Formeln XIX bis XXVIII, 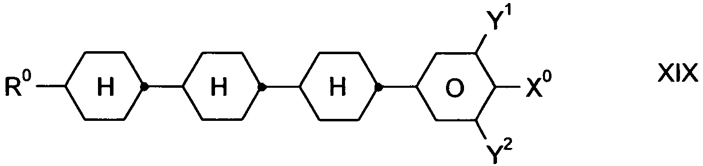 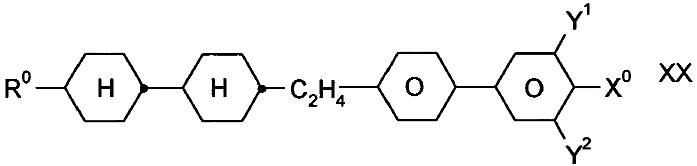 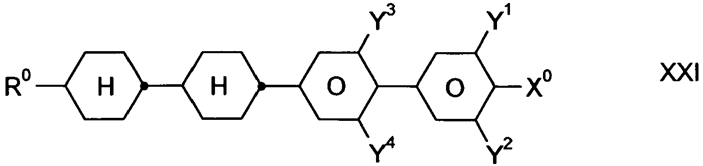 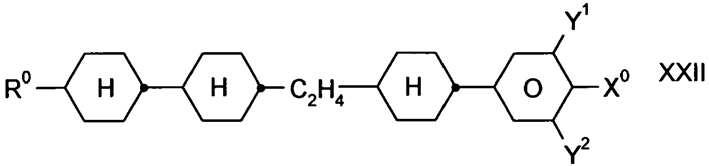 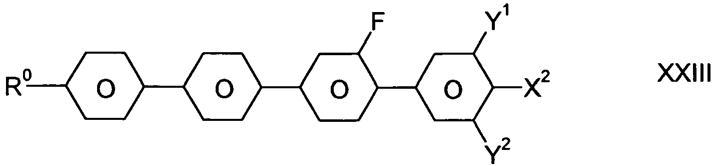 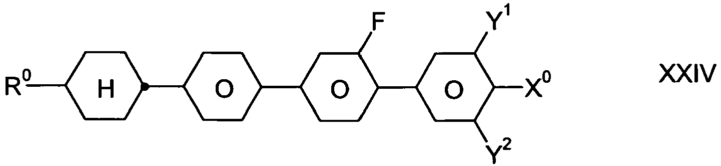 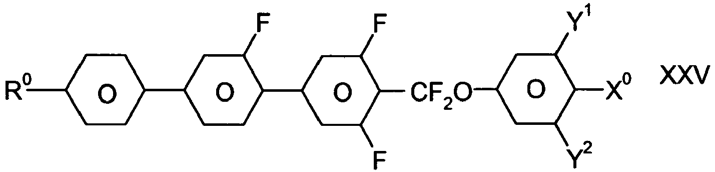 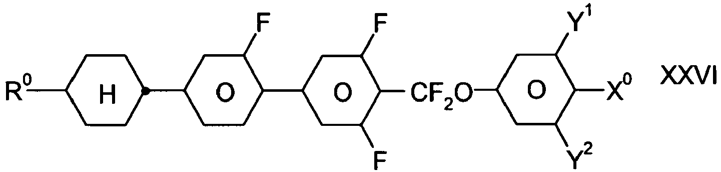 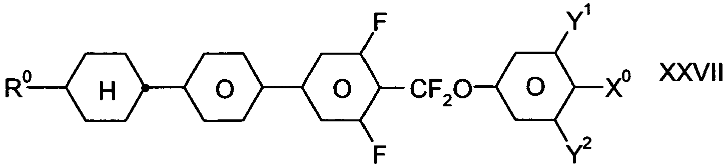 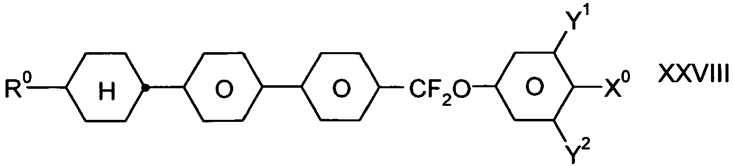 worin Y¹⁻⁴, R⁰ und X⁰ jeweils unabhängig voneinander eine der oben angegebenen Bedeutungen haben. X⁰ ist vorzugsweise F, Cl, CF₃, OCF₃ oder OCHF₂. R⁰ bedeutet vorzugsweise Alkyl, Alkoxy, Oxaalkyl, Fluoralkyl oder Alkenyl mit jeweils bis zu 8 C-Atomen.
   In den Verbindungen der Formeln XIX bis XXVIII bedeuten R⁰ vorzugsweise geradkettiges Alkyl. X⁰ ist vorzugsweise F oder OCF₃, ferner CF₃. Y¹ und Y² bedeuten vorzugsweise Y¹ = F und Y² = H oder Y¹=Y²=F.
   Besonders bevorzugte Verbindungen der Formel XIX bis XXVIII sind die Verbindungen der Formeln XXV, worin X⁰ vorzugsweise F, ferner OCF₃ bedeutet.
   Bevorzugte Mischungen enthalten mindestens eine Verbindung aus der Gruppe S-1, S-2, S-3 und S-4 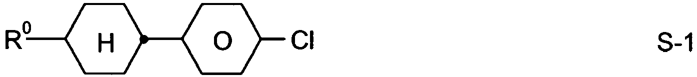 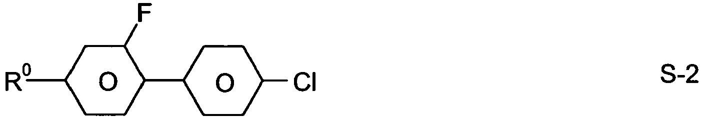 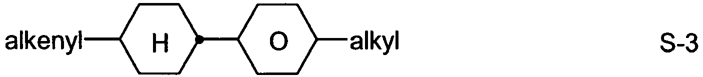 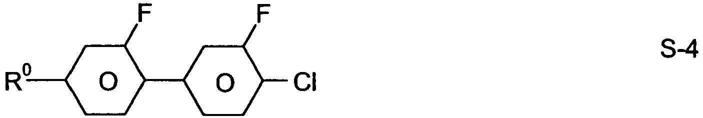 da diese Verbindungen u.a. die smektischen Phasen der Mischungen unterdrücken helfen.
   Das Medium enthält vorzugsweise ein oder mehrere neutrale Verbindungen der allgemeinen Formel N, 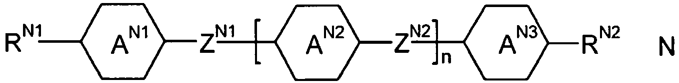 worin
   - R^{N1} und R^{N2}: jeweils unabhängig voneinander einen Alkyl- oder Alkoxyrest mit 1 bis 15 C-Atomen, wobei in diesen Resten auch eine oder mehrere CH₂-Gruppen jeweils unabhängig voneinander durch -C≡C-, -CF₂O-, 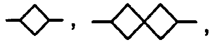 -O-, -CO-O-, -O-CO-so ersetzt sein können, dass O-Atome nicht direkt miteinander verknüpft sind, und worin auch ein oder mehrere H-Atome durch Halogen ersetzt sein können,
   - Ringe A^{N1}, A^{N2} und A^{N3}: jeweils unabhängig voneinander 1,4-Phenylen, 2-Fluor-1,4-Phenylen, 3-Fluor-1,4-Phenylen, trans-1,4-Cyclohexylen, worin auch eine oder zwei CH₂-Gruppen durch -O- ersetzt sein können oder 1,4-Cyclohexenylen,
   - Z^{N1} und Z^{N2}: jeweils unabhängig voneinander eine Einfachbindung, -CH₂CH₂-, -COO-, -OCO-, -C≡C-, -CH₂O-, -OCH₂-, -CF₂O-, -OCF₂- oder -CH=CH-,
   - n: 0, 1 oder 2,
   bedeuten.
   Bevorzugte Verbindungen der Formel N werden nachfolgend genannt: 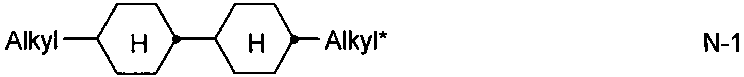 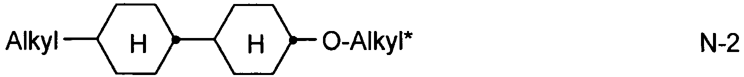 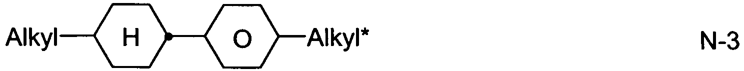 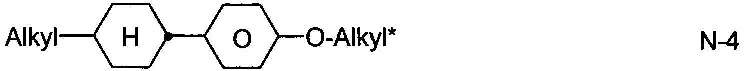 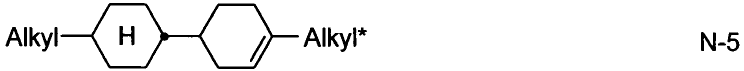 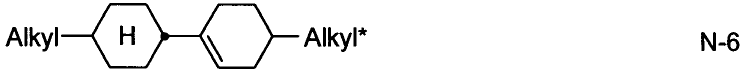 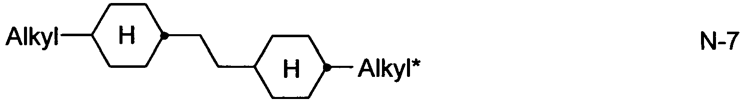 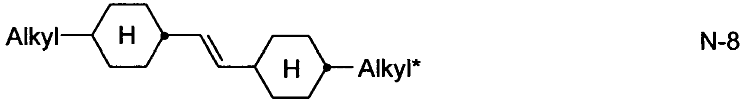 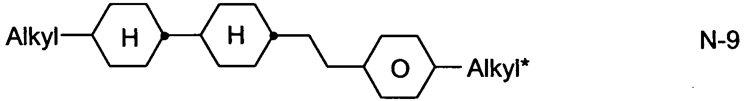 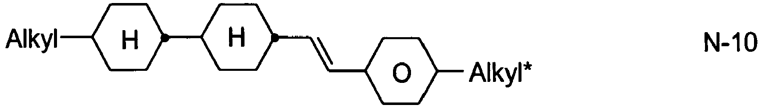 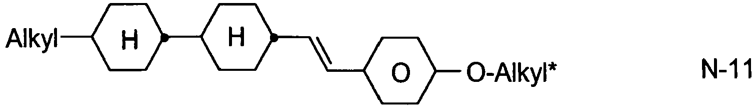 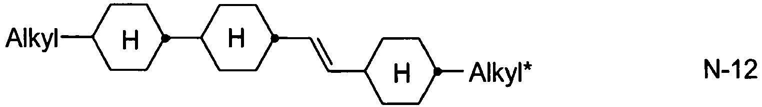 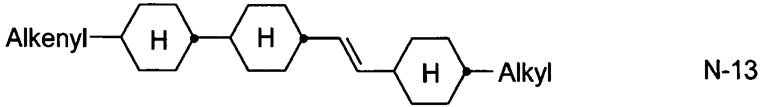 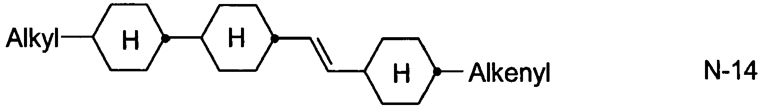 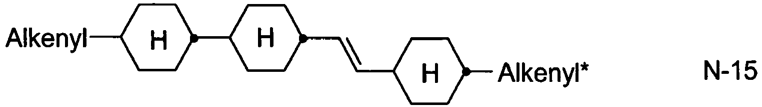 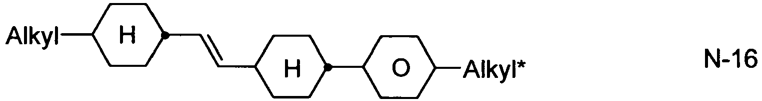 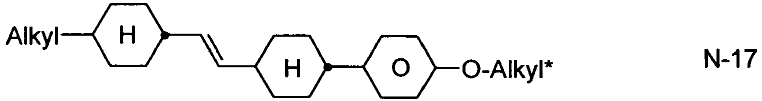 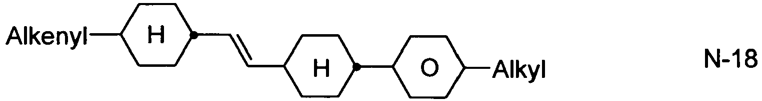 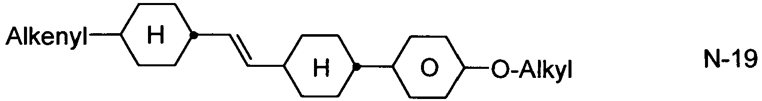 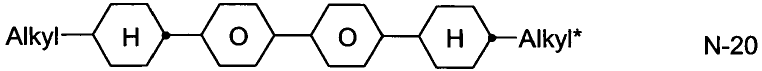 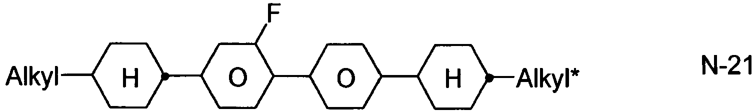 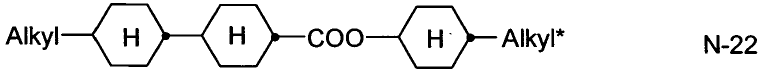 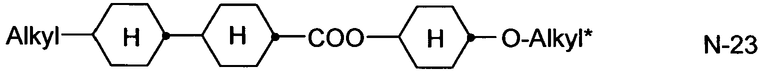 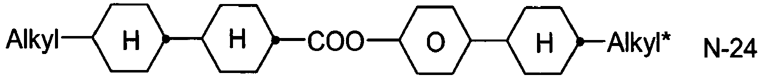 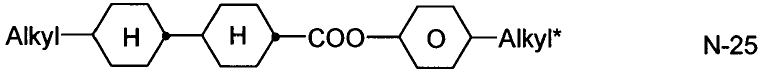 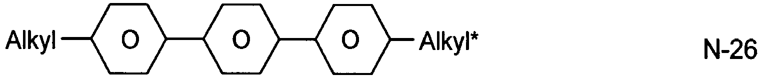 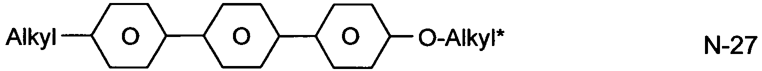 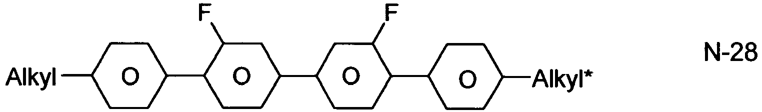 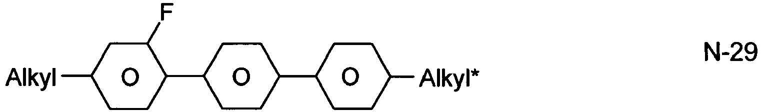 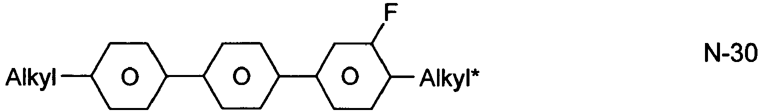 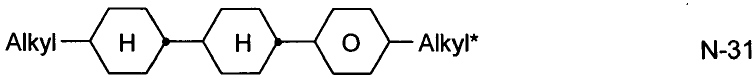 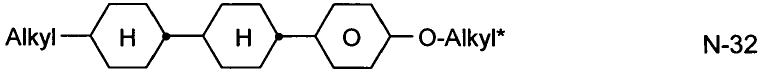 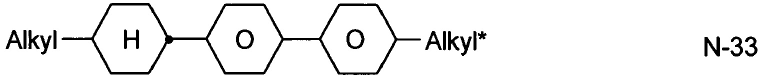 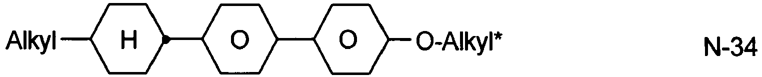 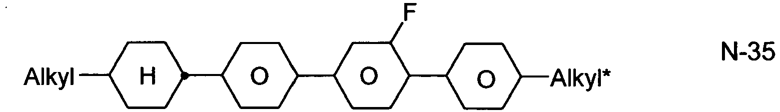 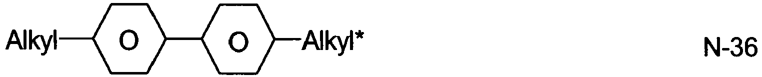 worin
   Alkyl und Alkyl* jeweils unabhängig voneinander einen geradkettigen Alkylrest mit 1 bis 9 C-Atomen, vorzugsweise 2 bis 6 C-Atomen, bedeuten und Alkenyl und Alkenyl* jeweils unabhängig voneinander einen geradkettigen Alkenylrest mit 2-6 C-Atomen bedeuten.
   Von den Verbindungen der Formel N sind insbesondere die Verbindungen der Formeln N-1, N-2, N-3, N-4, N-8, N-9, N-14, N-15, N-17, N-18, N-19, N-20, N-21, N-22, N-23, N-24, N-25, N-31, N-33 und N-36 bevorzugt.
- Das Medium enthält zusätzlich eine oder mehrere Verbindungen der Formeln St-1 bis St-3, 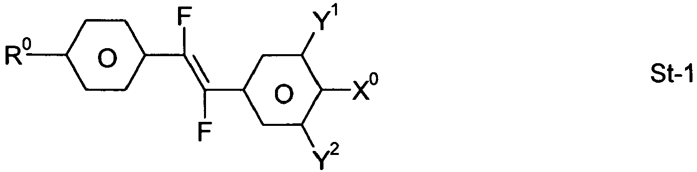 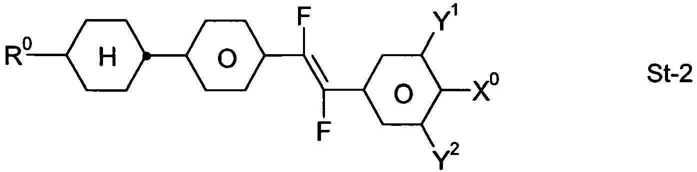 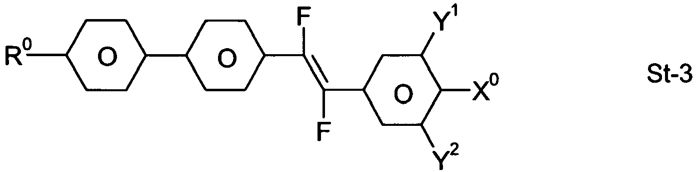 worin R⁰, Y¹, Y² und X⁰ die in Anspruch 7 angegebenen Bedeutungen haben. R⁰ bedeutet vorzugsweise geradkettiges Alkyl, vorzugsweise mit 1-6 C-Atomen. X⁰ ist vorzugsweise F, CF₃ oder OCF₃. Y¹ bedeutet vorzugsweise F. Y² bedeutet vorzugsweise F. Weiterhin sind Verbindungen bevorzugt, worin Y¹=F und Y²=H bedeuten.
- Das Medium enthält eine oder mehrere Pyrimidin- oder Pyridin-Verbindungen der Formeln Py-1 bis Py-5, 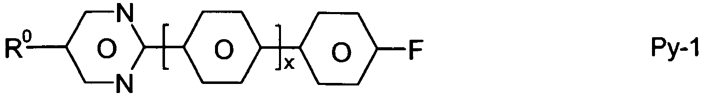 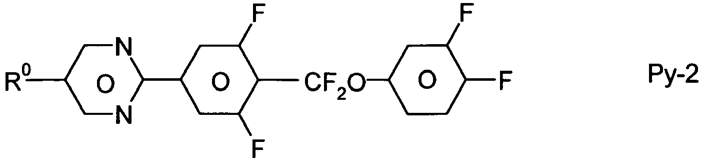 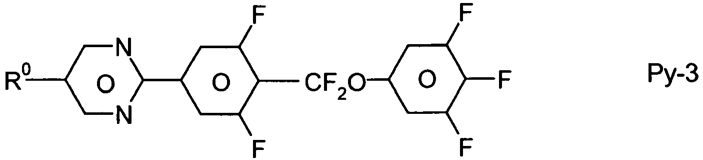 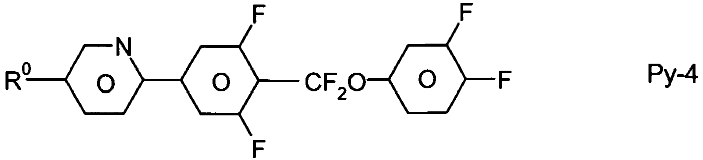 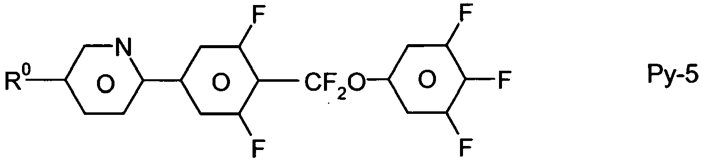 worin R⁰ vorzugsweise geradkettiges Alkyl mit 2-5 C-Atomen ist. x bedeutet 0 oder 1, vorzugsweise ist x=1.
- Das Medium enthält eine oder mehrere Verbindungen ausgewählt aus der Gruppe der Verbindungen der Formeln Y-1, Y-2, Y-3 und Y-4, 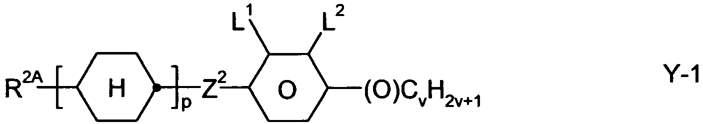 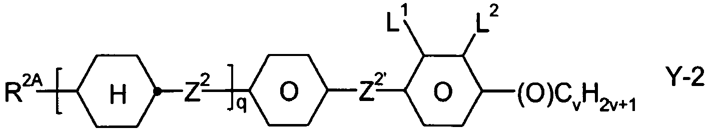 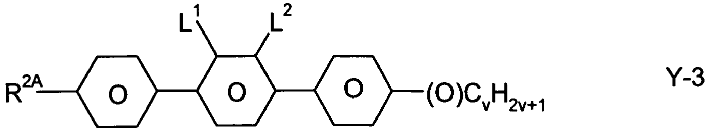 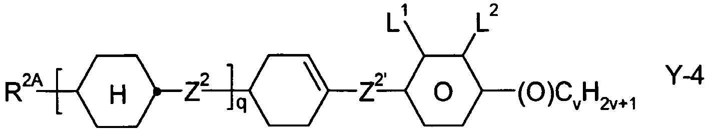 worin
   - R^{2A}: H, einen Alkyl- oder Alkoxyrest mit 1 bis 15 C-Atomen, wobei in diesen Resten auch eine oder mehrere CH₂-Gruppen jeweils unabhängig voneinander durch -C≡C-, -CF₂O-, -CH=CH-, 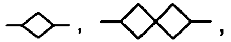 -O-, -CO-O-, -O-CO- so ersetzt sein können, dass O-Atome nicht direkt miteinander verknüpft sind, und worin auch ein oder mehrere H-Atome durch Halogen ersetzt sein können,
   - L¹ und L²: jeweils unabhängig voneinander F, Cl, CF₃ oder CHF₂, vorzugsweise jeweils F,
   - Z² und Z^{2'}: jeweils unabhängig voneinander eine Einfachbindung, -CH₂CH₂-, -CH=CH-, -CF₂O-, -OCF₂-,-CH₂O-, -OCH₂-, -COO-, -OCO-, -C₂F₄-, -CF=CF-, -CH= CHCH₂O-,
   - p: 0, 1 oder 2,
   - q: 0 oder 1,
   - (O)CᵥH₂ᵥ₊₁: OCᵥH₂ᵥ₊₁ oder CᵥH₂ᵥ₊₁, und
   - v: 1 bis 6.
   bedeuten.
   Besonders bevorzugte Verbindungen der Formeln Y-1 bis Y-4 werden nachfolgend genannt: 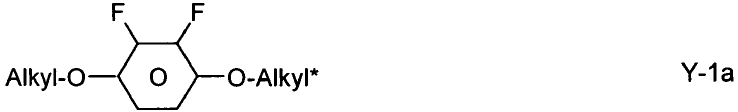 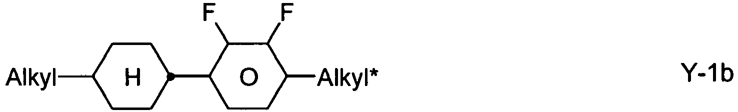 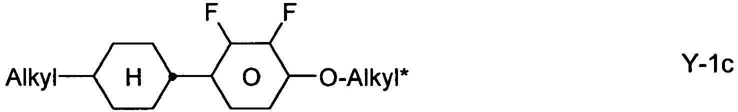 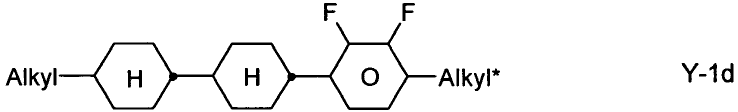 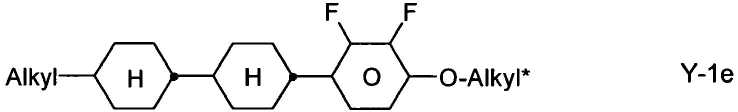 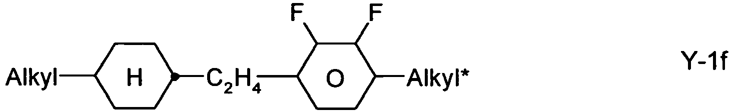 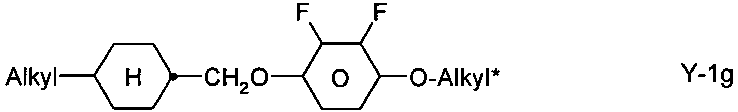 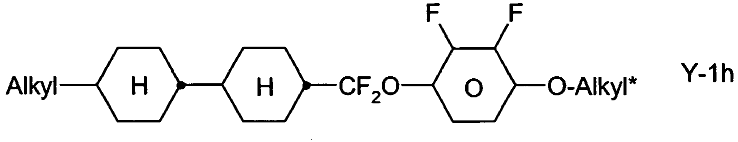 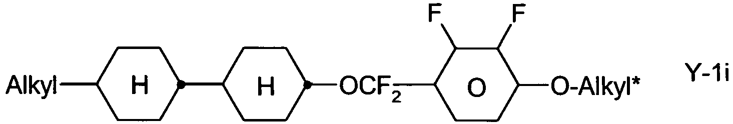 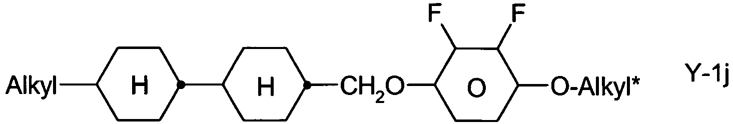 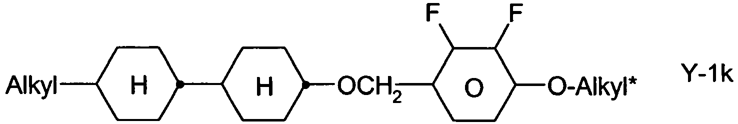 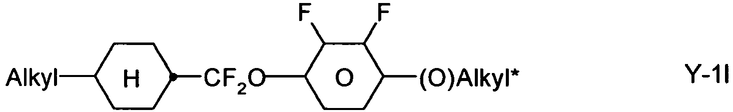 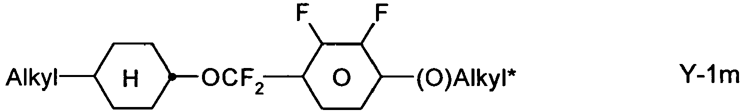 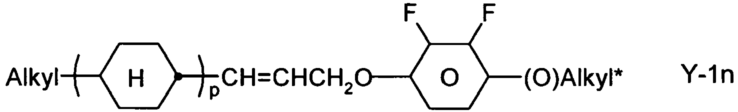 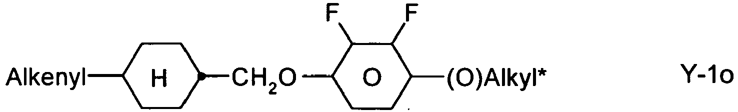 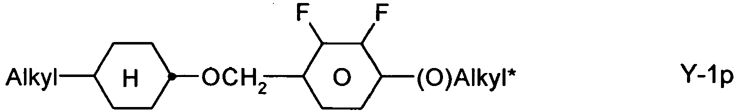 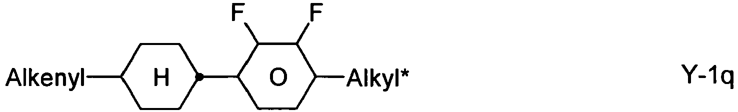 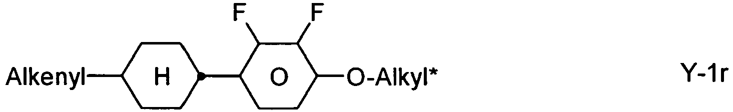 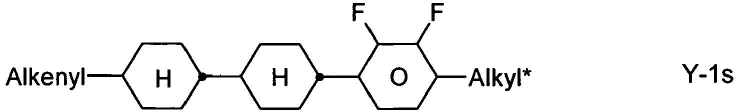 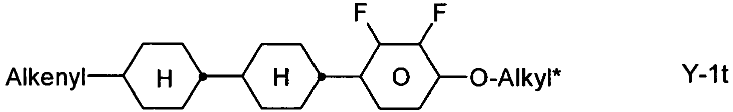 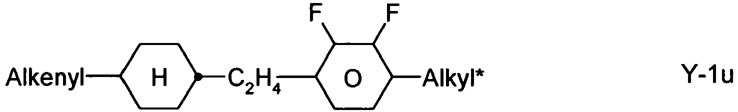 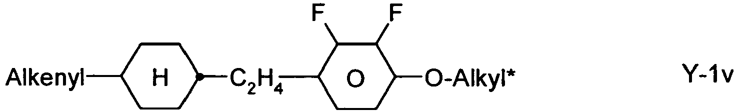 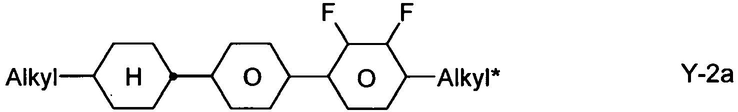 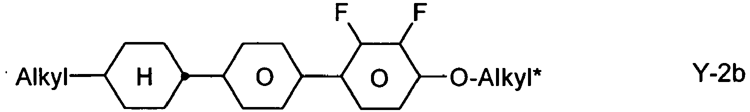 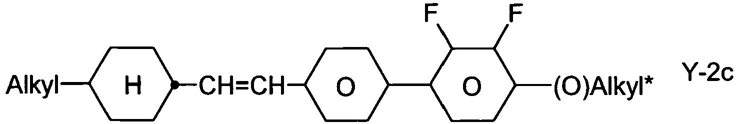 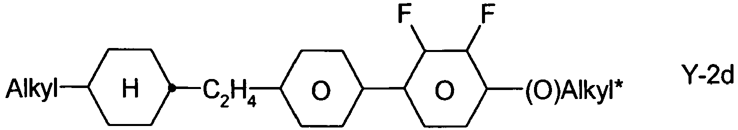 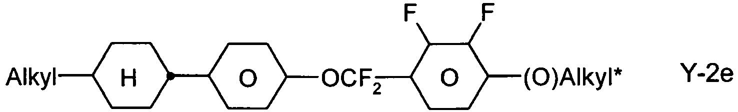 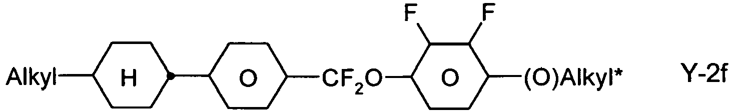 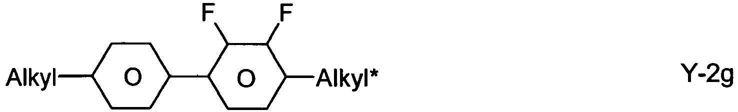 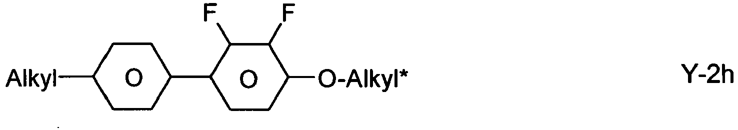 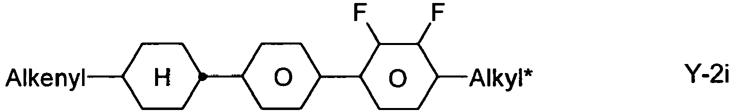 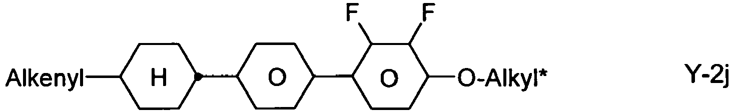 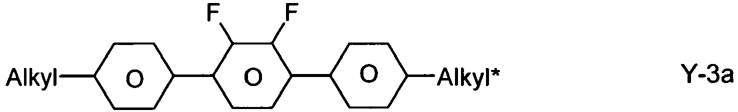
   Von den genannten Verbindungen sind insbesondere die Verbindungen der Formel Y-1a, Y-1c, Y-1e, Y-1g, Y-1j, Y-1r, Y-1t, Y-2b, Y-2h, Y-2j und Y-3a bevorzugt.
   In den vorstehend und nachfolgend genannten Formeln bedeutet 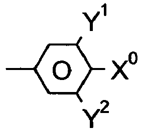 vorzugsweise 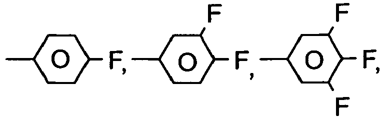 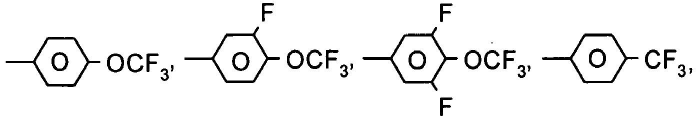 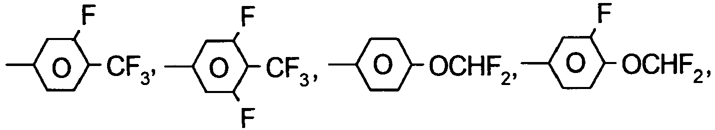 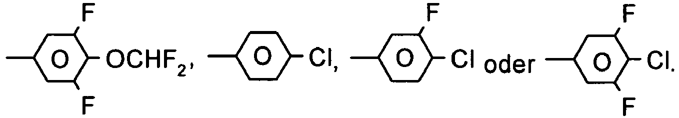
- R⁰ ist vorzugsweise geradkettiges Alkyl oder Alkenyl mit 2 bis 7 C-Atomen;
- X⁰ ist vorzugsweise F, ferner OCF₃, OCH=CF₂, Cl oder CF₃.

Auch andere mesogene Verbindungen, die oben nicht explizit genannt sind, können gegebenenfalls und in vorteilhafter Weise in den Medien gemäß der vorliegenden Erfindung verwendet werden. Solche Verbindungen sind dem Fachmann bekannt.

Der Ausdruck "Alkyl" bzw. "Alkyl*" umfasst in dieser Anmeldung geradkettige und verzweigte Alkylgruppen mit 1-7 Kohlenstoffatomen, insbesondere die geradkettigen Gruppen Methyl, Ethyl, Propyl, Butyl, Pentyl, Hexyl und Heptyl. Gruppen mit 1-6 Kohlenstoffatomen sind im allgemeinen bevorzugt.

Der Ausdruck "OAlkyl" umfasst in dieser Anmeldung geradkettige und verzweigte Alkoxygruppen.

Der Ausdruck "Alkenyl" bzw. "Alkenyl*" umfasst in dieser Anmeldung geradkettige und verzweigte Alkenylgruppen mit 2-7 Kohlenstoffatomen,insbesondere die geradkettigen Gruppen. Bevorzugte Alkenylgruppen sind C₂-C₇-1 E-Alkenyl, C₄-C₇-3E-Alkenyl, C₅-C₇-4-Alkenyl, C₆-C₇-5-Alkenyl und C₇-6-Alkenyl, insbesondere C₂-C₇-1E-Alkenyl, C₄-C₇-3E-Alkenyl und C₅-C₇-4-Alkenyl. Beispiele besonders bevorzugter Alkenylgruppen sind Vinyl, 1 E-Propenyl, 1 E-Butenyl, 1E-Pentenyl, 1 E-Hexenyl, 1E-Heptenyl, 3-Butenyl, 3E-Pentenyl, 3E-Hexenyl, 3E-Heptenyl, 4-Pentenyl, 4Z-Hexenyl, 4E-Hexenyl, 4Z-Heptenyl, 5-Hexenyl, 6-Heptenyl und dergleichen. Gruppen mit bis zu 5 Kohlenstoffatomen sind im allgemeinen bevorzugt.

Der Ausdruck "Fluoralkyl" umfasst in dieser Anmeldung geradkettige Gruppen mit mindestens einem Fluoratom, vorzugsweise einem endständigem Fluor, d.h. Fluormethyl, 2-Fluorethyl, 3-Fluorpropyl, 4-Fluorbutyl, 5-Fluorpentyl, 6-Fluorhexyl und 7-Fluorheptyl. Andere Positionen des Fluors sind jedoch nicht ausgeschlossen.

Der Ausdruck "Oxaalkyl" bzw. "Alkoxy" umfasst in dieser Anmeldung geradkettige Reste der Formel CₙH₂ₙ₊₁-O-(CH₂)ₘ, worin n und m jeweils unabhängig voneinander 1 bis 6 bedeuten. m kann auch 0 bedeuten. Vorzugsweise ist n = 1 und m 1-6 oder m = 0 und n = 1-3.

Die einzelnen Verbindungen der oben genannten Formeln und deren Unterformeln, die in den erfindungsgemäßen Medien verwendet werden können, sind entweder bekannt, oder sie können analog zu den bekannten Verbindungen hergestellt werden.

Die Flüssigkristallmedien gemäß der vorliegenden Erfindung weisen bevorzugt einen Klärpunkt von 70°C oder mehr, stärker bevorzugt von 75°C oder mehr, besonders bevorzugt von 80°C oder mehr und ganz besonders bevorzugt von 85°C oder mehr, auf.

Vorzugsweise erstreckt sich die nematische Phase der erfindungsgemäßen Medien mindestens von 0°C oder weniger bis 70°C oder mehr, stärker bevorzugt mindestens von -20°C oder weniger bis 75°C oder mehr, ganz bevorzugt mindestens von -30°C oder weniger bis 75°C oder mehr und insbesondere mindestens von -40°C oder weniger bis 80°C oder mehr.

Das optimale Mengenverhältnis der Verbindungen der oben genannten Formeln hängt weitgehend von den gewünschten Eigenschaften, von der Wahl der Komponenten der oben genannten Formeln und der Wahl weiterer gegebenenfalls vorhandener Komponenten ab.

Die Gesamtmenge an Verbindungen der oben genannten Formeln in den erfindungsgemäßen Gemischen ist nicht kritisch. Die Gemische können daher eine oder mehrere weitere Komponenten enthalten zwecks Optimierung verschiedener Eigenschaften. Der beobachtete Effekt auf die gewünschte Verbesserung der Eigenschaften der Mischung ist jedoch in der Regel umso größer je höher die Gesamtkonzentration an Verbindungen der oben genannten Formeln ist.

Wie zuvor schon erwähnt, verwenden ECB-Anzeigen wie ASV-Anzeigen flüssigkristalline Medien mit negativer dielektrischer Anisotropie (Δε), wohingegen TN- und bislang alle gebräuchlichen IPS-Anzeigen flüssigkristalline Medien mit positiver dielektrischer Anisotropie verwenden.

In einer bevorzugten Ausführungsform betrifft die vorliegende Erfindung daher ein flüssigkristallines Medium mit positiver dielektrischer Anisotropie.

Bevorzugte Verbindungen die neben einer, zwei oder mehreren Verbindungen der Formel I in einem erfindungsgemäßen flüssigkristallinen Medium mit positiver dielektrischer Anisotropie eingesetzt werden können sind im Folgenden angegeben:
- Das Medium enthält vorzugsweise eine oder mehrere Verbindungen ausgewählt aus der Gruppe der Verbindungen der Formeln I, II, III, V, VI-1, VI-2, XII, XIII, XIV, XVII, XXIII, XXV.
- Das Medium enthält vorzugsweise eine oder mehrere Verbindungen der Formel VI-1;
- Das Medium enthält vorzugsweise eine oder mehrere Verbindungen der Formel VI-2;
- Der Anteil an Verbindungen der Formeln II-XXVII im Gesamtgemisch beträgt vorzugsweise 20 bis 99 Gew.%;
- Das Medium enthält vorzugsweise 25-80 Gew.%, besonders bevorzugt 30-70 Gew.% an Verbindungen der Formel II und/oder III;
- Das Medium enthält vorzugsweise 0-70 Gew.%, besonders bevorzugt 20-60 Gew.% an Verbindungen der Formel IIa-1;
- Das Medium enthält vorzugsweise 0-25 Gew.%, besonders bevorzugt 5-25 Gew.% an Verbindungen der Formel IIa-2;
- Das Medium enthält vorzugsweise 0-30 Gew.%, besonders bevorzugt 5-25 Gew.% an Verbindungen der Formel IIa-3;
- Das Medium enthält vorzugsweise 0-25 Gew.%, besonders bevorzugt 5-25 Gew.% an Verbindungen der Formel IIa-5;
- Das Medium enthält vorzugsweise 5-40 Gew.%, besonders bevorzugt 10-30 Gew.% an Verbindungen der Formel V;
- Das Medium enthält vorzugsweise 3-30 Gew.%, besonders bevorzugt 6-25 Gew.% an Verbindungen der Formel VI-1;
- Das Medium enthält vorzugsweise 2-30 Gew.%, besonders bevorzugt 4-25 Gew.% an Verbindungen der Formel VI-2;
- Das Medium enthält 2-40 Gew.%, besonders bevorzugt 5-30 Gew.% an Verbindungen der Formel XII;
- Das Medium enthält vorzugsweise 1-25 Gew.%, besonders bevorzugt 2-15 Gew.%, an Verbindungen der Formel XIII;
- Das Medium enthält vorzugsweise 5-45 Gew.%, besonders bevorzugt 10-35 Gew.%, an Verbindungen der Formel XIV;
- Das Medium enthält vorzugsweise 1-20 Gew.%, besonders bevorzugt 2-15 Gew.% an Verbindungen der Formel XVI;
- Das Medium enthält vorzugsweise 5 - 30 Gew.%, besonders bevorzugt 8 - 22 Gew.%, an Verbindungen der Formel Va, worin X⁰ = OCH=CF₂ bedeutet;

In einer besonders bevorzugten Ausführungsform enthalten die erfindungsgemäßen Medien mit positiver dielektrischer Anisotropie Verbindungen der Formel IV bis VIII, worin X⁰ F, OCF₃, OCHF₂, OCH=CF₂, OCF=CF₂ oder OCF₂-CF₂H bedeutet. Eine günstige synergistische Wirkung mit den Verbindungen der Formel I führt zu besonders vorteilhaften Eigenschaften. Insbesondere Mischungen enthaltend Verbindungen der Formeln VI, bzw. XI, bzw. VI und XI zeichnen sich durch ihre niedrigen Schwellenspannungen aus.

Gegenstand der Erfindung sind somit auch elektrooptische Anzeigen, wie z. B. TN-, STN-, TFT-, OCB-, IPS-, PS-IPS, FFS-, PS-FFS-, positive VA- oder MFK-Anzeigen mit zwei planparallelen Trägerplatten, die mit einer Umrandung eine Zelle bilden, integrierten nicht-linearen Elementen zur Schaltung einzelner Bildpunkte auf den Trägerplatten und einer in der Zelle befindlichen nematischen Flüssigkristallmischung mit positiver dielektrischer Anisotropie und hohem spezifischem Widerstand), die derartige Medien enthalten sowie die Verwendung dieser Medien für elektrooptische Zwecke. Die IPS- und FFS-Anzeigen können sowohl LC-Mischungen mit negativer dielektrischer Anisotropie als auch LC-Mischungen mit positiver dielektrischer Anisotropie enthalten.

Weiterhin sind die erfindungsgemäßen Mischungen mit positiver dielektrischer Anisotropie auch für positiv-VA-Anwendungen geeignet, auch HT-VA-Anwendungen genannt. Hierunter versteht man elektrooptische Anzeigen mit einer In-plane Ansteuerelektroden-Konfiguration und homeotroper Anordnung des Flüssigkristallmediums mit positiver dielektrischer Anisotropie.

Insbesondere bevorzugt sind die erfindungsgemäßen Mischungen mit positiver dielektrischer Anisotropie für TN-TFT-Display-Anwendungen mit kleiner Operationsspannung, d.h. besonders bevorzugt für Notebook-Anwendungen.

Die erfindungsgemäßen Mischungen mit positiver dielektrischer Anisotropie sind insbesondere für mobile Anwendungen und high-Δn-TFT-Anwendungen wie z. B. PDAs, Notebooks, LCD-TV und Monitore geeignet.

Die erfindungsgemäßen Flüssigkristallmischungen mit positiver dielektrischer Anisotropie ermöglichen es, bei Beibehaltung der nematischen Phase bis -20 °C und bevorzugt bis -30 °C, besonders bevorzugt bis -40 °C, und des Klärpunkts ≥ 70 °C, vorzugsweise ≥ 74 °C, gleichzeitig Rotationsviskositäten γ₁ von ≤ 120 mPa·s, besonders bevorzugt 60 mPa·s zu erreichen, wodurch hervorragende MFK-Anzeigen mit schnellen Schaltzeiten erzielt werden können.

Die dielektrische Anisotropie der erfindungsgemäßen Flüssigkristallmischungen mit positiver dielektrischer Anisotropie Δε ist vorzugsweise ≥ +3, besonders bevorzugt ≥ +4. Die Mischungen sind außerdem durch kleine Operationsspannungen gekennzeichnet. Die Schwellenspannung der erfindungsgemäßen Flüssigkristallmischungen ist vorzugsweise ≤ 2,5 V, insbesondere ≤ 2,2 V.

Die Doppelbrechung Δn der erfindungsgemäßen Flüssigkristallmischungen mit positiver dielektrischer Anisotropie ist vorzugsweise ≥ 0,08, insbesondere ≥ 0,10.

Sofern die erfindungsgemäßen Mischungen in IPS- oder FFS-Anwendungen zum Einsatz kommen, besitzen die Mischungen mit positiver dielektrischer Anisotropie vorzugsweise einen Wert für die dielektrische Anisotropie von 3-20 und einen Wert für die optische Anisotropie von 0,07-0,13.

In einer gleichermaßen bevorzugten Ausführungsform betrifft die vorliegende Erfindung aber auch ein flüssigkristallines Medium mit negativer dielektrischer Anisotropie.

Bevorzugte Verbindungen die neben einer, zwei oder mehreren Verbindungen der Formel I in einem erfindungsgemäßen flüssigkristallinen Medium mit negativer dielektrischer Anisotropie eingesetzt werden können sind im Folgenden angegeben:
- Das Medium enthält vorzugsweise eine oder mehrere Verbindungen der Formel II, bevorzug ausgewählt aus der Gruppe der Verbindungen der Formeln CC-n-V und CC-n-Vm, bevorzugt CC-3-V, CC-3-V1, CC-4-V und CC-5-V, besonders bevorzugt ausgewählt aus der Gruppe der Verbindungen CC-3-V, CC-3-V1 und CC-4-V, ganz besonders bevorzugt der Verbindung CC-3-V und gegebenenfalls zusätzlich der Verbindung CC-4-V und/oder CC-3-V1,
- Das Medium enthält vorzugsweise die Verbindung PP-1-2V1;
- Das Medium enthält vorzugsweise eine oder mehrere Verbindungen der Formel Y-1, bevorzugt der Formel Y-1c, ausgewählt aus der Gruppe der Verbindungen der Formeln CY-3-O, CY-3-O4, CY-5-02 und CY-5-O4,
- Das Medium enthält vorzugsweise eine oder mehrere Verbindungen der Formel Y-1, bevorzugt ausgewählt aus der Gruppe der Verbindungen der Formel Y-1e und Y-1d, bevorzugt der Formel CCY-n-Om, bevorzugt ausgewählt aus der Gruppe der Verbindungen der Formeln CCY-3-O2, CCY-2-O2, CCY-3-O1, CCY-3-03, CCY-4-O2, CCY-3-O2 und CCY-5-O2,
- Das Medium enthält vorzugsweise eine oder mehrere Verbindungen der Formel Y-2, bevorzugt der Formel Y-2b, bevorzugt ausgewählt aus der Gruppe der Verbindungen der Formeln CPY-2-O2 und CPY-3-O2, CPY-4-O2 und CPY-5-O2,
- Das Medium enthält vorzugsweise eine oder mehrere Verbindungen der Formel Y-2h, bevorzugt ausgewählt aus der Gruppe der Verbindungen der Formeln PY-3-O2, PY-1-O4 und PY-4-02,
- Das Medium enthält vorzugsweise eine oder mehrere Verbindungen der Formel Y-3, bevorzugt ausgewählt aus der Gruppe der Verbindungen der Formeln PYP-2-3 und PYP-2-4,
- Das Medium enthält vorzugsweise optional eine oder mehrere Verbindungen der Y-4, bevorzugt der Formel CLY-n-Om, bevorzugt ausgewählt aus der Gruppe der Verbindungen der Formeln CLY-2-04, CLY-3-02, CLY-3-03,
- Das Medium enthält vorzugsweise Verbindungen der Formeln II und Y-1 bis Y-4 im Gesamtgemisch von 20 bis 99 Gew.%;
- Das Medium enthält vorzugsweise 10 Gew.% oder mehr bis 60 Gew.% oder weniger, bevorzugt 15 Gew.% oder mehr bis 50 Gew.% oder weniger, besonders bevorzugt 20 Gew.% oder mehr bis 45 Gew.% oder weniger, an Verbindungen der Formel 11 und/oder 111;
- Das Medium enthält vorzugsweise 45 Gew.% oder mehr bis 80 Gew.% oder weniger an Verbindungen der Formeln Y-1 bis Y-4,
- Das Medium enthält vorzugsweise 10 Gew.% oder mehr bis 40 Gew.% oder weniger an Verbindungen der Formeln Y-1,
- Das Medium enthält vorzugsweise 10 Gew.% oder mehr bis 40 Gew.% oder weniger an Verbindungen der Formeln Y-2,
- Das Medium enthält vorzugsweise 10 Gew.% oder mehr bis 40 Gew.% oder weniger an Verbindungen der Formeln Y-3,
- Das Medium enthält vorzugsweise 0 Gew.% oder mehr bis 40 Gew.% oder weniger an Verbindungen der Formeln Y-4.

In einer bevorzugten Ausführungsform sind die erfindungsgemäßen Flüssigkristallmedien mit negativer dielektrischer Anisotropie durch Werte der optischen Anisotropien im mittleren bis niedrigen Bereich gekennzeichnet. Die Werte der Doppelbrechung liegen bevorzugt im Bereich von 0,065 oder mehr bis 0,140 oder weniger, besonders bevorzugt im Bereich von 0,090 oder mehr bis 0,130 oder weniger und ganz besonders bevorzugt im Bereich von 0,095 oder mehr bis 0,120 oder weniger.

In dieser Ausführungsform haben die erfindungsgemäßen Flüssigkristallmedien mit negativer dielektrischer Anisotropie relativ hohe Werte des Betrags der dielektrischen Anisotropie (|Δε|) auf, die bevorzugt im Bereich von 2,7 oder mehr bis 6,0 oder weniger, bevorzugt bis 5,0 oder weniger, bevorzugt von 2,9 oder mehr bis 5,0 oder weniger, besonders bevorzugt von 3,0 oder mehr bis 4,5 oder weniger und ganz besonders bevorzugt von 3,5 oder mehr bis 4,3 oder weniger, liegen.

Die erfindungsgemäßen Flüssigkristallmedien mit negativer dielektrischer Anisotropie weisen relativ kleine Werte für die Schwellenspannung (Vo) im Bereich von 1,7 V oder mehr bis 2,5 V oder weniger, bevorzugt von 1,8 V oder mehr bis 2,4 V oder weniger, besonders bevorzugt von 1,9 V oder mehr bis 2,3 V oder weniger und ganz besonders bevorzugt von 1,95 V oder mehr bis 2,1 V oder weniger, auf.

Die erfindungsgemäßen Mischungen mit negativer dielektrischer Anisotropie sind für alle VA-TFT-Anwendungen geeignet, wie z.B. VAN, MVA, (S)-PVA und ASV. Weiterhin sind sie für IPS (In plane switching)-, FFS (Fringe field switching)- und PALC- Anwendungen mit negativem as geeignet.

Gegenstand der Erfindung sind somit auch elektrooptische Anzeigen die auf dem VA- oder dem ECB-Effekt basieren und insbesondere solche, die mittels einer Aktivmatrix-Adressierungsvorrichtung angesteuert werden.

Es versteht sich, dass durch geeignete Wahl der Komponenten der erfindungsgemäßen Mischungen auch höhere Klärpunkte (z.B. oberhalb 100 °C) bei höheren Schwellenspannungen oder niedrigere Klärpunkte bei niedrigeren Schwellenspannungen unter Erhalt der anderen vorteilhaften Eigenschaften realisiert werden können. Ebenso können bei entsprechend wenig erhöhten Viskositäten Mischungen mit größerem absoluten Δε und somit geringen Schwellen erhalten werden.

Der Aufbau der erfindungsgemäßen MFK-Anzeige aus Polarisatoren, Elektrodengrundplatten und Elektroden mit Oberflächenbehandlung entspricht der für derartige Anzeigen üblichen Bauweise. Dabei ist der Begriff der üblichen Bauweise hier weit gefasst und umfasst auch alle Abwandlungen und Modifikationen der MFK-Anzeige, insbesondere auch Matrix-Anzeigeelemente auf Basis poly-Si TFT oder MIM.

Die Herstellung der erfindungsgemäß verwendbaren Flüssigkristallmischungen erfolgt in an sich üblicher Weise, beispielsweise indem man eine oder mehrere Verbindungen der Formel I mit mindestens einer weiteren mesogenen Verbindung und optional mit einem oder mehreren Additiv(en) und/oder einer oder mehreren polymerisierbaren Verbindungen mischt.

In der Regel wird die gewünschte Menge der in geringerer Menge verwendeten Komponenten in der den Hauptbestandteil ausmachenden Komponenten gelöst, zweckmäßig bei erhöhter Temperatur. Es ist auch möglich Lösungen der Komponenten in einem organischen Lösungsmittel, z.B. in Aceton, Chloroform oder Methanol, zu mischen und das Lösungsmittel nach Durchmischung wieder zu entfemen, beispielsweise durch Destillation.

Die Dielektrika können auch weitere, dem Fachmann bekannte und in der Literatur beschriebene Zusätze, wie z. B. UV-Stabilisatoren wie Tinuvin^{®} der Fa. Ciba Chemicals, insbesondere Tinuvin^{®} 770, Antioxidantien, Radikalfänger, Nanopartikel, etc. enthalten. Beispielsweise können 0-15 % pleochroitische Farbstoffe oder chirale Dotierstoffe zugesetzt werden. Geeignete Stabilisatoren und Dotierstoffe werden nachfolgend in den Tabellen C und D genannt.

Den erfindungsgemäßen Mischungen zusätzlich auch polymerisierbare Verbindungen, sogenannte "Reaktive Mesogene", zugesetzt werden. Bevorzugte polymerisierbare Verbindungen sind in der Tabelle E gelistet.

Für die vorliegende Erfindung bedeutet zug" kleiner oder gleich, bevorzugt kleiner und ">_" größer oder gleich, bevorzugt größer.

Für die vorliegende Erfindung bedeuten 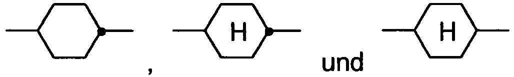 trans-1,4-Cyclohexylen und 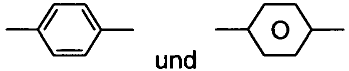 1,4-Phenylen.

Für die vorliegende Erfindung bedeuten die Begriffe "dielektrisch positive Verbindungen" solche Verbindungen mit einem As > 1,5, dielektrisch neutrale Verbindungen" solche mit -1,5 ≤ Δε ≤ 1,5 und dielektrische negative" Verbindungen solche mit As < -1,5. Hierbei wird die dielektrische Anisotropie der Verbindungen bestimmt, indem 10 % der Verbindungen in einem flüssigkristallinen Host gelöst werden und von der resultierenden Mischung die Kapazität in mindestens jeweils einer Testzelle mit 20 µm Schichtdicke mit homeotroper und mit homogener Oberflächenorientierung bei 1 kHz bestimmt wird. Die Messspannung beträgt typischerweise 0,5 V bis 1,0 V, sie ist jedoch stets niedriger als die kapazitive Schwelle der jeweiligen untersuchten Flüssigkristallmischung.

Als Hostmischung für dielektrisch positive und dielektrisch neutrale Verbindungen wird ZLI-4792 und für dielektrisch negative Verbindungen ZLI-2857, beide von Merck KGaA, Deutschland, verwendet. Aus der Änderung der Dielektrizitätskonstante der Hostmischung nach Zugabe der zu untersuchenden Verbindung und Extrapolation auf 100 % der eingesetzten Verbindung werden die Werte für die jeweiligen zu untersuchenden Verbindungen erhalten. Die zu untersuchende Verbindung wird zu 10 % in der Hostmischung gelöst. Wenn die Löslichkeit der Substanz hierzu zu gering ist, wird die Konzentration schrittweise solange halbiert, bis die Untersuchung bei der gewünschten Temperatur erfolgen kann.

Alle Konzentrationen sind für die Zwecke der vorliegenden Erfindung, soweit nicht explizit anders vermerkt, in Massenprozent angegeben und beziehen sich auf die entsprechende Mischung oder Mischungskomponente, soweit nicht explizit anders angegeben.

Alle angegebenen Werte für Temperaturen in der vorliegenden Anmeldung, wie z. B. der Schmelzpunkt T(C,N), der Übergang von der smektischen (S) zur nematischen (N) Phase T(S,N) und der Klärpunkt T(N,I), sind in Grad Celsius (°C) und alle Temperaturdifferenzen entsprechend Differenzgrad (° oder Grad) angegeben, sofern nicht explizit anders angegeben.

Der Begriff "Schwellenspannung" bezieht sich für die vorliegende Erfindung auf die kapazitive Schwelle (Vo), auch Freedericksz-Schwelle genannt, Sofern nicht explizit anders angegeben.

Alle physikalischen Eigenschaften werden und wurden nach "Merck Liquid Crystals, Physical Properties of Liquid Crystals", Status Nov. 1997, Merck KGaA, Deutschland bestimmt und gelten für eine Temperatur von 20°C und An wird bei 589 nm und As bei 1 kHz bestimmt, Sofern nicht jeweils explizit anders angegeben.

Die verwendeten Flüssigkristallmischungen sind, wenn nicht anders angegeben, nicht mit einem chiralen Dotierstoff versetzt, sie eignen sich aber auch besonders für Anwendungen, in denen eine solche Dotierung erforderlich ist.

Die VHR wird in bei der Firma Merck Japan hergestellten Testzellen bestimmt. Die Messzellen haben Substrate aus Natriumglas (Sodalime Glas) und sind mit Polyimidorientierungsschichten (AL-16301 der Firma Japan Synthetic Rubber, Japan) mit einer Schichtdicke von 50 nm, die senkrecht zueinander gerieben sind ausgeführt. Die Schichtdicke beträgt einheitlich 6,0 µm. Die Fläche der durchsichtigen Elektroden aus ITO beträgt 1 cm².

Die VHR wird bei 20°C (VHR₂₀) und nach 5 Minuten im Ofen bei 100°C (VHR₁₀₀) in einem kommerziell erhältlichen Gerät der Firma Autronic Melchers, Deutschland bestimmt. Die verwendete Spannung hat eine Frequenz von 60 Hz.

Die Genauigkeit der Messwerte der VHR hängt vom jeweiligen Wert der VHR ab. Dabei nimmt die Genauigkeit mit geringer werdenden Werten ab. Die bei Werten in den verschiedenen Größenbereichen in der Regel beobachten Abweichungen sind in ihrer Größenordnung in der folgenden Tabelle zusammen gestellt.

| VHR-Bereich | | Abweichung (relativ) |
|---|---|---|
| VHR-Werte | | Δ_{G}VHR/VHR/% |
| von | bis | ca. |
| 99,6 % | 100 % | +/- 0,1 |
| 99,0 % | 99,6 % | +/- 0,2 |
| 98 % | 99 % | +/- 0,3 |
| 95 % | 98 % | +/- 0,5 |
| 90 % | 95 % | +/- 1 |
| 80 % | 90 % | +/- 2 |
| 60 % | 80 % | +/- 4 |
| 40 % | 60 % | +/- 8 |
| 20 % | 40 % | +/- 10 |
| 10 % | 20 % | +/- 20 |

Die Stabilität gegen Bestrahlung mit UV wird in einem "Suntest CPS" einem kommerziellen Gerät der Firma Heraeus, Deutschland untersucht. Dabei werden die versiegelten Testzellen 2,0 Stunden ohne zusätzliche thermische Belastung bestrahlt. Die Bestrahlungsleistung im Wellenlängebereich von 300 nm bis 800 nm beträgt 765 W/m² V.

Eine weitere Kenngröße, die neben der VHR die Leitfähigkeit der Flüssigkristallmischungen charakterisieren kann, ist die Ionendichte ("ion density"). Hohe Werte der Ionendichte führen oft zur Entstehung von Displayfehler wie Imagesticking und Flackern. Die lonendichte wird bevorzugt in Testzellen bestimmt, die bei Merck Japan^{Ltd.} hergestellten werden. Die Testzellen haben Substrate aus Natriumglas (Sodalime Glas) und sind mit Polyimidorientierungsschichten (AL-3046 der Firma Japan Synthetic Rubber, Japan) mit einer Schichtdicke des Polyimids von 40 nm ausgeführt. Die Schichtdicke der Flüssigkristallmischung beträgt einheitlich 5,8 µm. Die Fläche der kreisförmigen, durchsichtigen Elektroden aus ITO, die zusätzlich mit einem "guard-ring" ausgestattet sind, beträgt 1 cm². Die Genauigkeit der Messmethode beträgt ca. ± 15 %. Die Zellen werden vor der Befüllung mit der betreffenden Flüssigkristallmischung über Nacht in einem Ofen bei 120°C getrocknet.
Die Ionendichte wird mit einem kommerziell erhältlichen Gerät der Firma TOYO, Japan gemessen. Bei der Messmethode handelt es sich im Wesentlichen um eine zur Cyclovoltametrie analogen Messmethode, wie in M. Inoue, "Recent measurement of Liquid Crystal Material Characterisitcs", Proceedings IDW 2006, LCT-7-1,647 beschrieben. Hierbei wird eine angelegte Gleichspannung gemäß eines vorgegebenen Dreiecksprofils zwischen einem positiven bzw. negativen Maximalwert variiert. Ein komplettes Durchlaufen des Profils bildet somit einen Messzyklus. Ist die angelegte Spannung groß genug, so dass sich die Ionen im Feld zur jeweiligen Elektrode bewegen können, entsteht durch Entladung der Ionen ein Ionenstrom. Die übertragene Ladungsmenge liegt hierbei typischerweise im Bereich von einigen pC bis zu wenigen nC. Dieses macht eine hochempfindliche Detektion notwendig, die durch das oben genannte Gerät gewährleistet ist. Die Resultate werden in einer Strom/Spannungs-Kurve dargestellt. Der Ionenstrom ist hierbei durch Auftreten eines Peaks bei Spannungen, die kleiner sind als die Schwellspannung der Flüssigkristallmischung, zu erkennen. Durch Integration der Peakfläche erhält man den Wert für die Ionendichte der untersuchten Mischung. Pro Mischung werden vier Testzellen gemessen. Die Wiederholfrequenz der Dreiecksspannung beträgt 0,033 Hz, die Messtemperatur 60°C, die Maximalspannung ± 3 V bis ± 10 V je nach der Größe der dielektrischen Anisotropie der betreffenden Mischung.

Die Rotationsviskosität wird mit der Methode des rotierenden Permanentmagneten und die Fließviskosität in einem modifizierten Ubbelohde-Viskosimeter bestimmt. Für die Flüssigkristallmischungen ZLI-2293, ZLI-4792 und MLC-6608, alle Produkte der Firma Merck KGaA, Darmstadt, Deutschland, betragen die bei 20°C bestimmten Werte der Rotationsviskosität 161 mPa·s, 133 mPa·s bzw. 186 mPa·s und die der Fließviskosität (v) 21 mm²·s⁻¹, 14 mm²·s⁻¹ bzw. 27 mm²·s⁻¹.

Es werden, wenn nicht explizit anders angegeben, die folgenden Symbole verwendet:
- Vₒ: Schwellenspannung, kapazitiv [V] bei 20°C,
- nₑ: außerordentlicher Brechungsindex gemessen bei 20°C und 589 nm,
- nₒ: ordentlicher Brechungsindex gemessen bei 20°C und 589 nm,
- Δn: optische Anisotropie gemessen bei 20°C und 589 nm,
- ei: dielektrische Suszeptibilität senkrecht zum Direktor bei 20°C und 1 kHz,
- ε||: dielektrische Suszeptibilität parallel zum Direktor bei 20°C und 1 kHz,
- Δε: dielektrische Anisotropie bei 20°C und 1 kHz,
- cp. bzw. T(N,I): Klärpunkt [°C],
- ν: Fließviskosität gemessen bei 20°C [mm²·s⁻¹],
- γ₁: Rotationsviskosität gemessen bei 20°C [mPa·s],
- K₁: elastische Konstante, "splay"-Deformation bei 20°C [pN],
- K₂: elastische Konstante, "twist"-Deformation bei 20°C [pN],
- K₃: elastische Konstante, "bend"-Deformation bei 20°C [pN] und
- LTS: Tieftemperaturstabilität der Phase ("low temperature stability"), bestimmt in Testzellen,
- VHR: Spannungshaltevermögen ("voltage holding ratio"),

Die folgenden Beispiele erläutern die vorliegende Erfindung ohne sie zu begrenzen. Sie zeigen dem Fachmann jedoch bevorzugte Mischungskonzepte mit bevorzugt einzusetzenden Verbindungen und deren jeweiligen Konzentrationen sowie deren Kombinationen miteinander. Außerdem illustrieren die Beispiele, welche Eigenschaften und Eigenschaftskombinationen zugänglich sind.
In der vorliegenden Anmeldung und in den folgenden Beispielen sind die Strukturen der Flüssigkristallverbindungen durch Acronyme angegeben, wobei die Transformation in chemische Formeln gemäß Tabelle A erfolgt. Alle Reste CₙH₂ₙ₊₁ und CₘH₂ₘ₊₁ sind geradkettige Alkylreste mit n bzw. m C-Atomen; n, m und k sind ganze Zahlen und bedeuten vorzugsweise 0, 1, 2, 3, 4, 5, 6, 7, 8, 9, 10, 11 oder 12. Die Codierung gemäß Tabelle B versteht sich von selbst. In Tabelle A ist nur das Acronym für den Grundkörper angegeben. Im Einzelfall folgt getrennt von Acronym für den Grundkörper mit einem Strick ein Code für die Substituenten R^{1*}, R^{2*}, L^{1*} und L^{2*}:

| Code für R^{1*}, R^{2*}, L^{1*}, L^{2*}, L^{3*} | R^{1*} | R^{2*} | L^{1*} | L^{2*} |
|---|---|---|---|---|
| nm | CnH2n+₁ | CmH2m+₁ | H | H |
| nOm | CₙH₂ₙ₊₁ | OCmH2m+₁ | H | H |
| nO.m | OCₙH₂ₙ₊₁ | CmH2m+₁ | H | H |
| n | CₙH₂ₙ₊₁ | CN | H | H |
| nN.F | CₙH₂ₙ₊₁ | CN | F | H |
| nN.F.F | CₙH₂ₙ₊₁ | CN | F | F |
| nF | CₙH₂ₙ₊₁ | F | H | H |
| nCI | CₙH₂ₙ₊₁ | Cl | H | H |
| nOF | OCₙH₂ₙ₊₁ | F | H | H |
| nF.F | CₙH₂ₙ₊₁ | F | F | H |
| nF.F.F | CₙH₂ₙ₊₁ | F | F | F |
| nOCF₃ | CₙH₂ₙ₊₁ | OCF₃ | H | H |
| NOCH3.F | CₙH₂ₙ₊₁ | OCF₃ | F | H |
| n-Vm | CₙH₂ₙ₊₁ | -CH=CH-CₘH₂ₘ₊₁ | H | H |
| nV-Vm | CnH2n+1-CH=CH- | -CH=CH-CₘH₂ₘ₊₁ | H | H |

Bevorzugte Mischungskomponenten finden sich in den Tabellen A und B.

**Tabelle A**

| | |
|---|---|
| 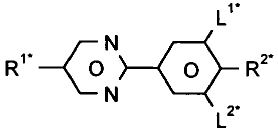 | 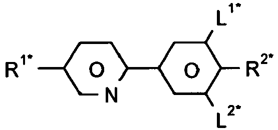 |
| **PYP** | **PYRP** |
| 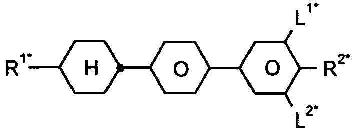 | 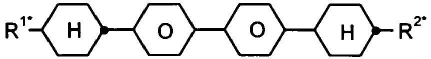 |
| **BCH** | **CBC** |
| 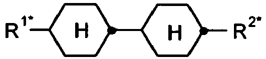 | 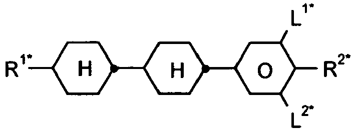 |
| **CCH** | **CCP** |
| 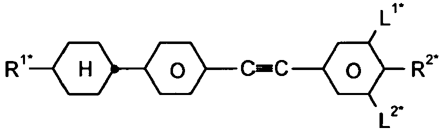 | 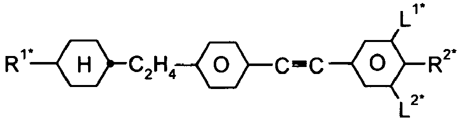 |
| **CPTP** | **CEPTP** |
| 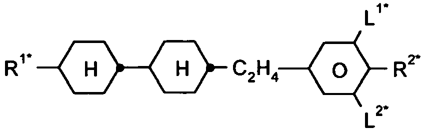 | 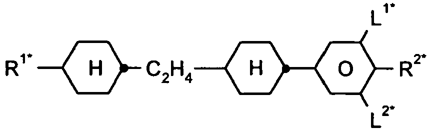 |
| **ECCP** | **CECP** |
| 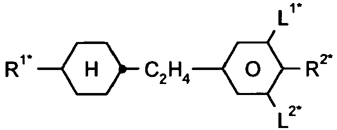 | 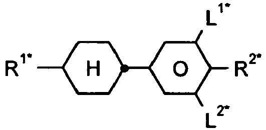 |
| **EPCH** | **PCH** |
| 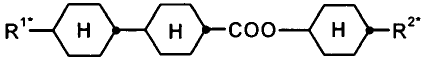 | |
| **CH** | |
| 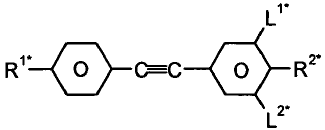 | 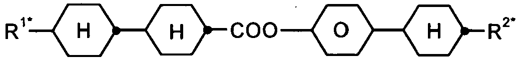 |
| **PTP** | **CCPC** |
| 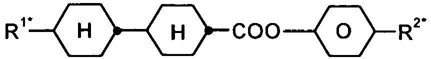 | 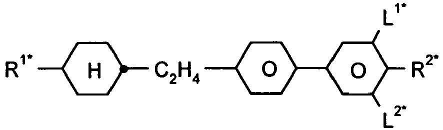 |
| **CP** | **BECH** |
| 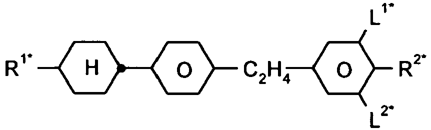 | 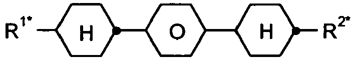 |
| **EBCH** | **CPC** |
| 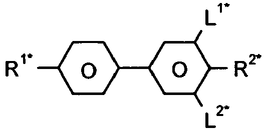 | 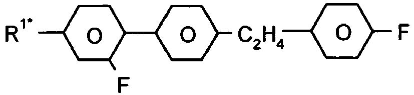 |
| **B** | **FET-nF** |
| 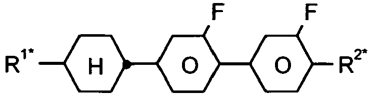 | 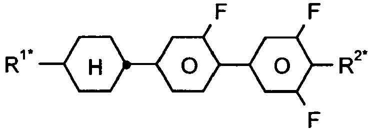 |
| **CGG** | **CGU** |
| 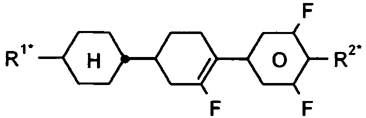 | |
| **CFU** | |

**Tabelle B**

| | |
|---|---|
| (n, m, I = 1-15; (0)CnH2n+i bedeutet CnH2n+i oder OCnH2n+i) | |
| 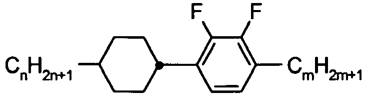 | |
| **CY-n-m** | |
| 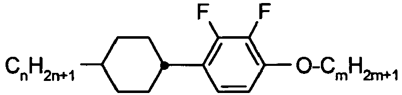 | |
| **CY-n-0m** | |
| 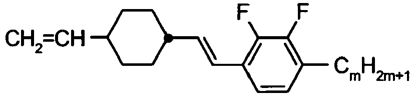 | |
| **CVY-n-m** | |
| 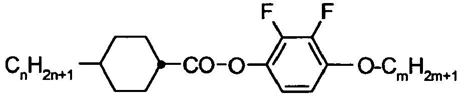 | |
| **CZY-n-Om** | |
| 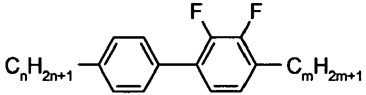 | |
| **PY-n-m** | |
| 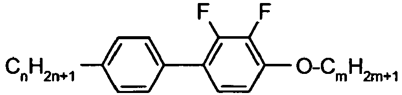 | |
| **PY-n-Om** | |
| 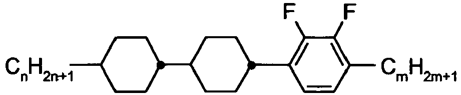 | |
| **CCY-n-m** | |
| 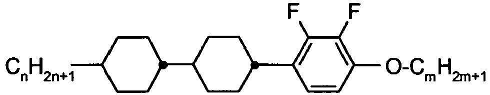 | |
| **CCY-n-Om** | |
| 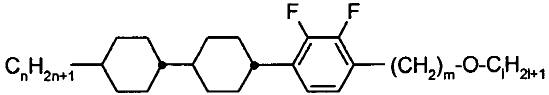 | |
| **CCY-n-m01** | |
| 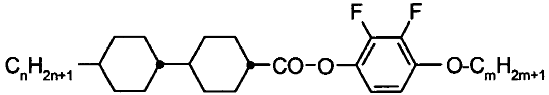 | |
| **CCZY-n-Om** | |
| 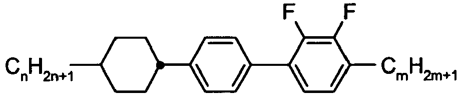 | |
| **CPY-n-m** | |
| 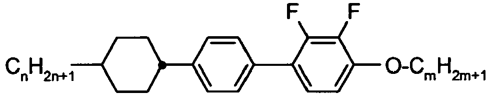 | |
| **CPY-n-0m** | |
| 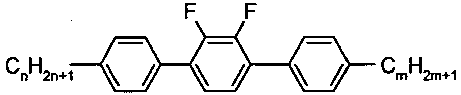 | |
| **PYP-n-m** | |
| 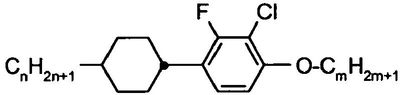 | |
| **CP(F,CI)-n-Om** | |
| 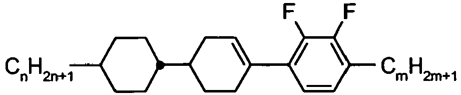 | |
| **CLY-n-m** | |
| 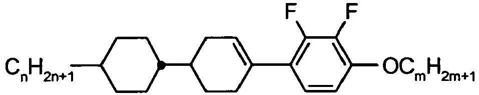 | |
| **CLY-n-Om** | |
| 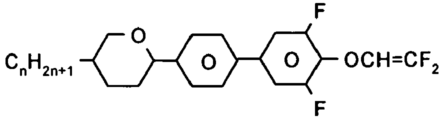 | |
| **APU-n-OXF** | |
| 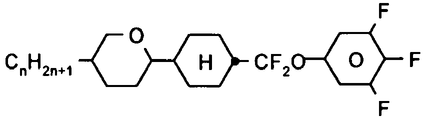 | |
| **ACQU-n-F** | |
| 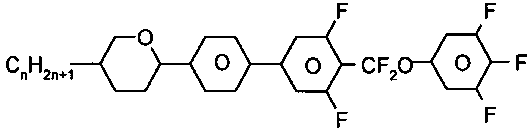 | |
| **APUQU-n-F** | |
| 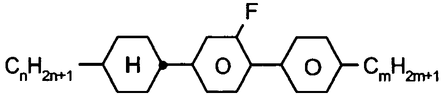 | 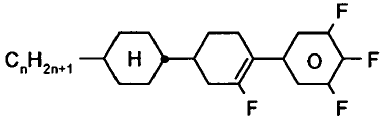 |
| **BCH-n.Fm** | **CFU-n-F** |
| 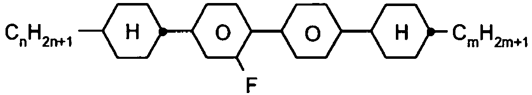 | |
| **CBC-nmF** | |
| 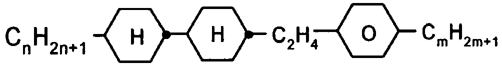 | 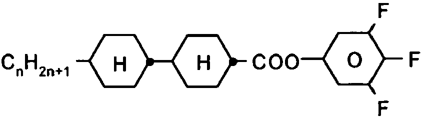 |
| **ECCP-nm** | **CCZU-n-F** |
| 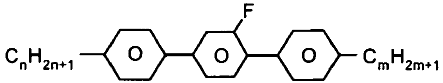 | 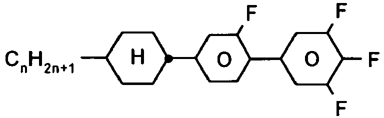 |
| **PGP-n-m** | **CGU-n-F** |
| 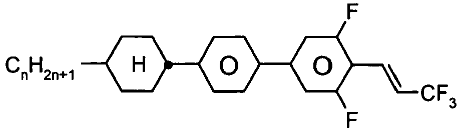 | 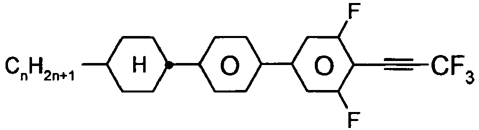 |
| **CPU-n-VT** | **CPU-n-AT** |
| 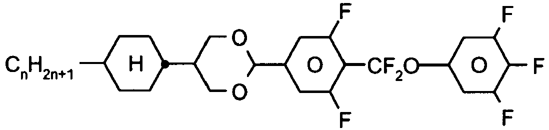 | |
| **CDUQU-n-F** | |
| 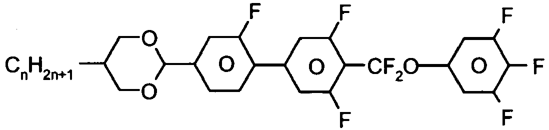 | |
| **DGUQU-n-F** | |
| 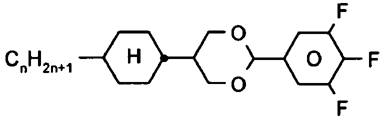 | 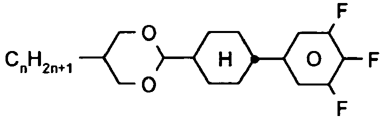 |
| **CDU-n-F** | **DCU-n-F** |
| 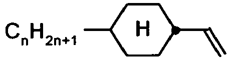 | 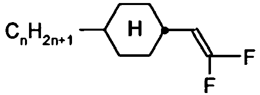 |
| **C-n-V** | **C-n-XF** |
| 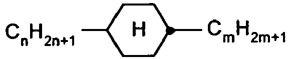 | 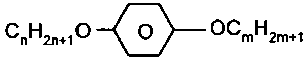 |
| **C-n-m** | **Y-nO-Om** |
| 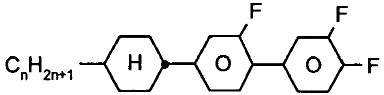 | 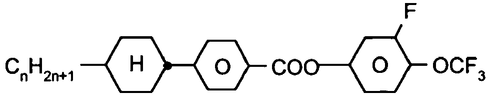 |
| **CGG-n-F** | **CPZG-n-OT** |
| 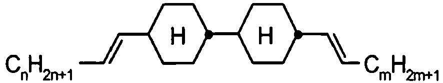 | 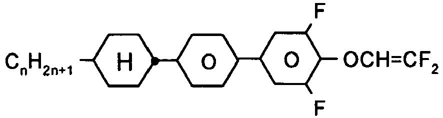 |
| **CC-nV-Vm** | **CPU-n-OXF** |
| 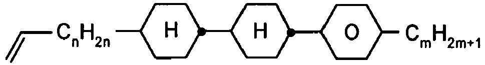 | 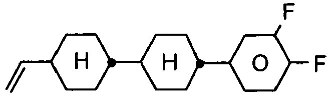 |
| **CCP-Vn-m** | **CCG-V-F** |
| 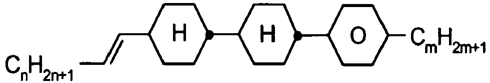 | 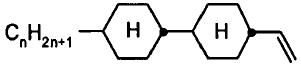 |
| **CCP-nV-m** | **CC-n-V** |
| 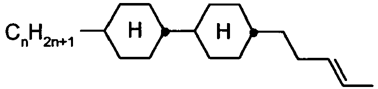 | 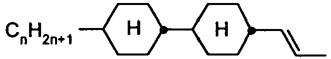 |
| **CC-n-2V1** | **CC-n-V1** |
| 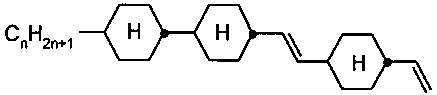 | |
| **CCVC-n-V** | |
| 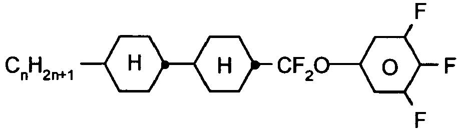 | 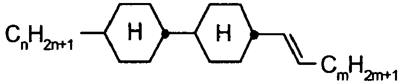 |
| **CCQU-n-F** | **CC-n-Vm** |
| 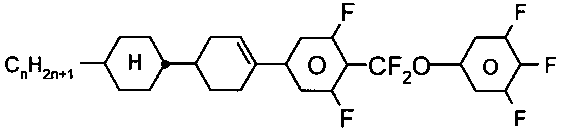 | |
| **CLUQU-n-F** | |
| 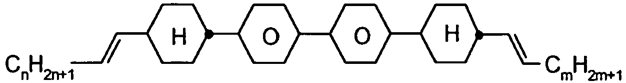 | |
| **CPPC-nV-Vm** | |
| 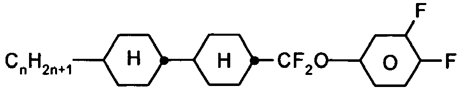 | 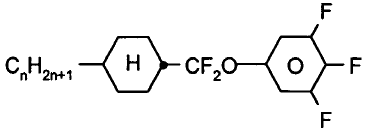 |
| **CCQG-n-F** | **CQU-n-F** |
| 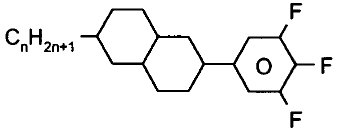 | 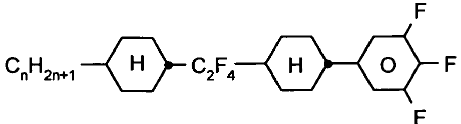 |
| **Dec-U-n-F** | **CWCU-n-F** |
| 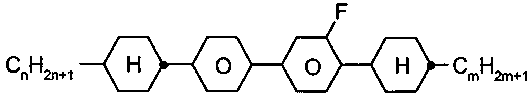 | |
| **CPGP-n-m** | |
| 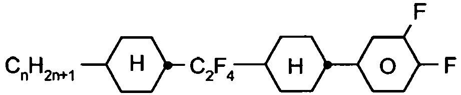 | |
| **CWCG-n-F** | |
| 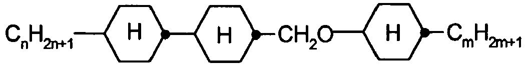 | |
| **CCOC-n-m** | |
| 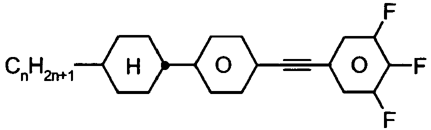 | 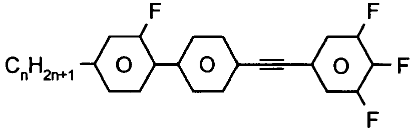 |
| **CPTU-n-F** | **GPTU-n-F** |
| 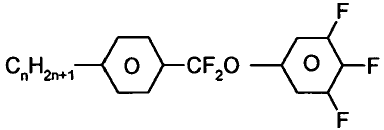 | 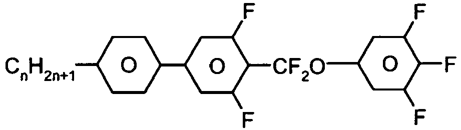 |
| **PQU-n-F** | **PUQU-n-F** |
| 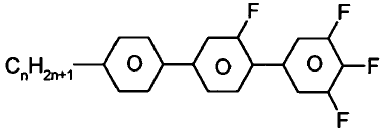 | 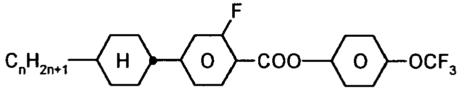 |
| **PGU-n-F** | **CGZP-n-OT** |
| 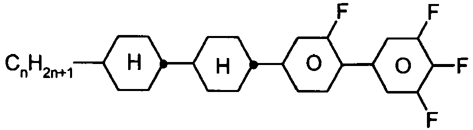 | 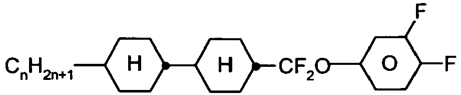 |
| **CCGU-n-F** | **CCQG-n-F** |
| 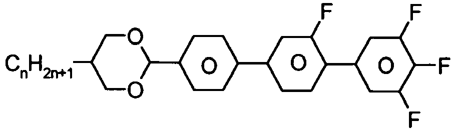 | 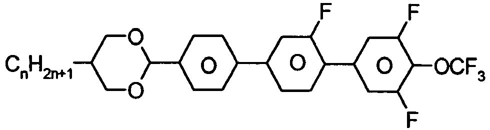 |
| **DPGU-n-F** | **DPGU-n-OT** |
| 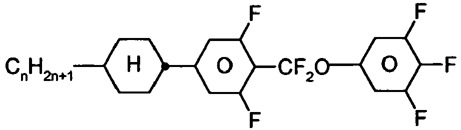 | 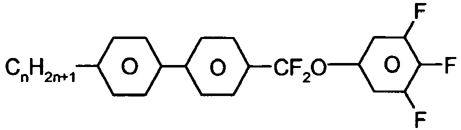 |
| **CUQU-n-F** | **PPQU-n-F** |
| 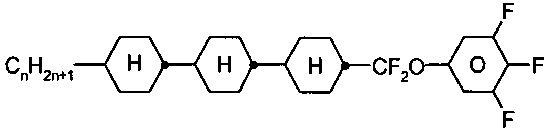 | |
| **CCCQU-n-F** | |
| 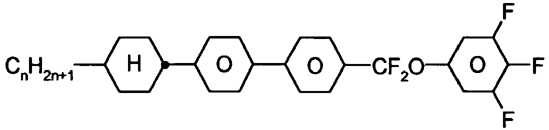 | |
| **CPPQU-n-F** | |
| 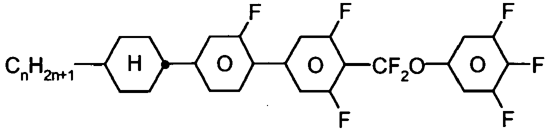 | |
| **CGUQU-n-F** | |
| 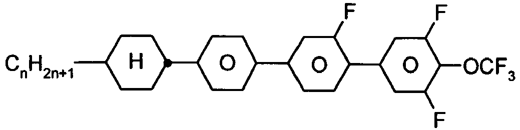 | |
| **CPGU-n-OT** | |
| 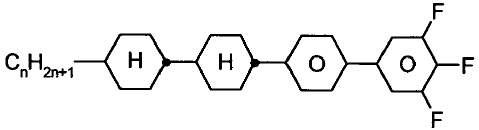 | |
| **CCPU-n-F** | |
| 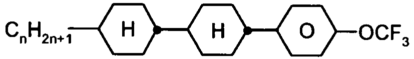 | |
| **CCP-nOCF₃** | |
| 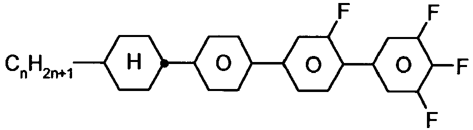 | |
| **CPGU-n-F** | |
| 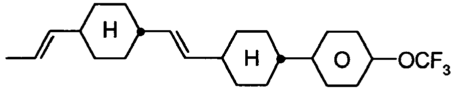 | 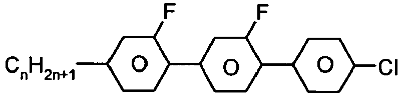 |
| **CVCP-1V-OT** | **GGP-n-Cl** |
| 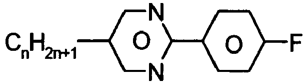 | |
| **PYP-nF** | |
| 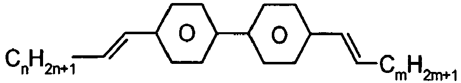 | 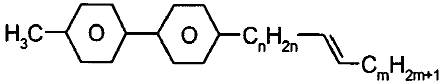 |
| **PP-nV-Vm** | **PP-1-nVm** |
| 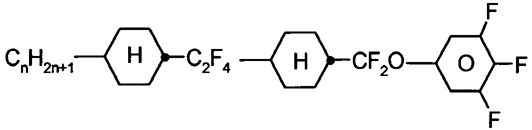 | |
| **CWCQU-n-F** | |
| 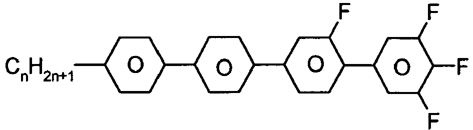 | |
| **PPGU-n-F** | |
| 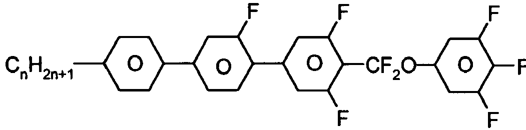 | |
| **PGUQU-n-F** | |
| 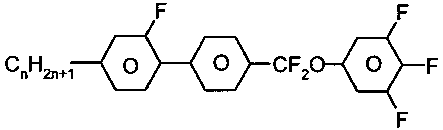 | 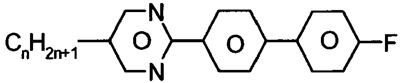 |
| **GPQU-n-F** | **MPP-n-F** |
| 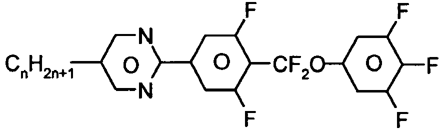 | 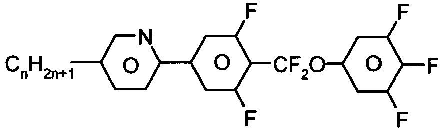 |
| **MUQU-n-F** | **NUQU-n-F** |
| 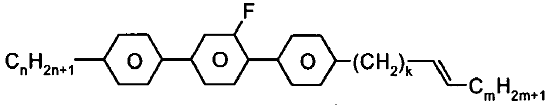 | |
| **PGP-n-kVm** | |
| 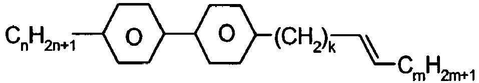 | |
| **PP-n-kVm** | |
| 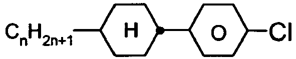 | 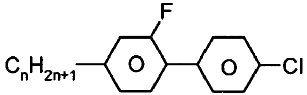 |
| **PCH-nCl** | **GP-n-Cl** |
| 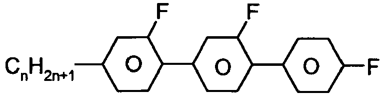 | 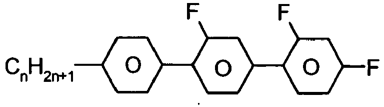 |
| **GGP-n-F** | **PGIGI-n-F** |
| 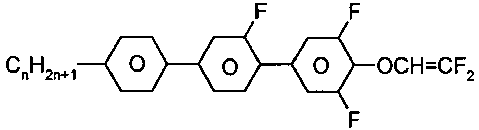 | |
| **PGU-n-OXF** | |

Besonders bevorzugt sind flüssigkristalline Mischungen, die neben den Verbindungen der Formeln I mindestens ein, zwei, drei, vier oder mehr Verbindungen aus der Tabelle B enthalten.

**Tabelle C**

| In der Tabelle C werden mögliche Dotierstoffe angegeben, die in der Regel den erfindungsgemäßen Mischungen zugesetzt werden. Vorzugsweise enthalten die Mischungen 0-10 Gew.%, insbesondere 0,01-5 Gew.% und besonders bevorzugt 0,01-3 Gew.% an Dotierstoffen. | |
|---|---|
| 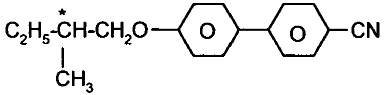 | 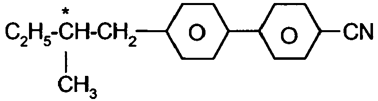 |
| **C 15** | **CB 15** |
| 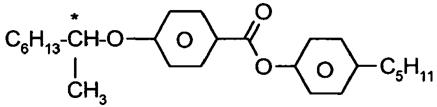 | 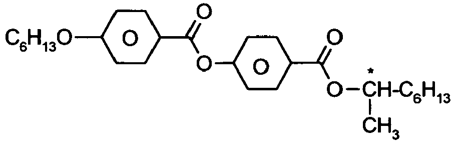 |
| **CM 21** | **R/S-811** |
| 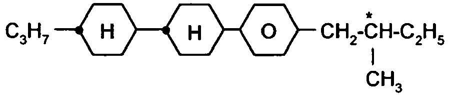 | 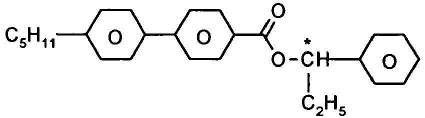 |
| **CM 44** | **CM 45** |
| 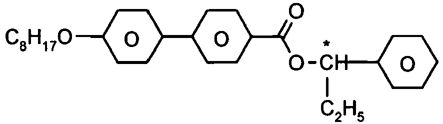 | 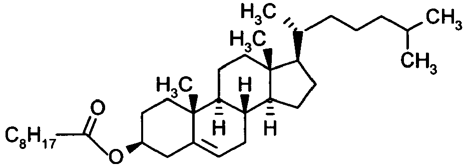 |
| **CM 47** | **CN** |
| 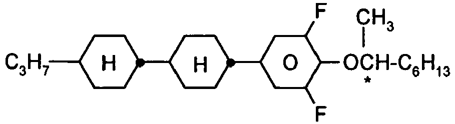 | 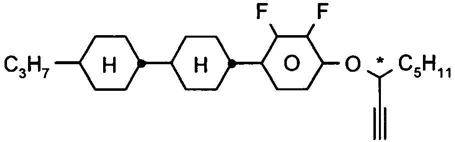 |
| **RIS-2011** | **R/S-3011** |
| 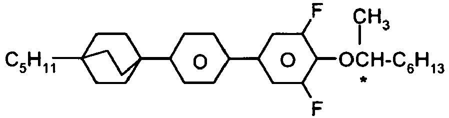 | 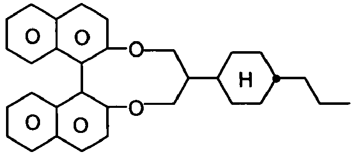 |
| **RIS-4011** | **R/S-5011** |
| 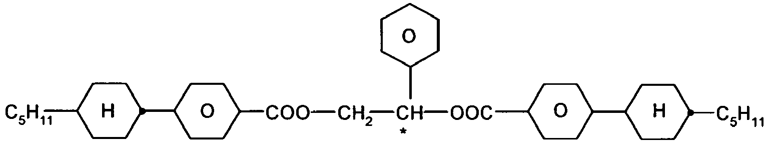 | |
| **R/S-1011** | |

**Tabelle D**

| Stabilisatoren, die beispielsweise den erfindungsgemäßen Mischungen in Mengen von 0-10 Gew.% zugesetzt werden können, werden nachfolgend genannt. | |
|---|---|
| 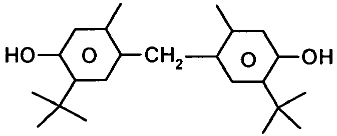 | 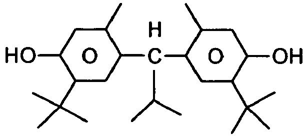 |
| 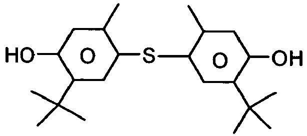 | 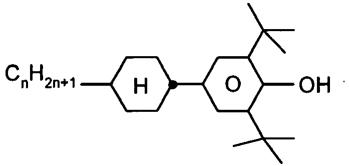 |
| | n=1, 2, 3, 4, 5, 6 oder 7 |
| 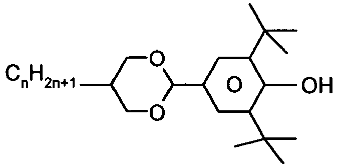 | |
| n = 1, 2, 3, 4, 5, 6 oder 7 | |
| 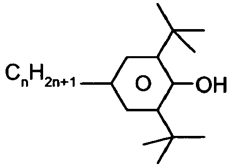 | 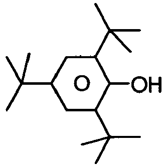 |
| n = 1, 2, 3, 4, 5, 6 oder 7 | |
| 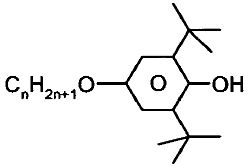 | 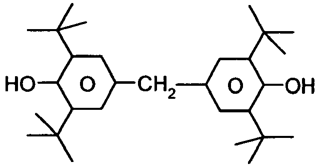 |
| 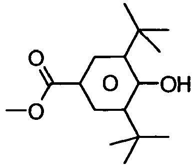 | 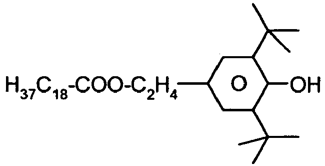 |
| 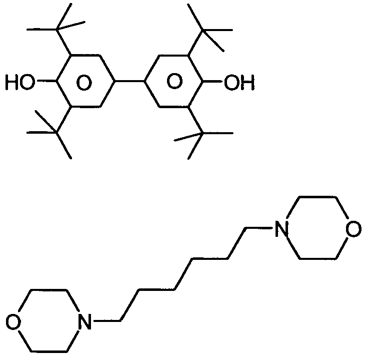 | 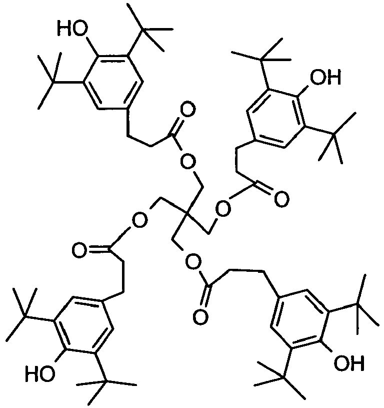 |
| 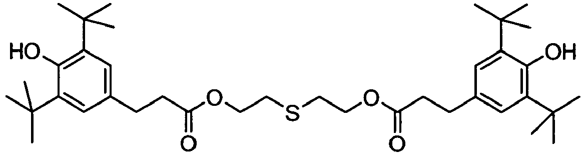 | |
| 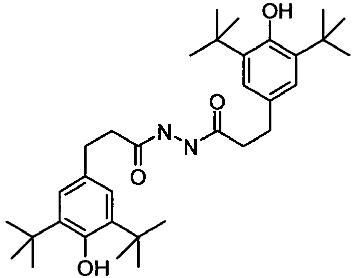 | 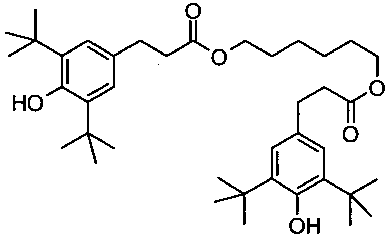 |
| 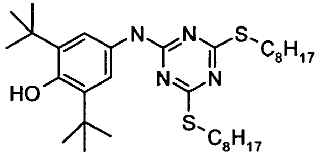 | 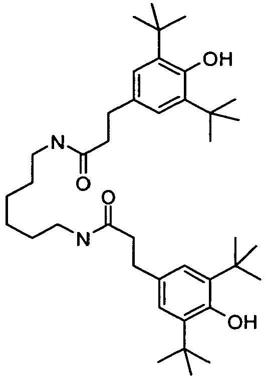 |
| 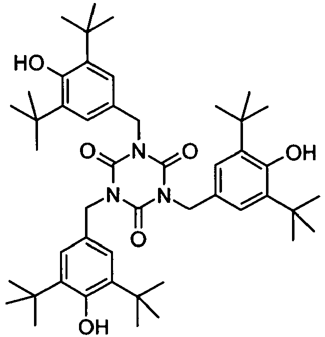 | 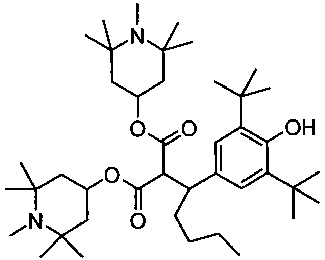 |
| 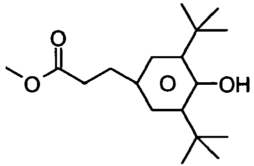 | 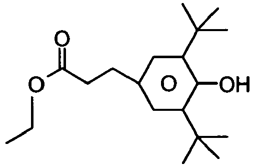 |
| 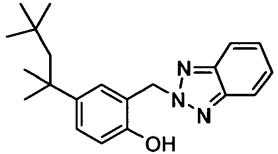 | 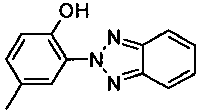 |
| 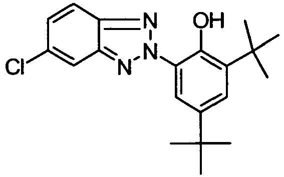 | 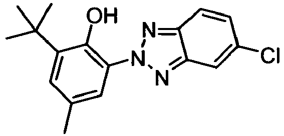 |
| 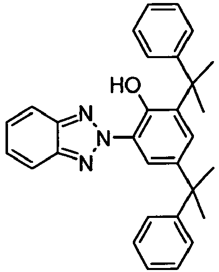 | 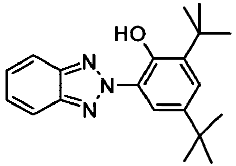 |
| 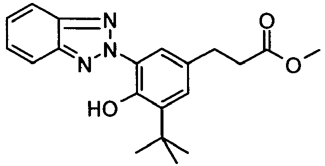 | 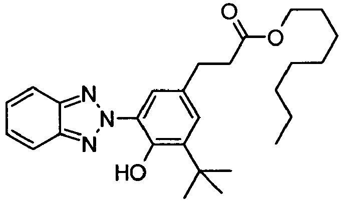 |
| 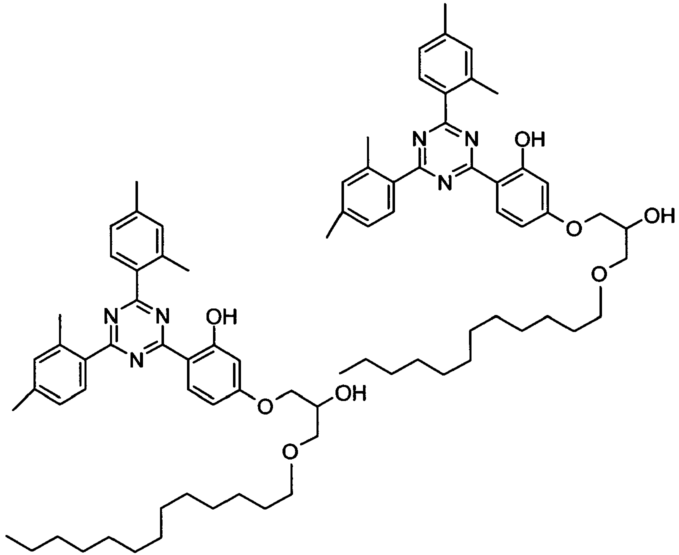 | |
| 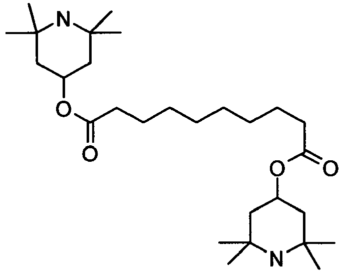 | 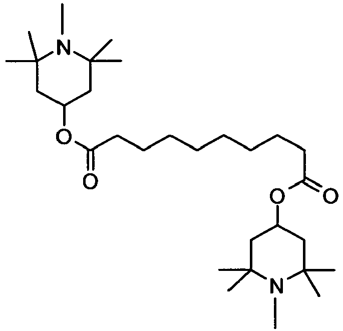 |
| 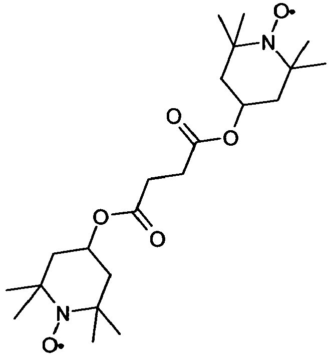 | 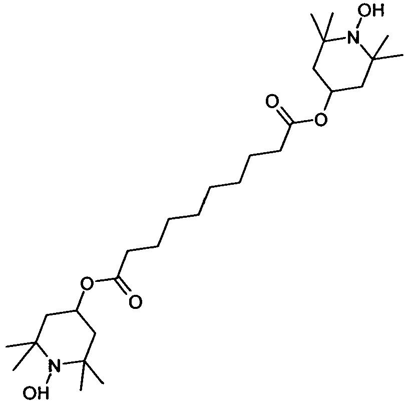 |

**Tabelle E**

| In der Tabelle E sind Beispielverbindungen zusammengestellt, die in den FK-Medien gemäß der vorliegenden Erfindung vorzugsweise als reaktive mesogene Verbindungen verwendet werden können. Sofern die erfindungsgemäßen Mischungen ein oder mehrere reaktive Verbindungen enthalten, werden sie vorzugsweise in Mengen von 0,01-5 Gew.% eingesetzt. Gegebenenfalls muss für die Polymerisation noch ein Initiator oder ein Gemisch aus zwei oder mehr Initiatoren zugesetzt werden. Der Initiator oder das Initiatorgemisch wird vorzugsweise in Mengen von 0,001-2 Gew.% bezogen auf die Mischung zugesetzt. Ein geeigneter Initiator ist z. B. Irgacure (Fa. BASF) oder Irganox (Fa. BASF). | |
|---|---|
| 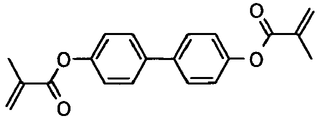 | RM-1 |
| 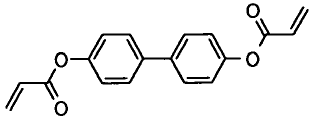 | RM-2 |
| 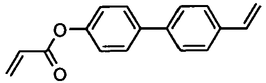 | RM-3 |
| 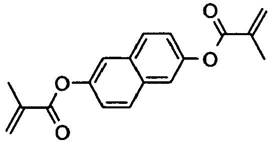 | RM-4 |
| 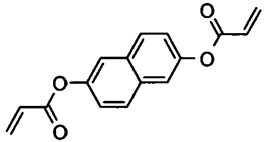 | RM-5 |
| 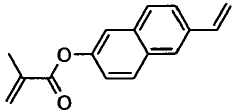 | RM-6 |
| 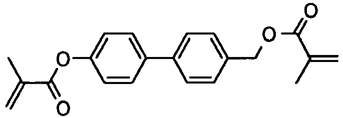 | RM-7 |
| 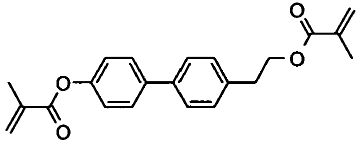 | RM-8 |
| 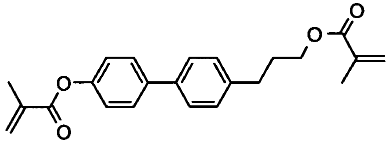 | RM-9 |
| 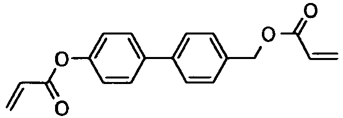 | RM-10 |
| 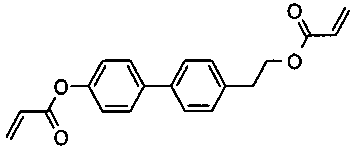 | RM-11 |
| 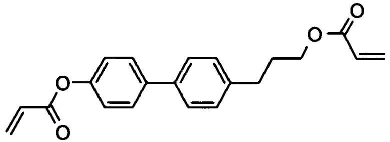 | RM-12 |
| 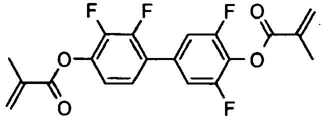 | RM-13 |
| 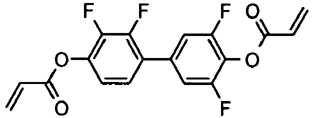 | RM-14 |
| 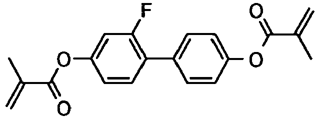 | RM-15 |
| 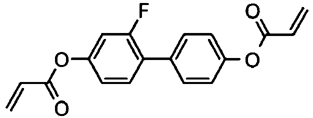 | RM-16 |
| 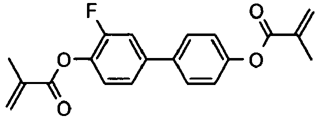 | RM-17 |
| 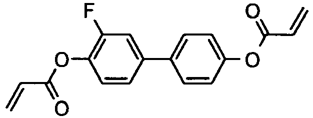 | RM-18 |
| 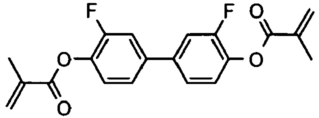 | RM-19 |
| 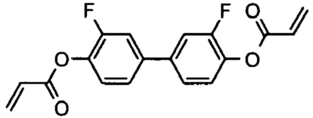 | RM-20 |
| 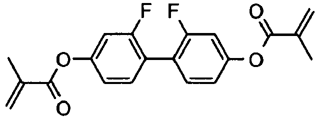 | RM-21 |
| 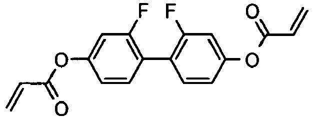 | RM-22 |
| 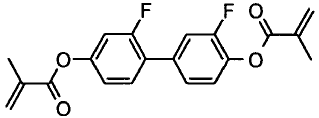 | RM-23 |
| 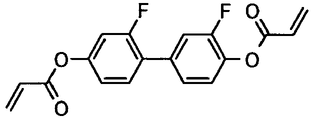 | RM-24 |
| 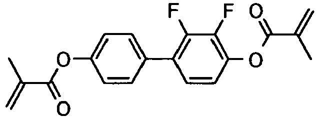 | RM-25 |
| 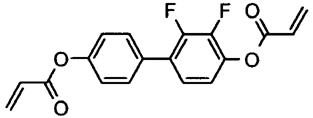 | RM-26 |
| 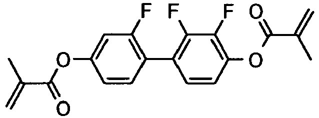 | RM-27 |
| 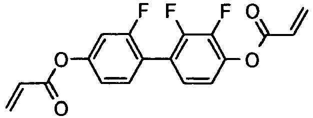 | RM-28 |
| 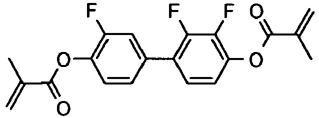 | RM-29 |
| 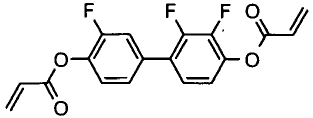 | RM-30 |
| 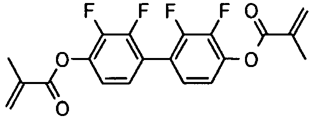 | RM-31 |
| 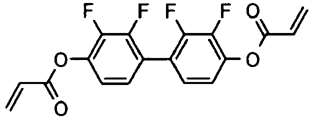 | RM-32 |
| 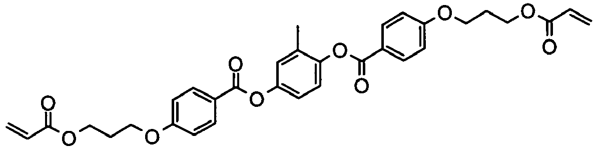 | RM-33 |
| 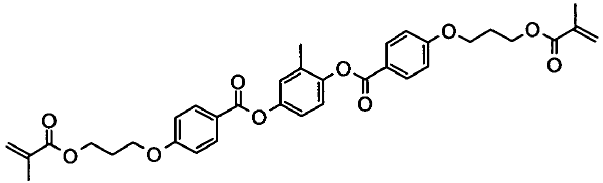 | RM-34 |
| 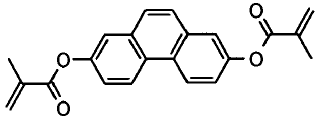 | RM-35 |
| 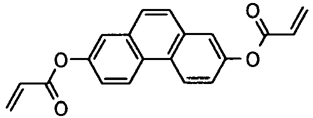 | RM-36 |
| 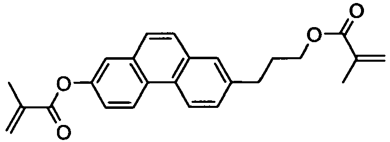 | RM-37 |
| 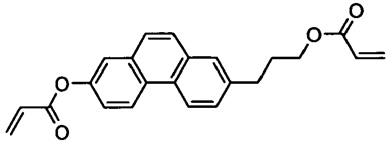 | RM-38 |
| 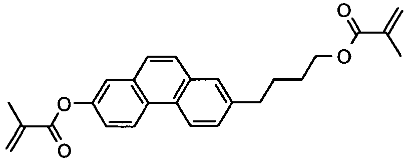 | RM-39 |
| 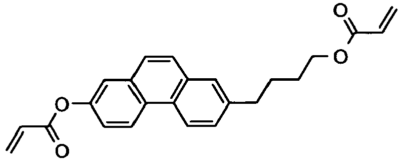 | RM-40 |
| 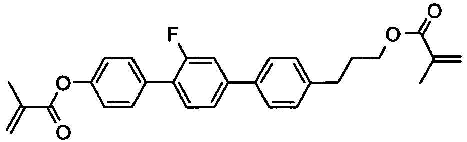 | RM-41 |
| 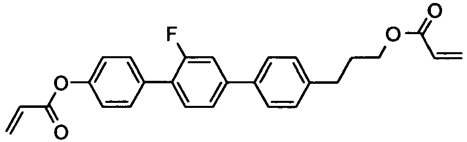 | RM-42 |
| 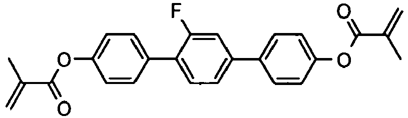 | RM-43 |
| 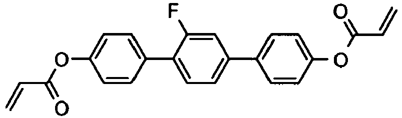 | RM-44 |
| 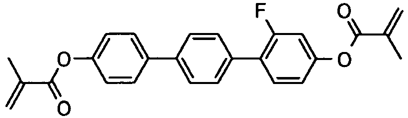 | RM-45 |
| 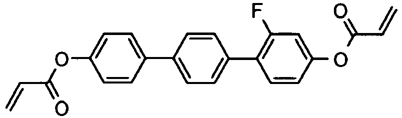 | RM-46 |
| 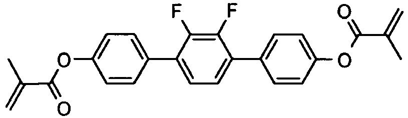 | RM-47 |
| 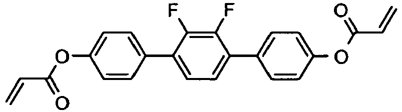 | RM-48 |
| 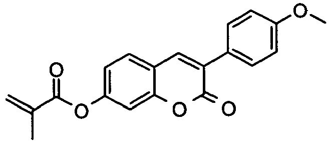 | RM-49 |
| 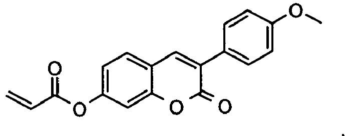 | RM-50 |
| 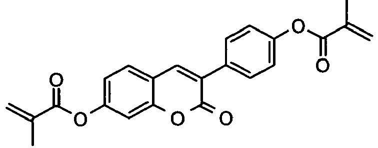 | RM-51 |
| 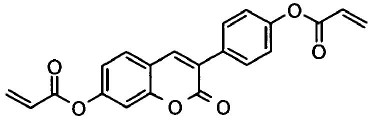 | RM-52 |
| 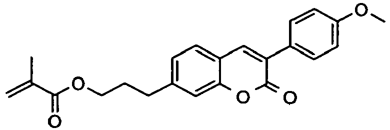 | RM-53 |
| 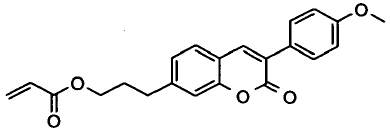 | RM-54 |
| 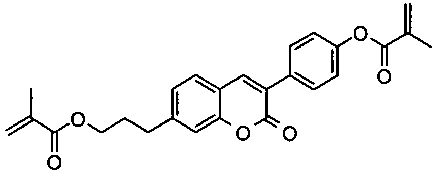 | RM-55 |
| 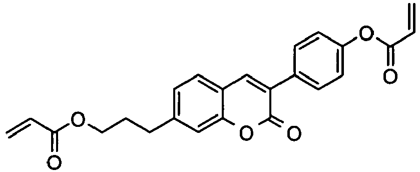 | RM-56 |
| 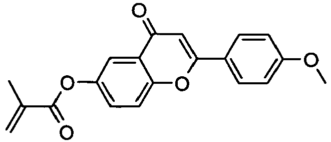 | RM-57 |
| 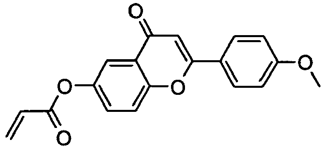 | RM-58 |
| 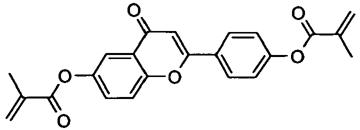 | RM-59 |
| 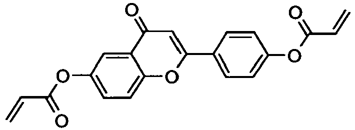 | RM-60 |
| 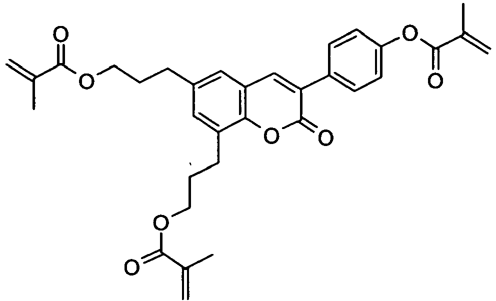 | RM-61 |
| 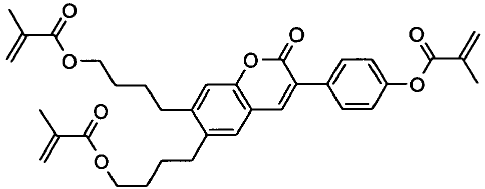 | RM-62 |
| 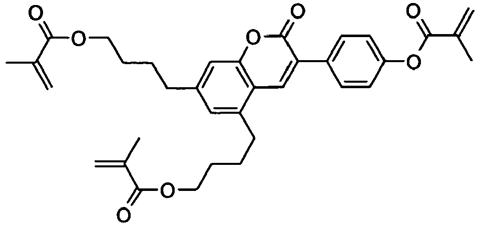 | RM-63 |
| 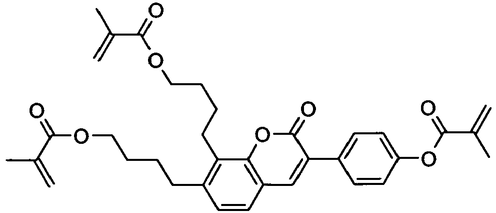 | RM-64 |
| 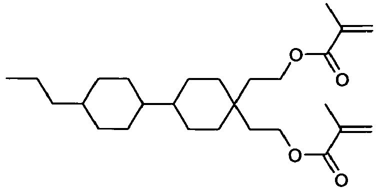 | RM-65 |
| 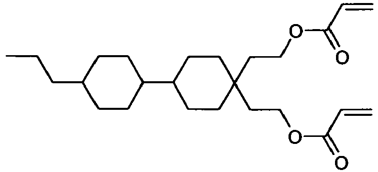 | RM-66 |
| 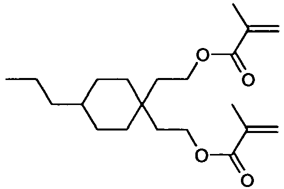 | RM-67 |
| 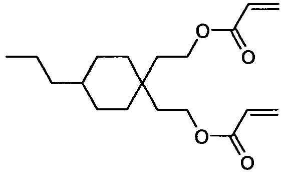 | RM-68 |
| 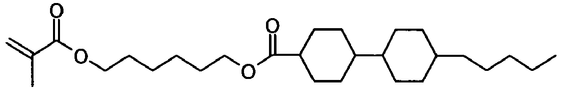 | RM-69 |
| 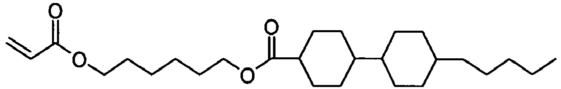 | RM-70 |
| 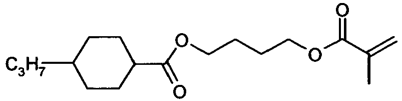 | RM-71 |
| 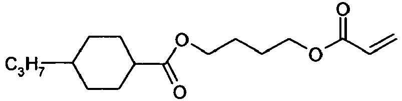 | RM-72 |
| 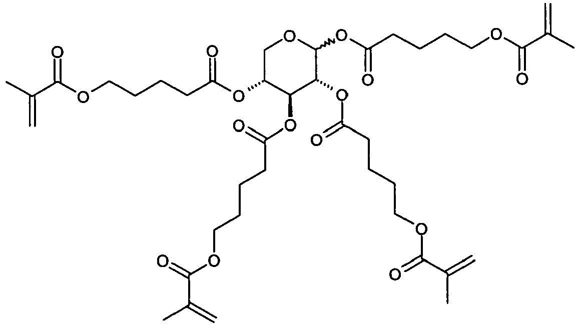 | RM-73 |
| 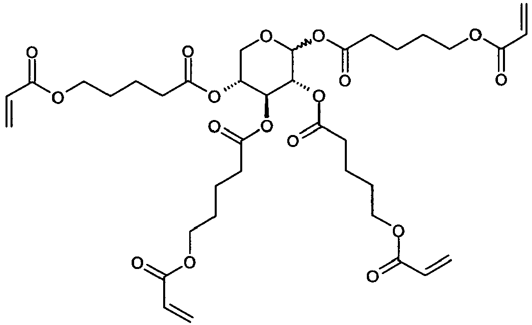 | RM-74 |
| 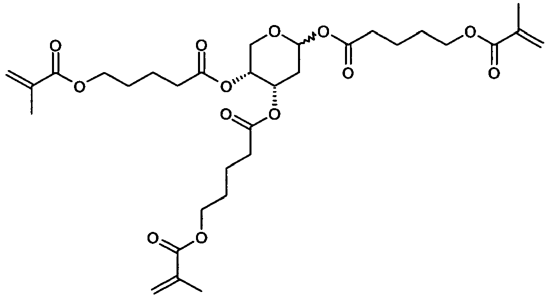 | RM-75 |
| 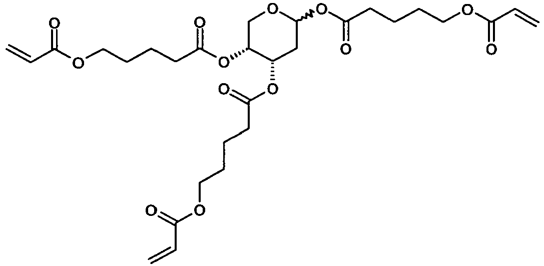 | RM-76 |
| 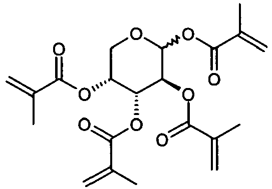 | RM-77 |
| 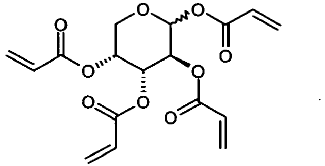 | RM-78 |
| 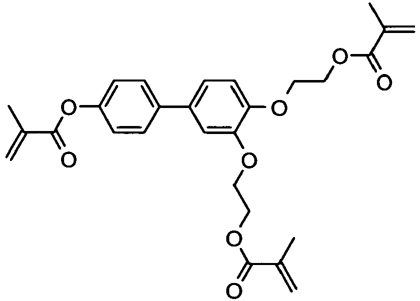 | RM-79 |
| 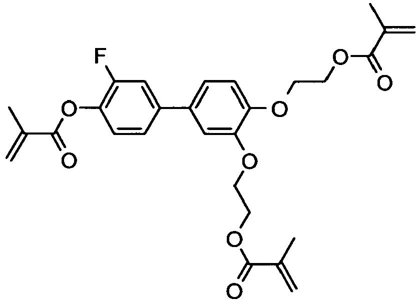 | RM-80 |
| 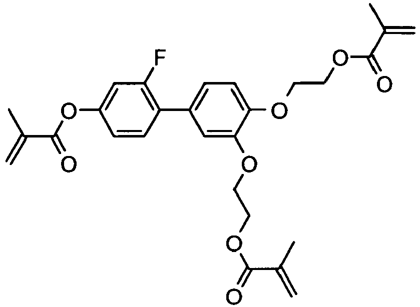 | RM-81 |
| 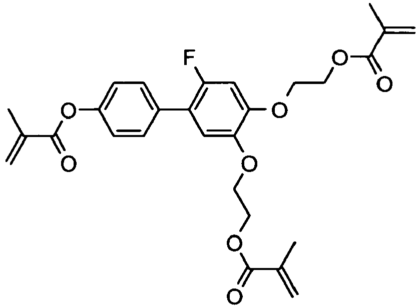 | RM-82 |
| 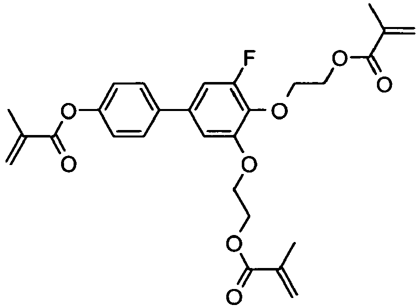 | RM-83 |

In einer bevorzugten Ausführungsform enthalten die erfindungsgemäßen Mischungen eine oder mehrere polymerisierbare Verbindungen, vorzugsweise ausgewählt aus den polymerisierbaren Verbindungen der Formeln RM-1 bis RM-83. Derartige Medien sind insbesondere für PS-VA, PS-FFS- und PS-IPS-Anwendungen geeignet. Von den in der Tabelle E genannten reaktiven Mesogenen sind die Verbindungen RM-1, RM-2, RM-3, RM-4, RM-5, RM-11, RM-17, RM-35, RM-41, RM-61 und RM-80 insbesondere bevorzugt.

### Beispiele

Die folgenden Ausführungsbeispiele sollen die Erfindung erläutern, ohne sie zu begrenzen.

### Synthesebeispiele

"Übliche Aufarbeitung" bedeutet: man gibt gegebenenfalls Wasser hinzu, extrahiert mit Methylenchlorid, Diethylether, Methyl-tert.Butylether (MTBE) oder Toluol, trennt ab, trocknet die organische Phase, dampft ein und reinigt das Produkt durch Destillation unter reduziertem Druck oder Kristallisation und/oder Chromatographie.

Synthese 2-Ethyl-decandisäure bis-(2,2,6,6-tetramethyl-piperidin-4-yl) ester **(1)**

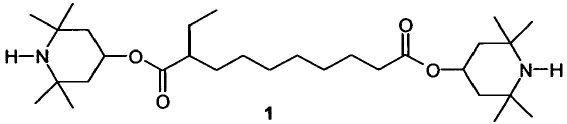

### 1) Synthese von 2-Ethoxycarbonyl-2-ethyl-decanedioic acid diethyl ester A

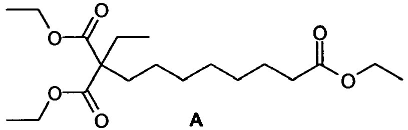

194.0 mL (1.035 mol) Diethylmalonat werden in 775.0 mL Tetrahydrofuran (THF) vorgelegt und bei Raumtemperatur (RT) tropfenweise mit 497.7 mL (0.995 mol) Lithiumdisopropylamid versetzt. Die Reaktionslösung erwärmt sich dabei auf 45°C. Es wird auf 30°C abkühlen gelassen und mit 200.0 g (796.3 mmol) 8-Bromoktansäureethylester, gelöst in 515.0 mL THF, tropfenweise versetzt. Die Reaktionsmischung wird nun auf 60°C erwärmt und für 56 h gerührt. Es wird auf RT abkühlen gelassen, vorsichtig mit Wasser versetzt und anschließend mit verdünnter Salzsäure vorsichtig neutralisiert. Das Reaktionsprodukt wird mit Methyl-tertbutylether (MTB-Ether) extrahiert, über Natriumsulfat getrocknet, filtriert und eingeengt. Das entstehende Rohprodukt wird ohne weitere Aufreinigung direkt weiter umgesetzt.

### 2) Synthese von 2-Carboxy-2-ethyl-decandisäure B

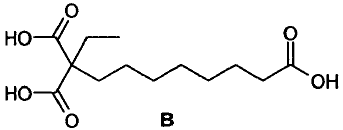

358.0 g (676.1 mmol, 67%ig) Carbonsäureester Rohprodukt **A** wird in 2300 mL Ethanol gelöst und mit 363.0 mL (3.92 mol, 32%ig) Natronlauge versetzt. Die Reaktionsmischung wird für 16h unter Rückfluss gerührt. Nach vollständiger Umsetzung wird das Reaktionsgemisch (es entsteht ein Niederschlag) langsam mit Wasser versetzt (der Niederschlag geht in Lösung) und vorsichtig mit 10%iger HCI auf pH = 1 angesäuert. Das Reaktionsprodukt wird mit MTB-Ether extrahiert. Die vereinigten organischen Phasen werden eingeengt und das noch enthaltene Wasser mit Toluol azeotrop abgetrennt. Das entstandene Rohprodukt wird ohne weitere Aufreinigung in den nächsten Syntheseschritt eingesetzt.

### 3) Synthese von 2-Ethyl-decandisäure C

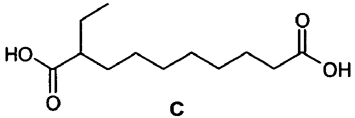

242.0 g (68.3%ig, 602.5 mmol) von Carbonsäure **B** werden ohne Lösungsmittel in einem Rundkolben mit Destillationsbrücke und Vorlage unter Ölpumpenvakuum (12 mbar) vorsichtig auf 250°C Badtemperatur erhitzt. Ab 160°C geht das Ölpumpenvakuum auf ca. 70 mbar zurück (CO₂-Abspaltung) und es destilliert eine leichtflüchtige Komponente in die Vorlage. Wenn die Gasentwicklung abgeschlossen ist, erreicht das Vakuum wieder 15 mbar. Das entstandene Rohprodukt wird anschließend bei 0.4-0.5 mbar und 192-198°C Kopftemperatur destilliert. Das erhaltene Destillat wird nun in 100 mL Ethanol und 100 mL Natronlauge (32%ig) gelöst und bei 40°C für 4h gerührt. (Hier werden Reste an Monoester gespalten.) Anschließend wird das Ethanol im Vakuum mittels Destillation abgetrennt und der Rückstand mit Wasser versetzt und vorsichtig mit 400 mL HCI (10%ig) angesäuert. Das Reaktionsprodukt wird mit MTB-Ether extrahiert, über Natriumsulfat getrocknet, filtriert und eingeengt. Man erhält 105 g des Produkts mit 97.1 % Reinheit was ohne weitere Aufreinigung in die nächste Synthesestufe eingesetzt wird.

¹H NMR (500 MHz, CDCl₃)
δ = 0.93 ppm (t, 7.45 Hz, 3 H, CH₃), 1.30 (s, 8 H, CH₂), 1.72 -1.42 (m, 6 H, CH₂), 2.28 (m_{c}, 1 H, CHtert(O)OH), 2.35 (t, 7.4 Hz, 2 H, C*H*₂C(O)OH), 10.73 (s_{(breit)}, 2 H, C(O)OH).

### 4) Synthese von Bis(2,2,6,6-tetramethyl-4-piperidinyl-1-oxyl)-2-Ethyl-decandisäure E

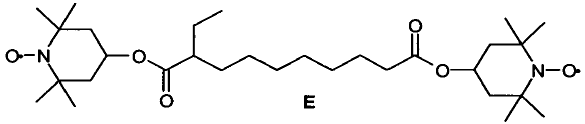

108.9 g (457.8 mmol) Carbonsäure **C**, 236.5 g (1.373 mol) 4-Hydroxy-2,2,6,6-tetramethylpiperidin-1-oxyl (freies Radikal) und 5.59 g 4-(Dimethylamino)-pyridin werden in 700 mL Dichlormethan gelöst und auf 2°C-5°C gekühlt. Es wird nun tropfenweise mit einer Lösung aus 297.5 g (1.442 mol) N,N'-Dicyclohexylcarbodiimid in 300 mL Dichlormethan versetzt. Das Kühlbad wird anschließend entfernt und die Reaktionsmischung wird für 16h bei Raumtemperatur (RT) gerührt. Nach beendeter Umsetzung wird vorsichtig mit 86.6 g (686.8 mmol) Oxalsäure-Dihydrat versetzt und für eine Stunde bei RT gerührt. Die Reaktionsmischung wird über eine Fritte abgesaugt und direkt über 41 Kieselgel mit Dichlormethan (DCM) / MTB-Ether (9:1) filtriert. Das erhaltene Reaktionsprodukt wird 2 Mal aus gut entgastem Acetonitril / Wasser (4:1) bei +3°C unter Rühren kristallisiert. Man erhält 133g des Produkts mit 99.8% Reinheit (HPLC).
MS (EI) 539.5 [M+H⁺]

### 5) Synthese von 2-Ethyl-decandisäure bis-(2,2,6,6-tetramethylpiperidin-4-yl) ester 1

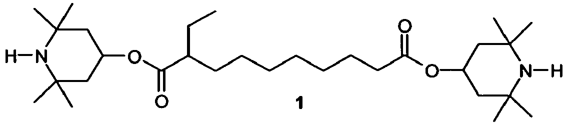

133.0 g (246.4 mmol) freies Radikal **E** wird in 1.33 I Tetrahydrofuran gelöst und mit 33.0 g Sponge-Nickel-Catalyst (watery, Johnson-Matthey) unter 5 bar Wasserstoffdruck bei 50°C für 83.8 h zum Amin reduziert. Das erhaltene Rohprodukt wird aus entgastem Heptan (1:10) bei -10°C kristallisiert. Das erhaltene Produkt wird anschließend mit MTB-Ether (Abtrennen von restlichem freien Radikal) und MTB-Ether / Ethanol (1:1) über 1 I Kieselgel filtriert. Das erhaltene Produkt wird erneut aus Heptan (1:10) bei -10°C kristallisiert und abgesaugt. Man erhält 83.2 g des gewünschten Produkts als weißen Feststoff mit einer Reinheit > 99.5% (HPLC und GC).

¹H NMR (500 MHz, CDCl₃)
δ = 0.89 ppm (t (überlagert), 7.46 Hz, 5 H, CH₃, 2 x NH), 1.20 - 1.07 (2 x s (überlagert), 16 H, CH₃, CH₂), 1.36 -1.2 (2 x s (überlagert), 20 H, CH₃, CH₂), 1.54 -1.38 (m, 2 H), 1.65 -1.54 (m_{c}, 4 H), 1.91 (dd, 12.8, 4.08 Hz, 4 H), 2.21 (m_{c}, 1 H, CHC(O)O), 2.27 (t, 7.46 Hz, 2H, C*H*₂C(O)O), 5.21 (m_{c}, 2 H, CHOC(O)).

In Analogie können folgende Verbindungen hergestellt werden:

| | |
|---|---|
| 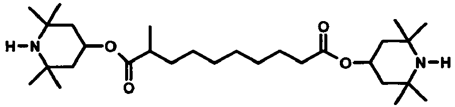 | 2 |
| 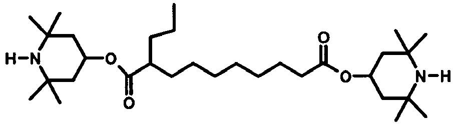 | 3 |
| 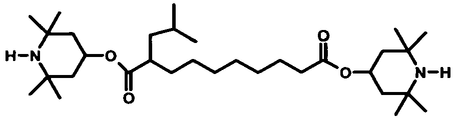 | 4 |

### Mischungsbeispiele

Die folgenden Mischungen M1 bis M3 werden hergestellt.

### Beispiel M1

| | | | |
|---|---|---|---|
| CC-3-V | 32,00 % | Klärpunkt [°C]: | 85,0 |
| CC-3-V1 | 11,00 % | | |
| CC-3-2V1 | 4,50 % | Δn [589 nm, 20 °C] | 0,1089 |
| PP-1-2V1 | 2,00 % | Δε [kHz, 20 °C]: | + 15,3 |
| CCP-3OCF3 | 7,50 % | γ₁ [mPa·s, 20 °C]: | 89 |
| CCP-5OCF3 | 1,50 % | K₁ [20 °C]: | 14,4 |
| PUQU-3-F | 1,50 % | K₃ [20 °C]: | 15,1 |
| APUQU-2-F | 7,00 % | V₀ [V]: | 1,01 |
| APUQU-3-F | 7,00 % | | |
| PGUQU-3-F | 3,00 % | | |
| PGUQU-4-F | 8,00 % | | |
| PGUQU-5-F | 2,00 % | | |
| DPGU-4-F | 5,00 % | | |
| DGUQU-4-F | 8,00 % | | |

### Beispiel M2

| | | | |
|---|---|---|---|
| CC-3-V | 26,50 % | Klärpunkt [°C]: | 94,5 |
| CC-3-V1 | 10,00 % | | |
| CC-3-2V1 | 6,50 % | Δn [589 nm, 20 °C] | 0,1035 |
| CCP-V2-1 | 10,00 % | Δε [kHz, 20 °C]: | + 17,2 |
| APUQU-2-F | 8,00 % | γ₁ [mPa·s, 20 °C]: | 109 |
| APUQU-3-F | 9,00 % | K₁ [20 °C]: | 15,8 |
| PGUQU-3-F | 3,00 % | K₃ [20 °C]: | 16,6 |
| CDUQU-3-F | 8,00 % | V₀ [V]: | 1,00 |
| DPGU-4-F | 4,00 % | | |
| DGUQU-4-F | 8,00 % | | |
| DGUQU-2-F | 3,00 % | | |
| PGU-4-T | 2,00 % | | |

### Beispiel M3

| | | | |
|---|---|---|---|
| CY-3-O2 | 12,00 % | Klärpunkt [°C]: | 86,5 |
| CY-3-O4 | 2,00 % | | |
| CY-5-O2 | 12,00 % | Δn [589 nm, 20 °C] | 0,1092 |
| CCY-3-O1 | 6,00 % | Δε [kHz, 20 °C]: | -4,2 |
| CCY-3-O2 | 8,00 % | γ₁ [mPa·s, 20 °C]: | 155 |
| CCY-4-O2 | 8,00 % | K₁ [20 °C]: | 14,6 |
| CPY-2-O2 | 9,00 % | K₃ [20 °C]: | 16,6 |
| CPY-3-O2 | 9,00 % | V₀ [V]: | 2,08 |
| PYP-2-3 | 5,00 % | | |
| CC-3-V1 | 5,00 % | | |
| CC-3-V | 19,00 % | | |
| BCH-32 | 5,00 % | | |

### Voltage Holding Ratio - Lichtbeständigkeit (Sonne)

Zunächst wird die Stabilität der Voltage Holding Ratio der jeweiligen Testmischung selbst, von einer weiteren Probe dieser Mischung, der die angegebenen Mengen der Verbindung TINUVIN®770 zugesetzt wurden, sowie von einer weiteren Probe dieser Mischung, der die angegebenen Mengen der Verbindung 1 aus Beispiel 1 zugesetzt wurden nach 5min bei 100°C, bei 1V und 60Hz bestimmt (VHR (initial)). Die resultierenden Mischungen werden in einer Testzelle mit einem Orientierungsmaterial für planare Ausrichtung und flächigen ITO-Elektroden auf ihre Lichtbeständigkeit (Sonne)_untersucht. Zur Bestimmung der VHR in Abhängigkeit der Sonnenlichtbeständigkeit wird eine Lampe die das Wellenlängen-Spektrum des Sonnenlichts emittiert verwendet. Der Test wird bei 20°C durchgeführt und die Bestrahlungsdauer entspricht 30 min. Die Voltage Holding Ratio wird nach 5 Minuten bei einer Temperatur von 100°C, 1V und 60Hz bestimmt (VHR (Sonne)). Die Ergebnisse sind in den folgenden Tabellen zusammengestellt. Andere Messbedingungen siehe Tabelle.

**Mischung M1**

| **Stabilisator** | **Konz. (ppm)** | **VHR (initial)** | **VHR (Sonne)** |
|---|---|---|---|
| - | - | 96,4 | 72,4 |
| TINUVIN®770 | 100 | 98,0 | 89,3 |
| TINUVIN®770 | 500 | 98,0 | 85,7 |
| TINUVIN®770 | 1000 | 97,8 | 85,9 |
| VERBINDUNG (1) | 100 | 98,0 | 88,7 |
| VERBINDUNG (1) | 500 | 97,6 | 82,9 |
| VERBINDUNG (1) | 1000 | 97,6 | 78,4 |

**Mischung M2**

| **Verbindung** | **Konz. (ppm)** | **VHR (initial)** | **VHR (Sonne)** |
|---|---|---|---|
| - | - | 95,9 | 72,2 |
| TINUVIN®770 | 100 | 97,2 | 82,9 |
| TINUVIN®770 | 500 | 98,0 | 76,4 |
| TINUVIN®770 | 1000 | 96,9 | 74,7 |
| VERBINDUNG (1) | 100 | 96,5 | 81,5 |
| VERBINDUNG (1) | 500 | 96,1 | 75,5 |
| VERBINDUNG (1) | 1000 | 96,4 | 71,3 |

**Mischung M3 (Raumfemperafur, 10 Hz)**

| **Stabilisator** | **Konz. (ppm)** | **VHR (initial)** | **VHR (Sonne)** |
|---|---|---|---|
| - | - | 97,1 | 90,2 |
| TINUVIN®770 | 100 | 99,1 | 96,7 |
| TINUVIN®770 | 500 | 99,0 | 98,8 |
| TINUVIN®770 | 1000 | 98,8 | 97,2 |
| VERBINDUNG (1) | 100 | 98,9 | 96,8 |
| VERBINDUNG (1) | 500 | 98,7 | 96,9 |
| VERBINDUNG (1) | 1000 | 98,4 | 96,3 |

**Mischung M3**

| **Stabilisator** | **Konz. (ppm)** | **VHR (initial)** | **VHR (Sonne)** |
|---|---|---|---|
| - | - | 65,8 | 54,5 |
| TINUVIN®770 | 100 | 98,0 | 69,3 |
| TINUVIN®770 | 500 | 98,0 | 70,7 |
| TINUVIN®770 | 1000 | 72,2 | 70,7 |
| VERBINDUNG (1) | 100 | 69,4 | 70,2 |
| VERBINDUNG (1) | 500 | 98,0 | 70,2 |
| VERBINDUNG (1) | 1000 | 98,0 | 67,8 |

### VHR - Lichtbeständigkeit (Hintergrundbeleuchtung)

Zunächst wird die Stabilität der Voltage Holding Ratio der jeweiligen Testmischung selbst, von einer weiteren Probe dieser Mischung, der die angegebenen Mengen der Verbindung TINUVIN®770 zugesetzt wurden, sowie von einer weiteren Probe dieser Mischung, der die angegebenen Mengen der Verbindung 1 aus Beispiel 1 zugesetzt wurden nach 5min bei 100°C, bei 1V und 60Hz bestimmt (0h) Die resultierenden Mischungen werden in einer Testzelle mit einem Orientierungsmaterial für planare Ausrichtung und flächigen ITO-Elektroden auf ihre Lichtbeständigkeit (Hintergrundbeleuchtung)_untersucht. Zur Bestimmung der VHR in Abhängigkeit der Lichtbeständigkeit im Zusammenhang einer Hintergrundbeleuchtung werden versiegelte Testzellen mit einer kommerziell erhältlichen Hintergrundbeleuchtungseinheit getestet. Die Bestrahlungsdauer entspricht max. 1000 h. Nach den angegebenen Zeitabständen wird jeweils die Voltage Holding Ratio nach 5 Minuten bei einer Temperatur von 100°C, 1 V und 60Hz bestimmt. Die Ergebnisse sind unten in der folgenden Tabellen zusammengestellt:

| **Stabilisator** | **Konz. (ppm)** | **0h** | **48h** | **168h** | **336h** | **504h** | **744h** | **1000h** |
|---|---|---|---|---|---|---|---|---|
| Mischung M1 | | | | | | | | |
| - | - | 98,0 | 81,5 | 69,0 | 65,1 | 62,7 | 61,4 | 59,7 |
| TINUVIN®770 | 100 | 97,8 | 95,9 | 90,8 | 86,5 | 82,9 | 79,3 | 76,8 |
| TINUVIN®770 | 500 | 97,5 | 95,7 | 92,6 | 89,9 | 87,4 | 85,0 | 82,5 |
| TINUVIN®770 | 1000 | 97,3 | 96,0 | 93,9 | 90,9 | 88,5 | 86,5 | 84,8 |
| VERBINDUNG (1) | 100 | 97,9 | 95,8 | 91,3 | 87,7 | 85,4 | 82,7 | 79,3 |
| VERBINDUNG (1) | 500 | 97,5 | 96,0 | 93,4 | 91,0 | 88,6 | 84,6 | 82,0 |
| VERBINDUNG (1) | 1000 | 97,4 | 96,5 | 93,7 | 90,6 | 88,0 | 85,4 | 82,4 |
| | | | | | | | | |

| Mischung M2 | | | | | | | | |
|---|---|---|---|---|---|---|---|---|
| - | - | 94,9 | 84,3 | 74,2 | 68,9 | 66.4 | 64,4 | 62,8 |
| TINUVIN®770 | 100 | 96,7 | 95,6 | 92,1 | 89,7 | 88,0 | 83,9 | 81,7 |
| TINUVIN®770 | 500 | 96,7 | 95,8 | 93,1 | 90,7 | 89,1 | 87,4 | 85,5 |
| TINUVIN®770 | 1000 | 96,6 | 96,3 | 94,7 | 92,5 | 91,0 | 89,4 | 87,8 |
| VERBINDUNG (1) | 100 | 96,1 | 94,6 | 90,7 | 87,4 | 85,1 | 83,5 | 81,6 |
| VERBINDUNG (1) | 500 | 96,0 | 94,7 | 91,9 | 89,4 | 87,7 | 86,4 | 84,8 |
| VERBINDUNG (1) | 1000 | 95,5 | 94,7 | 91,6 | 88,9 | 87,1 | 85,5 | 84,2 |

### VHR - Hitzebeständigkeit

Zunächst wird die Stabilität der Voltage Holding Ratio der jeweiligen Testmischung M1 selbst, von einer weiteren Probe dieser Mischung, der die angegebenen Mengen der Verbindung TINUVIN®770 zugesetzt wurden, sowie von einer weiteren Probe dieser Mischung, der die angegebenen Mengen der Verbindung 1 aus Beispiel 1 zugesetzt wurden nach 5min bei 100°C, bei 1V und 60Hz bestimmt (initial) Die resultierenden Mischungen werden in einer Testzelle mit einem Orientierungsmaterial für planare Ausrichtung und flächigen ITO-Elektroden auf ihre Hitzebeständigkeit untersucht. Zur Bestimmung der VHR in Abhängigkeit der Hitzebeständigkeit werden versiegelte Testzellen für 120h in einem herkömmlichen Laborheizschrank bei 100 °C aufbewahrt und jeweils die Voltage Holding Ratio nach 5 Minuten bei einer Temperatur von 100°C, 1V und 60Hz bestimmt (VHR
(heat, 120 h)). Die Ergebnisse sind unten in der folgenden Tabelle zusammengestellt:

**Mischung M1**

| **Stabilisator** | **Konz. (ppm)** | **VHR (initial)** | **VHR (heat, 120 h)** |
|---|---|---|---|
| - | - | 96,2 | 92,5 |
| TINUVIN®770 | 100 | 97,7 | 97,5 |
| TINUVIN®770 | 500 | 97,9 | 96,0 |
| TINUVIN®770 | 1000 | 97,6 | 95,0 |
| VERBINDUNG (1) | 100 | 97,4 | 95,8 |
| VERBINDUNG (1) | 500 | 97,5 | 95,5 |
| VERBINDUNG (1) | 1000 | 97,4 | 94,1 |

### Tieftemperaturstabilität

5ml Glasflaschen werden mit 1 g der jeweiligen Testmischung M1 bis M3 befüllt und bei verschiedenen Temperaturen in einer Temperaturkammer aufbewahrt. Die Proben werden täglich auf die Phasenbeständigkeit untersucht.

Die Ergebnisse sind unten in der folgenden Tabelle zusammengestellt:

| **Mischung** | **Temp.** | **ohne Stabilisator** | **1000 ppm Tinuvin 770** | **1000 ppm Verbindung (1)** |
|---|---|---|---|---|
| M1 | -20 °C | 792h | 168h | 528h |
| M2 | -20 °C | °C - | 96h | 240h |
| M3 | -20 °C | °C - | 192h | 1000h |

## Patentansprüche

1. Verbindungen der Formel I, 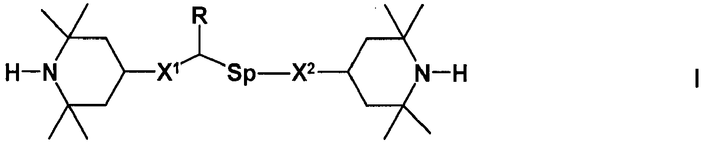 worin
X¹ und X² jeweils unabhängig von einander -O-, -(CO)-O-, -O-(CO)-, -O-(CO)-O-, -NH- oder -NH-(CO)-,
Sp geradkettiges Alkyl mit 1 bis 20 C-Atomen, auch Cycloalkyl, Cycloalkylalkyl, oder Alkylcycloalkyl, wobei in allen diesen Gruppen eine oder mehrere -CH₂- Gruppen durch -O- so ersetzt sein können, dass im Molekül keine zwei O-Atome direkt miteinander verbunden sind, und
R geradkettiges oder verzweigtes Alkyl mit 1 bis 10 C-Atomen auch Cycloalkyl, Cycloalkylalkyl, oder Alkylcycloalkyl, wobei in allen diesen Gruppen eine oder mehrere -CH₂- Gruppen durch -O- so ersetzt sein können, dass im Molekül keine zwei O-Atome direkt miteinander verbunden sind,
bedeuten.

2. Verbindungen der Formel I nach Anspruch 1 ausgewählt aus den Verbindungen der Formel IA bis IE, 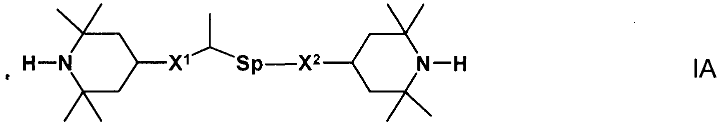 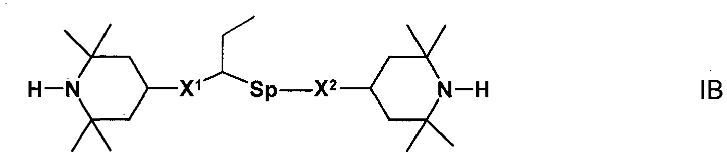 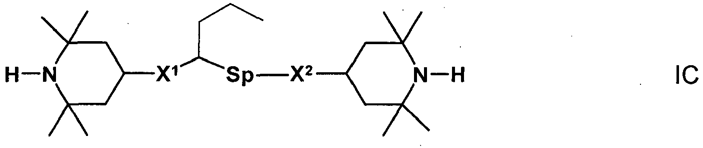 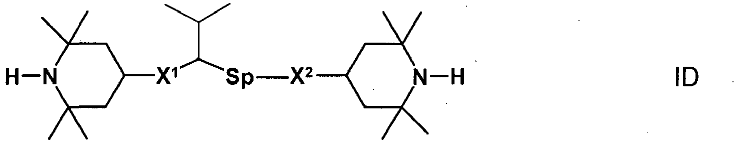 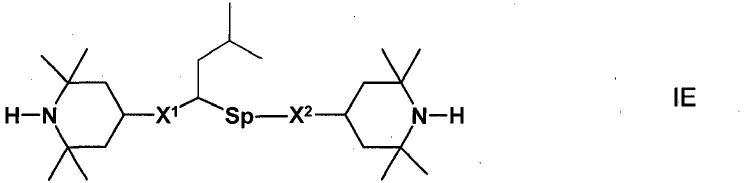 worin X¹, X² und Sp eine der unter Anspruch 1 angegebenen Bedeutung haben.

3. Verbindungen der Formel I nach Anspruch 1 oder 2 ausgewählt aus den Verbindungen der Formel IA-1 bis IE-1 und IA-2 bis IE-2, 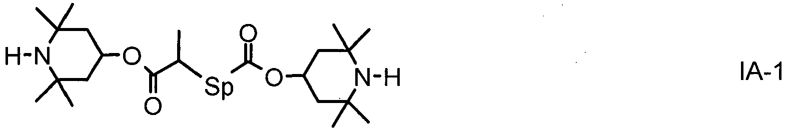 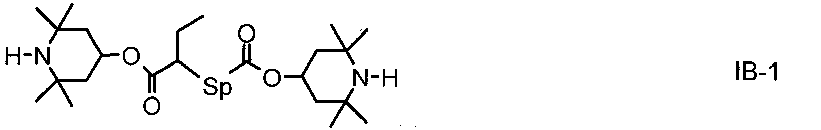 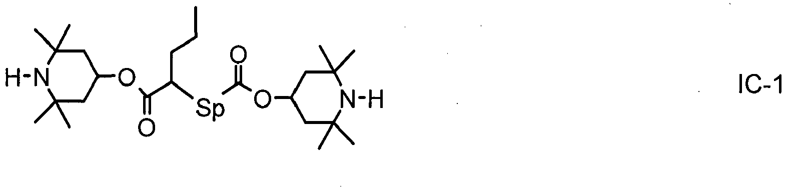 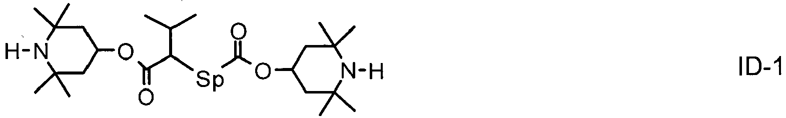 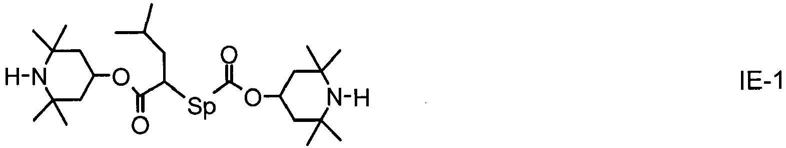 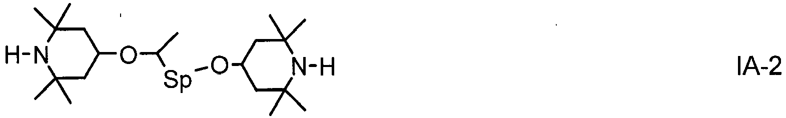 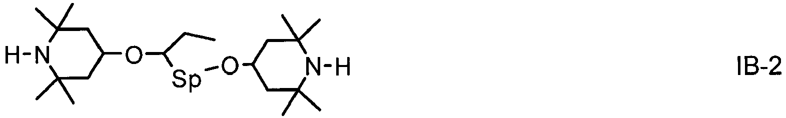 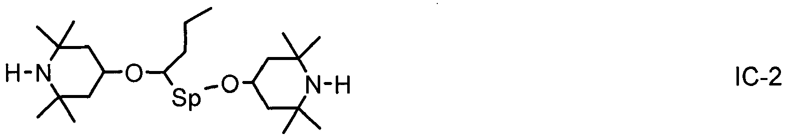 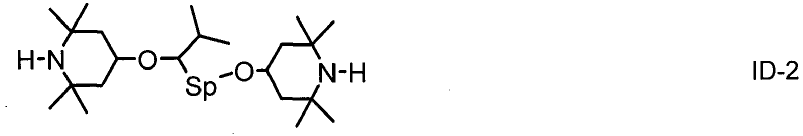 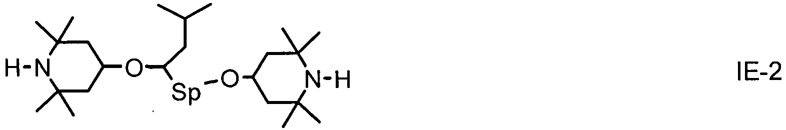 worin Sp eine der unter Anspruch 1 angegebenen Bedeutung hat.

4. Medium enthaltend eine oder mehrere Verbindungen der Formel I nach einem oder mehreren der Ansprüche 1 bis 3.

5. Medium enthaltend eine oder mehrere Verbindungen der Formel I nach einem oder mehreren der Ansprüche 1 bis 3 in einer Konzentration im Bereich von 1 ppm bis zu 5.000 ppm.

6. Medium nach Anspruch 4 oder 5, enthaltend eine oder mehrere Verbindungen der Formeln II und/oder III, 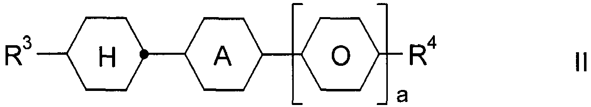 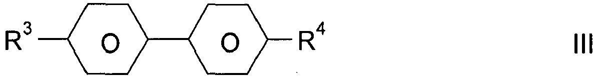 worin
A 1,4-Phenylen oder trans-1,4-Cyclohexylen bedeutet,
a 0 oder 1 ist,
R³ Alkenyl mit 2 bis 9 C-Atomen bedeutet,und
R⁴ einen Alkyl- oder Alkoxyrest mit 1 bis 15 C-Atomen, wobei in diesen Resten auch eine oder mehrere CH₂-Gruppen jeweils unabhängig voneinander durch -C≡C-, -CF₂O-, -CH=CH-, 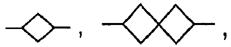 -O-, -CO-O-, -O-CO- so ersetzt sein können, dass O-Atome nicht direkt miteinander verknüpft sind, und worin auch ein oder mehrere H-Atome durch Halogen ersetzt sein können, vorzugsweise Alkyl mit 1 bis 12 C-Atomen oder Alkenyl mit 2 bis 9 C-Atomen bedeutet.

7. Medium nach einem oder mehreren der Ansprüche 4 bis 6, enthaltend eine oder mehrere Verbindungen der Formeln D1, D2, D3, D4 und/oder D5, 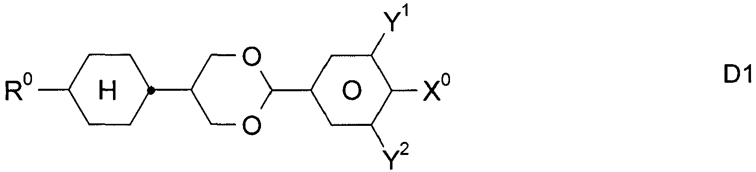 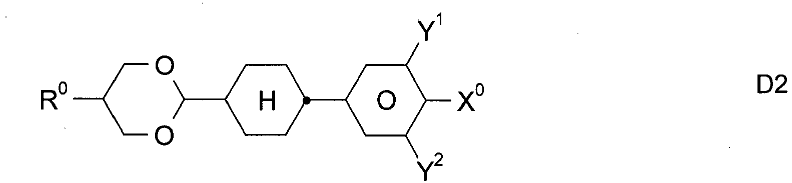 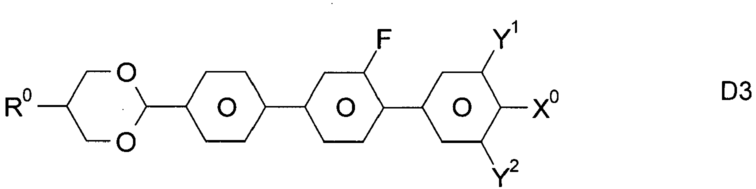 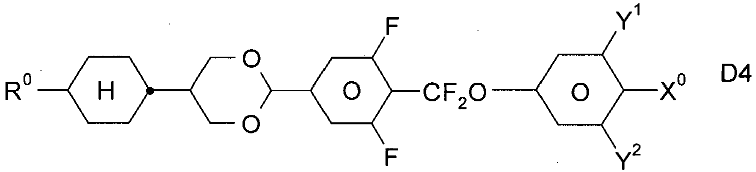 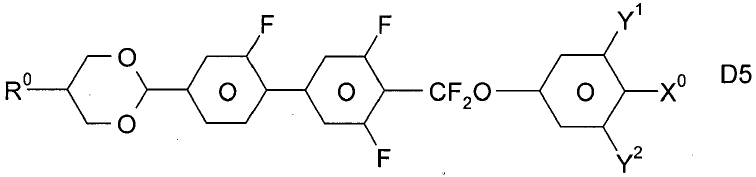 worin
R⁰ einen Alkyl- oder Alkoxyrest mit 1 bis 15 C-Atomen, wobei in diesen Resten auch eine oder mehrere CH₂-Gruppen jeweils unabhängig voneinander durch -C≡C-, -CF₂O-, -CH=CH-, 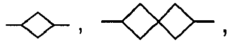 -O-, -CO-O- oder -O-CO- so ersetzt sein können, dass O-Atome nicht direkt miteinander verknüpft sind, und worin auch ein oder mehrere H-Atome durch Halogen ersetzt sein können,
X⁰ F, Cl, ein- oder mehrfach fluorierter Alkyl- oder Alkoxyrest mit 1 bis 6 C-Atomen, ein oder mehrfach fluorierter Alkenyl- oder Alkenyloxyrest mit 2 bis 6 C-Atomen, und
Y¹⁻² jeweils unabhängig voneinander H oder F,
bedeuten.

8. Medium nach einem oder mehreren der Ansprüche 4 bis 6, enthaltend eine oder mehrere Verbindungen ausgewählt aus der Gruppe der Verbindungen der Formeln Y-1, Y-2, Y-3 und Y-4, 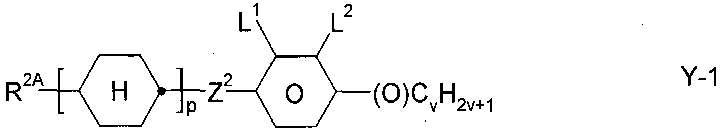 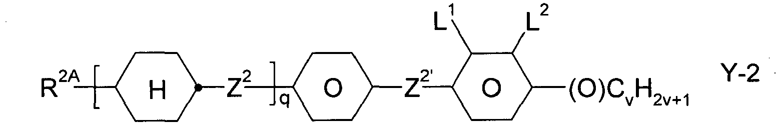 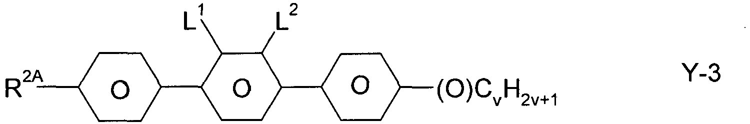 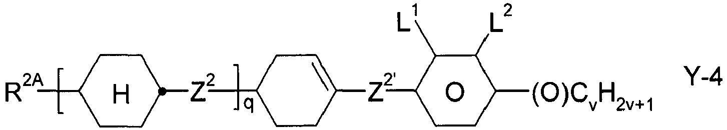 worin
R^{2A} H, einen Alkyl- oder Alkoxyrest mit 1 bis 15 C-Atomen, wobei in diesen Resten auch eine oder mehrere CH₂-Gruppen jeweils unabhängig voneinander durch -C≡C-, -CF₂O-, -CH=CH-, 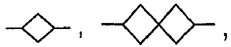 -O-, -CO-O-, -O-CO- so ersetzt sein können, dass O-Atome nicht direkt miteinander verknüpft sind, und worin auch ein oder mehrere H-Atome durch Halogen ersetzt sein können,
L¹ und L² jeweils unabhängig voneinander F, Cl, CF₃ oder CHF₂,
Z² und Z^{2'} jeweils unabhängig voneinander eine Einfachbindung, -CH₂CH₂-, -CH=CH-, -CF₂O-, -OCF₂-,-CH₂O-, -OCH₂-, -COO-, -OCO-, -C₂F₄-, -CF=CF-, -CH= CHCH₂O-,
p 0, 1 oder 2,
q 0 oder 1,
(O)CᵥH₂ᵥ₊₁ OCᵥH₂ᵥ₊₁ oder CᵥH₂ᵥ₊₁, und
v 1 bis 6,
bedeuten

9. Medium nach einem oder mehreren der Ansprüche 4 bis 8, **dadurch gekennzeichnet, dass** es die Verbindung der Formel IIa-1 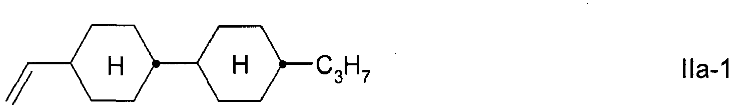 enthält.

10. Medium nach einem oder mehreren der Ansprüche 4 bis 9, **dadurch gekennzeichnet, dass** es zusätzlich eine oder mehrere polymerisierbare Verbindungen enthält.

11. Verfahren zur Herstellung eines Mediums nach einem oder mehreren der Ansprüche 4 bis 10, **dadurch gekennzeichnet, dass** man eine oder mehrere Verbindungen der Formel I mit mindestens einer weiteren mesogenen Verbindung und optional mit einem oder mehreren Additiv(en) und/oder einer oder mehreren polymerisierbaren Verbindungen mischt.

12. Verwendung eines Mediums nach einem oder mehreren der Ansprüche 4 bis 10 für elektrooptische Zwecke.

13. Elektrooptische Flüssigkristallanzeige enthaltend ein Medium nach einem oder mehreren der Ansprüche 4 bis 10.

## Claims

1. Compounds of the formula I, 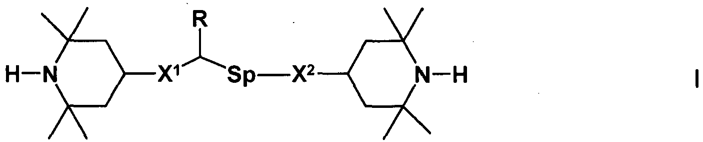 in which
X¹ and X² each, independently of one another, denote -O-, -(CO)-O-, -O-(CO)-, -O-(CO)-O-, -NH- or -NH-(CO)-,
Sp denotes straight-chain alkyl having 1 to 20 C atoms, also cycloalkyl, cycloalkylalkyl or alkylcycloalkyl, where one or more -CH₂- groups in all these groups may be replaced by -O- in such a way that no two O atoms in the molecule are connected directly to one another, and
R denotes straight-chain or branched alkyl having 1 to 10 C atoms, also cycloalkyl, cycloalkylalkyl or alkylcyclo-alkyl, where one or more -CH₂- groups in all these groups may be replaced by -O- in such a way that no two O atoms in the molecule are connected directly to one another.

2. Compounds of the formula I according to Claim 1 selected from the compounds of the formulae IA to IE, 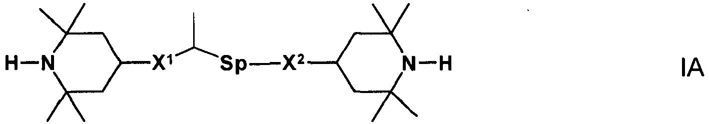 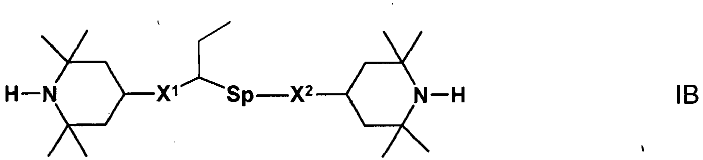 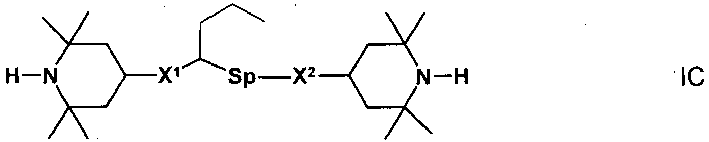 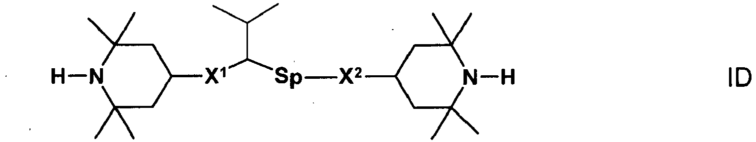 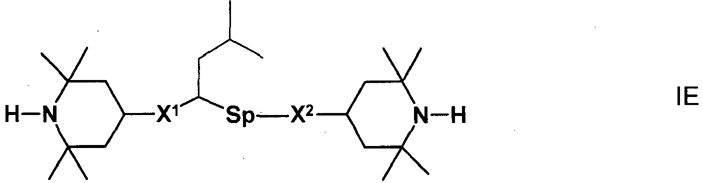 in which X¹, X² and Sp have one of the meanings indicated under Claim 1.

3. Compounds of the formula I according to Claim 1 or 2 selected from the compounds of the formulae IA-1 to IE-1 and IA-2 to IE-2, 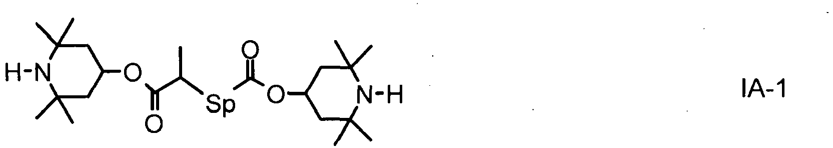 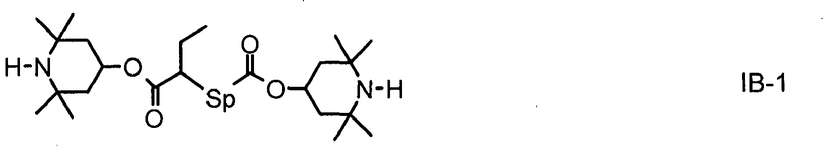 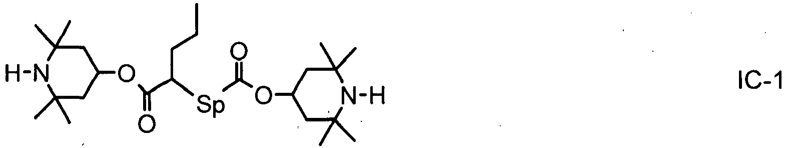 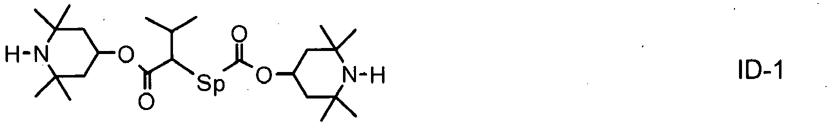 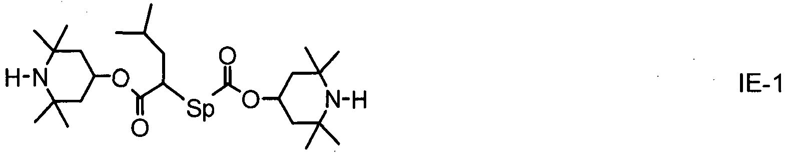 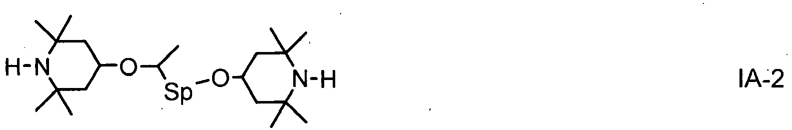 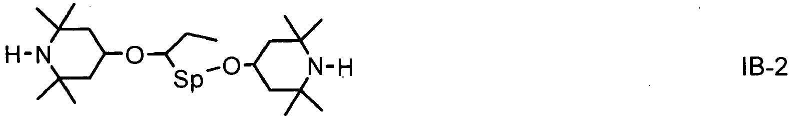 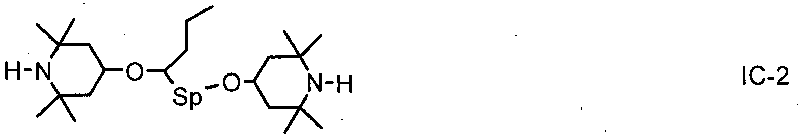 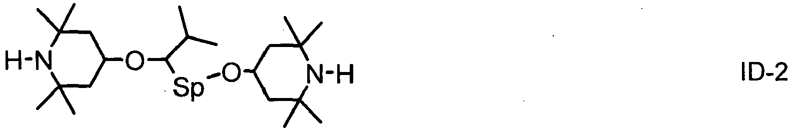 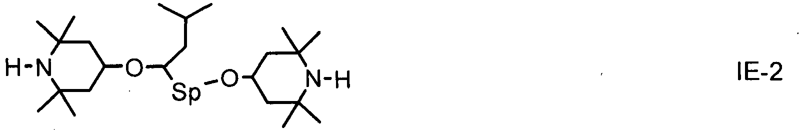 in which Sp has one of the meanings indicated under Claim 1.

4. Medium comprising one or more compounds of the formula I according to one or more of Claims 1 to 3.

5. Medium comprising one or more compounds of the formula I according to one or more of Claims 1 to 3 in a concentration in the range from 1 ppm to 5,000 ppm.

6. Medium according to Claim 4 or 5, comprising one or more compounds of the formulae II and/or III, 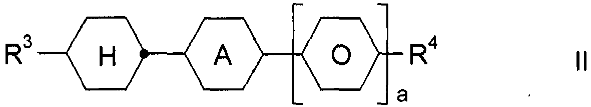 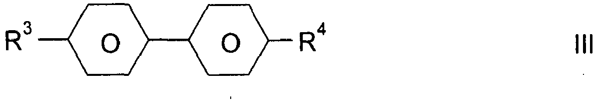 in which
A denotes 1,4-phenylene or trans-1,4-cyclohexylene,
a is 0 or 1,
R³ denotes alkenyl having 2 to 9 C atoms, and
R⁴ denotes an alkyl or alkoxy radical having 1 to 15 C atoms, where, in addition, one or more CH₂ groups in these radicals may each be replaced, independently of one another, by -C≡C-, -CF₂O-, -CH=CH-, 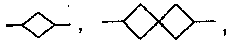 -O-, -CO-O- or -O-CO- in such a way that O atoms are not linked directly to one another, and in which, in addition, one or more H atoms may be replaced by halogen, preferably alkyl having 1 to 12 C atoms or alkenyl having 2 to 9 C atoms.

7. Medium according to one or more of Claims 4 to 6, comprising one or more compounds of the formulae D1, D2, D3, D4 and/or D5, 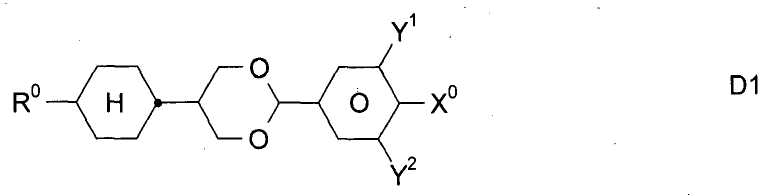 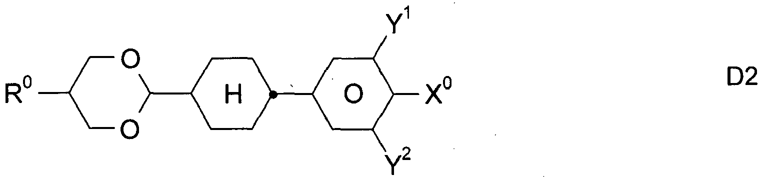 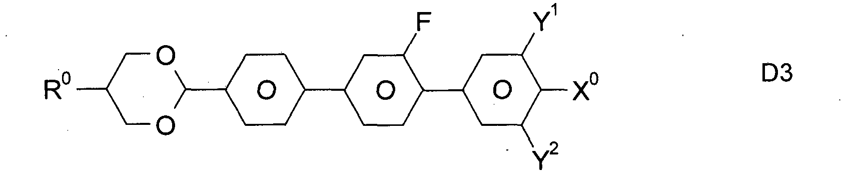 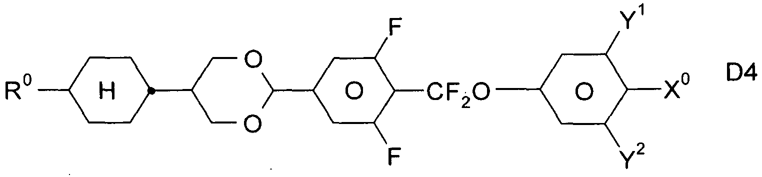 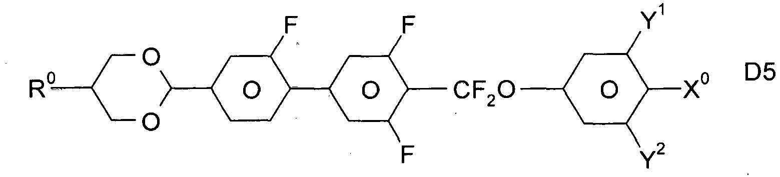 in which
R⁰ denotes an alkyl or alkoxy radical having 1 to 15 C atoms, where, in addition, one or more CH₂ groups in these radicals may each be replaced, independently of one another, by -C≡C-, -CF₂O-, -CH=CH-, 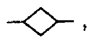 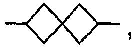 -O-, -CO-O- or -O-CO- in such a way that O atoms are not linked directly to one another, and in which, in addition, one or more H atoms may be replaced by halogen,
X⁰ denotes F, Cl, a mono- or polyfluorinated alkyl or alkoxy radical having 1 to 6 C atoms or a mono- or polyfluorinated alkenyl or alkenyloxy radical having 2 to 6 C atoms, and
Y¹⁻² each, independently of one another, denote H or F.

8. Medium according to one or more of Claims 4 to 6, comprising one or more compounds selected from the group of the compounds of the formulae Y-1, Y-2, Y-3 and Y-4, 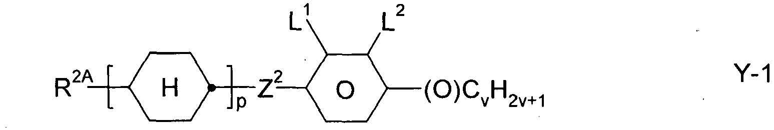 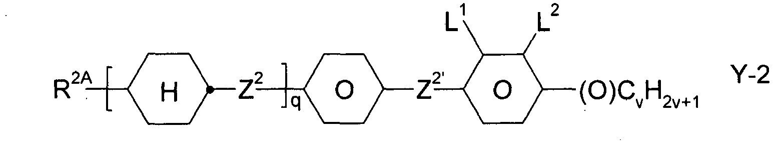 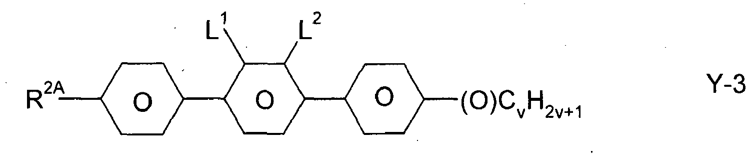 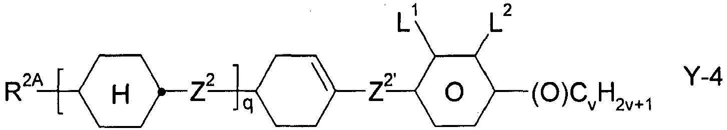 in which
R^{2A} denotes H, an alkyl or alkoxy radical having 1 to 15 C atoms, where, in addition, one or more CH₂ groups in these radicals may each be replaced, independently of one another, by -C≡C-, -CF₂O-, -CH=CH-, 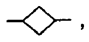 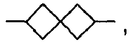 -O-, -CO-O- or -O-CO- in such a way that O atoms are not linked directly to one another, and in which, in addition, one or more H atoms may be replaced by halogen,
L¹ and L² each, independently of one another, denote F, Cl, CF₃ or CHF₂,
Z² and Z^{2'} each, independently of one another, denote a single bond, -CH₂CH₂-, -CH=CH-, -CF₂O-, -OCF₂-, -CH₂O-, -OCH₂-, -COO-, -OCO-, -C₂F₄-, -CF=CF- or -CH=CHCH₂O-,
p denotes 0, 1 or 2,
q denotes 0 or 1,
(O)CᵥH₂ᵥ₊₁ denotes OCᵥH₂ᵥ₊₁ or CᵥH₂ᵥ₊₁, and
v denotes 1 to 6.

9. Medium according to one or more of Claims 4 to 8, **characterised in that** it comprises the compound of the formula IIa-1

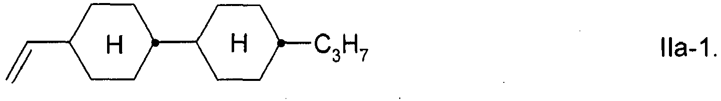

10. Medium according to one or more of Claims 4 to 9, **characterised in that** it additionally comprises one or more polymerisable compounds.

11. Process for the preparation of a medium according to one or more of Claims 4 to 10, **characterised in that** one or more compounds of the formula I are mixed with at least one further mesogenic compound and optionally with one or more additive(s) and/or one or more polymerisable compounds.

12. Use of a medium according to one or more of Claims 4 to 10 for electro-optical purposes.

13. Electro-optical liquid-crystal display containing a medium according to one or more of Claims 4 to 10.

## Revendications

1. Composés de la formule I : 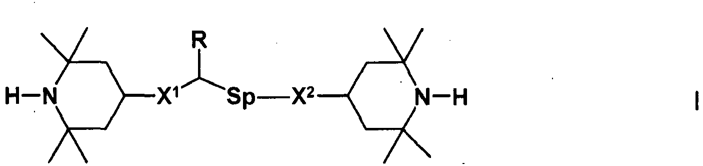 dans laquelle :
X¹ et X² représentent chacun, indépendamment l'un de l'autre, -O-,-(CO)-O-, -O-(CO)-, -O-(CO)-O-, -NH- ou -NH-(CO)-,
Sp représente alkyle en chaîne droite qui comporte 1 à 20 atome(s) de C, également cycloalkyle, cycloalkylalkyle ou alkylcycloalkyle, où un ou plusieurs groupe(s) -CH₂-dans tous ces groupes peut/peuvent être remplacé(s) par -O- de telle sorte que deux atomes de O dans la molécule ne soient pas connectés directement l'un à l'autre, et
R représente alkyle en chaîne droite ou ramifié qui comporte 1 à 10 atome(s) de C, également cycloalkyle, cycloalkylalkyle ou alkylcycloalkyle, où un ou plusieurs groupe(s) -CH₂- dans tous ces groupes peut/peuvent être remplacé(s) par -O- de telle sorte que deux atomes de O dans la molécule ne soient pas connectés directement l'un à l'autre.

2. Composés de la formule I selon la revendication 1, sélectionnés parmi les composés des formules IA à IE : 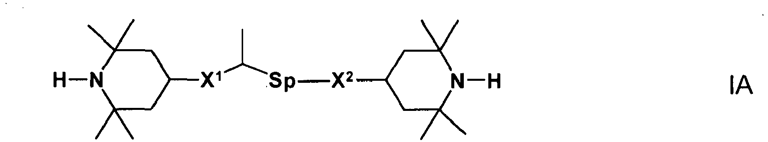 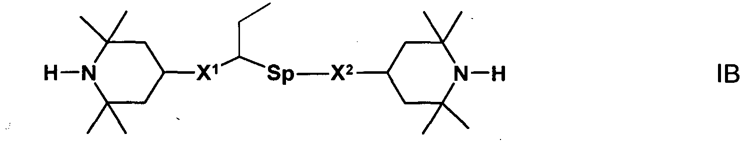 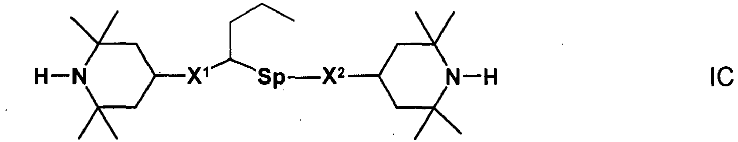 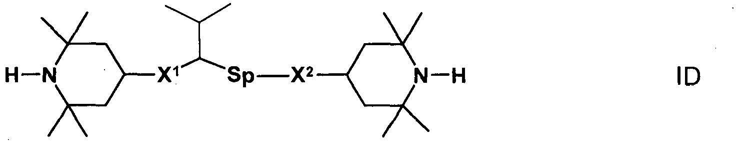 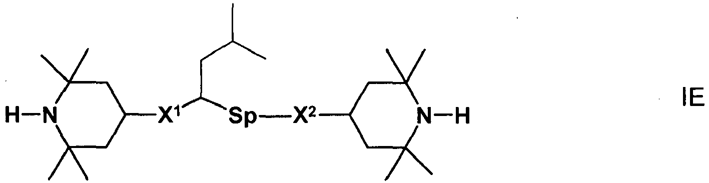 dans lesquelles X¹, X² et Sp présentent l'une des significations indiquées selon la revendication 1.

3. Composés de la formule I selon la revendication 1 ou 2, sélectionnés parmi les composés des formules IA-1 à IE-1 et IA-2 à IE-2 : 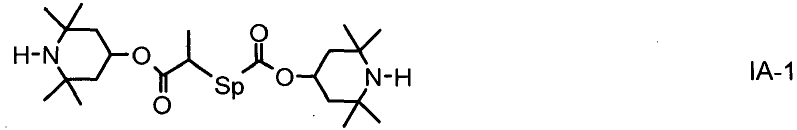 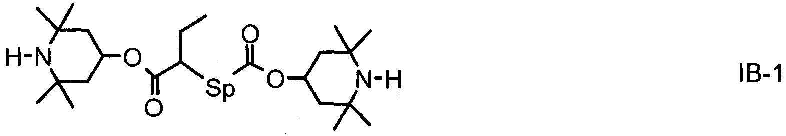 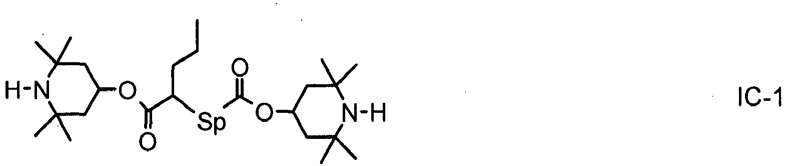 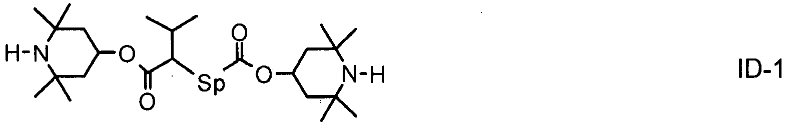 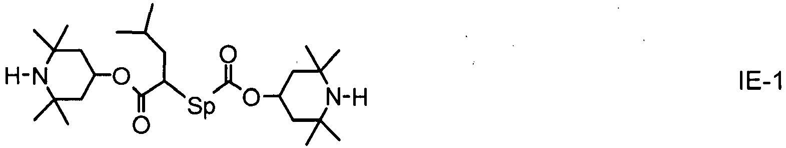 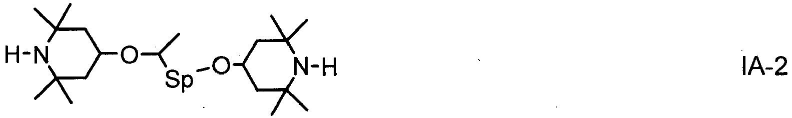 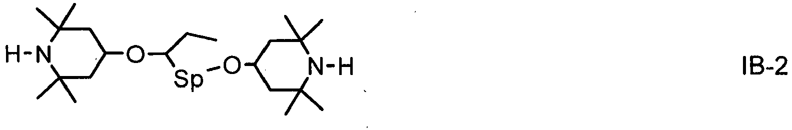 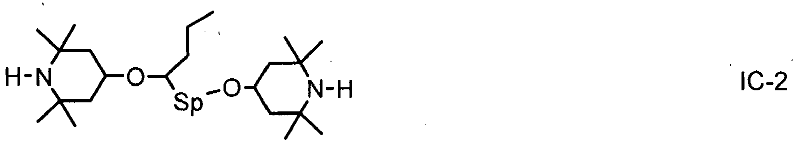 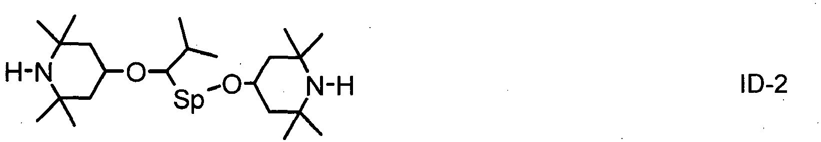 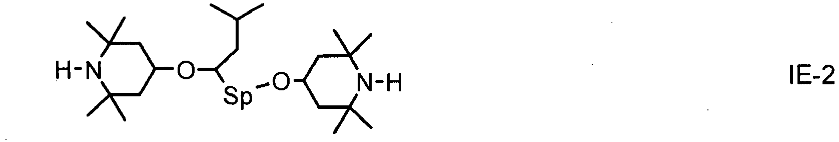 dans lesquelles Sp présente l'une des significations indiquées selon la revendication 1.

4. Milieu comprenant un ou plusieurs composé(s) de la formule I selon une ou plusieurs des revendications 1 à 3.

5. Milieu comprenant un ou plusieurs composé(s) de la formule I selon une ou plusieurs des revendications 1 à 3 selon une concentration dans la plage de 1 ppm à 5 000 ppm.

6. Milieu selon la revendication 4 ou 5, comprenant un ou plusieurs composé(s) des formules II et/ou III : 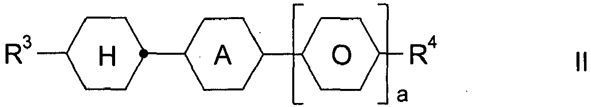 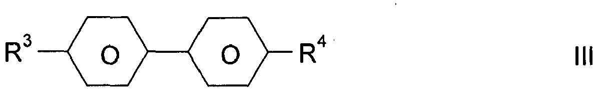 dans lesquelles :
A représente 1,4-phénylène ou trans-1,4-cyclohexylène,
a est 0 ou 1,
R³ représente alkényle qui comporte 2 à 9 atomes de C, et
R⁴ représente un radical alkyle ou alcoxy qui comporte 1 à 15 atome(s) de C, où, en outre, un ou plusieurs groupe(s) CH₂ dans ces radicaux peut/peuvent chacun être remplacé(s), indépendamment les uns des autres, par -C≡C-, -CF₂O-, -CH=CH-, 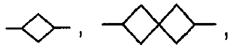 -O-, -CO-O- ou -O-CO- de telle sorte que des atomes de O ne soient pas liés directement les uns aux autres, et où, en outre, un ou plusieurs atome(s) de H peut/peuvent être remplacé(s) par halogène, de façon préférable alkyle qui comporte 1 à atome(s) de 12 C ou alkényle qui comporte 2 à 9 atomes de C.

7. Milieu selon une ou plusieurs des revendications 4 à 6, comprenant un ou plusieurs composé(s) des formules D1, D2, D3, D4 et/ou D5 : 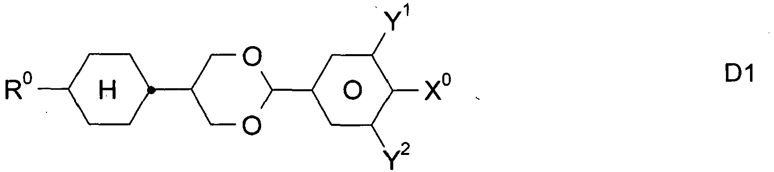 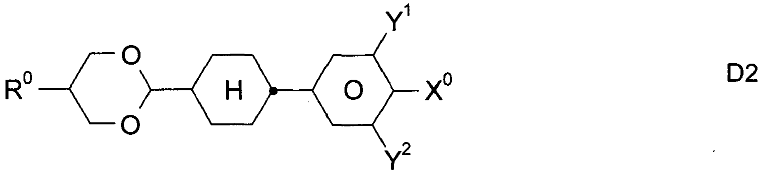 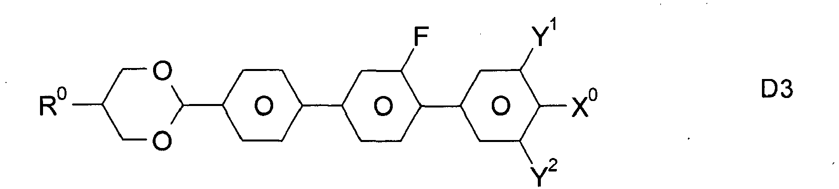 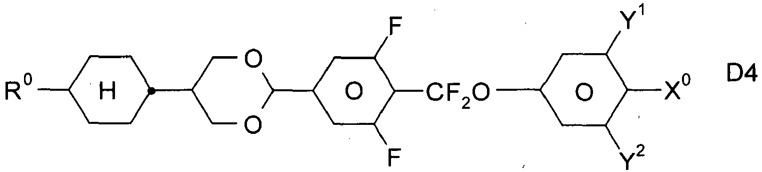 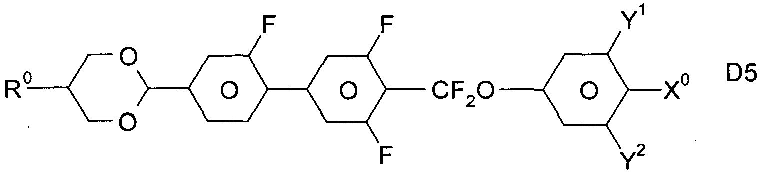 dans lesquelles :
R⁰ représente un radical alkyle ou alcoxy qui comporte 1 à 15 atome(s) de C, où, en outre, un ou plusieurs groupe(s) CH₂ dans ces radicaux peut/peuvent chacun être remplacé(s), indépendamment les uns des autres, par -C≡C-, -CF₂O-, -CH=CH-, 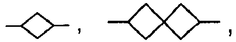 -O-, -CO-O- ou -O-CO- de telle sorte que des atomes de O ne soient pas liés directement les uns aux autres, et où, en outre, un ou plusieurs atome(s) de H peut/peuvent être remplacé(s) par halogène,
X⁰ représente F, Cl, un radical alkyle ou alcoxy monofluoré ou polyfluoré qui comporte 1 à 6 atome(s) de C ou un radical alkényle ou alkényloxy monofluoré ou polyfluoré qui comporte 2 à 6 atomes de C, et
Y¹⁻² représentent chacun, indépendamment les uns des autres, H ou F.

8. Milieu selon une ou plusieurs des revendications 4 à 6, comprenant un ou plusieurs composé(s) sélectionné(s) parmi le groupe des composés des formules Y-1, Y-2, Y-3 et Y-4 : 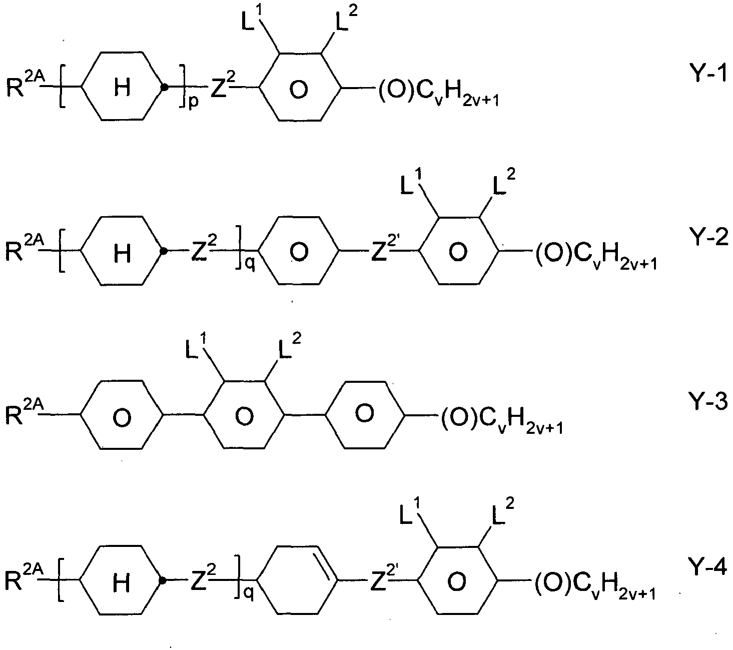 dans lesquelles :
R^{2A} représente H, un radical alkyle ou alcoxy qui comporte 1 à 15 atome(s) de C, où, en outre, un ou plusieurs groupe(s) CH₂ dans ces radicaux peut/peuvent chacun être remplacé(s), indépendamment les uns des autres, par -C≡C-, -CF₂O-, -CH=CH-, 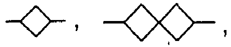 -O-, -CO-O- ou -O-CO- de telle sorte que des atomes de O ne soient pas liés directement les uns aux autres, et où, en outre, un ou plusieurs atome(s) de H peut/peuvent être remplacé(s) par halogène,
L¹ et L² représentent chacun, indépendamment l'un de l'autre, F, Cl, CF₃ ou CHF₂,
Z² et Z^{2'} représentent chacun, indépendamment l'un de l'autre, une liaison simple, -CH₂CH₂-, -CH=CH-, -CF₂O-, -OCF₂-, -CH₂O-, -OCH₂-, -COO-, -OCO-, -C₂F₄-, -CF=CF- ou -CH=CHCH₂O-,
p représente 0, 1 ou 2,
q représente 0 ou 1,
(O)CᵥH₂ᵥ₊₁ représente OCᵥH₂ᵥ₊₁ ou CᵥH₂ᵥ₊₁, et
v représente 1 à 6.

9. Milieu selon une ou plusieurs des revendications 4 à 8, **caractérisé en ce qu'**il comprend le composé de la formule IIa-1:

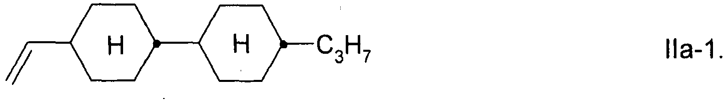

10. Milieu selon une ou plusieurs des revendications 4 à 9, **caractérisé en ce qu'**il comprend de manière additionnelle un ou plusieurs composé(s) polymérisable(s).

11. Procédé pour la préparation d'un milieu selon une ou plusieurs des revendications 4 à 10, **caractérisé en ce qu'**un ou plusieurs composé(s) de la formule I est/sont mélangé(s) avec au moins un composé mésogène supplémentaire et en option, avec un ou plusieurs additif(s) et/ou un ou plusieurs composé(s) polymérisable(s).

12. Utilisation d'un milieu selon une ou plusieurs des revendications 4 à 10 à des fins électro-optiques.

13. Affichage à cristaux liquides électro-optique qui contient un milieu selon une ou plusieurs des revendications 4 à 10.
